# EUROPEAN PATENT SPECIFICATION

(11) **EP 3 275 880 B1**
(45) Date of publication and mention of the grant of the patent: **09.09.2020**
(21) Application number: 16768758.1
(22) Date of filing: 22.03.2016
(51) Int. Cl.: C07D 403/14, C07D 403/04, H01L 27/146, H01L 51/42, H01L 51/46, H01L 51/50

(54) **2-(QUINAZOLIN-6-YL)-9H-CARBAZOLE AND 3-(QUINAZOLIN-6-YL)-9H-CARBAZOLE COMPOUNDS, AND ELECTRONIC DEVICES, LUMINESCENT ELEMENTS, PHOTOELECTRIC CONVERSION ELEMENTS, AND IMAGE SENSORS CONTAINING SAME**
2-(QUINAZOLIN-6-YL)-9H-CARBAZOL- UND 3-(QUINAZOLIN-6-YL)-9H-CARBAZOL-VERBINDUNGEN UND ELEKTRONISCHE VORRICHTUNGEN, LUMINESZENTE ELEMENTE, FOTOELEKTRISCHE UMWANDLUNGSELEMENTE UND BILDSENSOREN DAMIT
COMPOSÉS 2-(QUINAZOLIN-6-YL)-9H-CARBAZOLIQUES AND 3-(QUINAZOLIN-6-YL)-9H-CARBAZOLIQUES, ET DISPOSITIFS ÉLECTRONIQUES, ÉLÉMENTS LUMINESCENTS, ÉLÉMENTS DE CONVERSION PHOTOÉLECTRIQUE, ET CAPTEURS D'IMAGE CONTENANT CEUX-CI

(30) Priority: 26.03.2015 JP 2015064012
(43) Date of publication of application: 31.01.2018
(73) Proprietor: Toray Industries, Inc., Tokyo 103-8666 (JP)
(72) Inventor: NAITO, Kojiro, Otsu-shi Shiga 520-8558 (JP); MATSUKI, Shinichi, Otsu-shi Shiga 520-8558 (JP); TANAKA, Daisaku, Otsu-shi Shiga 520-8558 (JP)
(74) Representative: Hoefer & Partner Patentanwälte mbB
(86) International application number: PCT/JP2016/058988
(87) International publication number: WO 2016/152855

(56) References cited:
- WO-A1-2004/077886
- WO-A1-2013/154325
- JP-A- 2010 199 296
- JP-A- 2014 241 405
- KR-A- 20120 117 693
- KR-A- 20150 111 106

## Description

### [Technical Field]

The present invention relates to a compound, and an electronic device, a luminescent element, a photoelectric conversion element, and an image sensor containing the compound.

### [Background Art]

In an organic thin film luminescent element, electrons injected from the negative electrode and holes injected from the positive electrode are recombined in a layer containing an organic compound sandwiched between the two electrodes to generate excitons, and the excitons emit light when being transitioned to the ground state. In recent years, such an organic thin film luminescent element has been actively studied.

Organic thin film luminescent elements are required to be improved in luminous efficiency, reduced in driving voltage, and improved in durability. In particular, compatibility between luminous efficiency and durability life is a great challenge. For example, materials having a carbazole skeleton and a quinazoline skeleton have been developed in order to improve luminous efficiency and durability life (see, for example, PTLs 1 to 5).

In addition, as for photoelectric conversion elements capable of converting light into electric energy, photoelectric conversion elements containing an organic compound have been studied recently. An organic compound can selectively absorb light in a specific wavelength region of light being incident thereon depending on the molecular structure, and thus a photoelectric conversion element containing an organic compound does not have to include a color filter. Moreover, the organic compound can improve the light utilization efficiency since it has high absorption coefficient.

There have been known, as specific photoelectric conversion elements containing an organic compound, elements having, between two electrodes, a photoelectric conversion film into which a p-n junction structure or a bulk heterojunction structure is introduced.

In an image sensor including a photoelectric conversion element, a voltage is often externally applied in order to improve the photoelectric conversion efficiency and improve the response speed. When holes or electrons are injected from an electrode into a photoelectric conversion film by an external electric field, a dark current is generated. For the purpose of reducing the dark current, an element including a hole blocking layer or an electron blocking layer is known (for example, see PTL 6).

### [Citation List]

### [Patent Literatures]

[PTL 1] International Publication WO 2006/049013
[PTL 2] JP 2010-275255A
[PTL 3] International Publication WO 2012/050347
[PTL 4] International Publication WO 2013/154325
[PTL 5] International Publication WO 2014/054596
[PTL 6] JP 2007-059515A

### [Summary of Invention]

### [Technical Problem]

The compounds described in PTLs 1 to 5, however, are insufficient in luminous efficiency and durability for being put to practical use as a luminescent element. Further, none of other conventional techniques found so far has durability in addition to high luminous efficiency and low driving voltage.

In addition, in the photoelectric conversion element containing a compound described in PTL 6 and other conventional techniques, the photoelectric conversion efficiency and the on/off ratio are not sufficient for obtaining a high-sensitivity image sensor.

Accordingly, the present invention has been made to solve these problems of the conventional techniques, and is intended to provide a material applicable to an organic thin film luminescent element improved in all of the luminous efficiency, driving voltage, and durability life, and also to a photoelectric conversion element having high conversion efficiency and high on/off ratio.

### [Solution to Problem]

The present invention, which is defined in the appended claims, provides a compound represented by the following general formula (4) or (5). 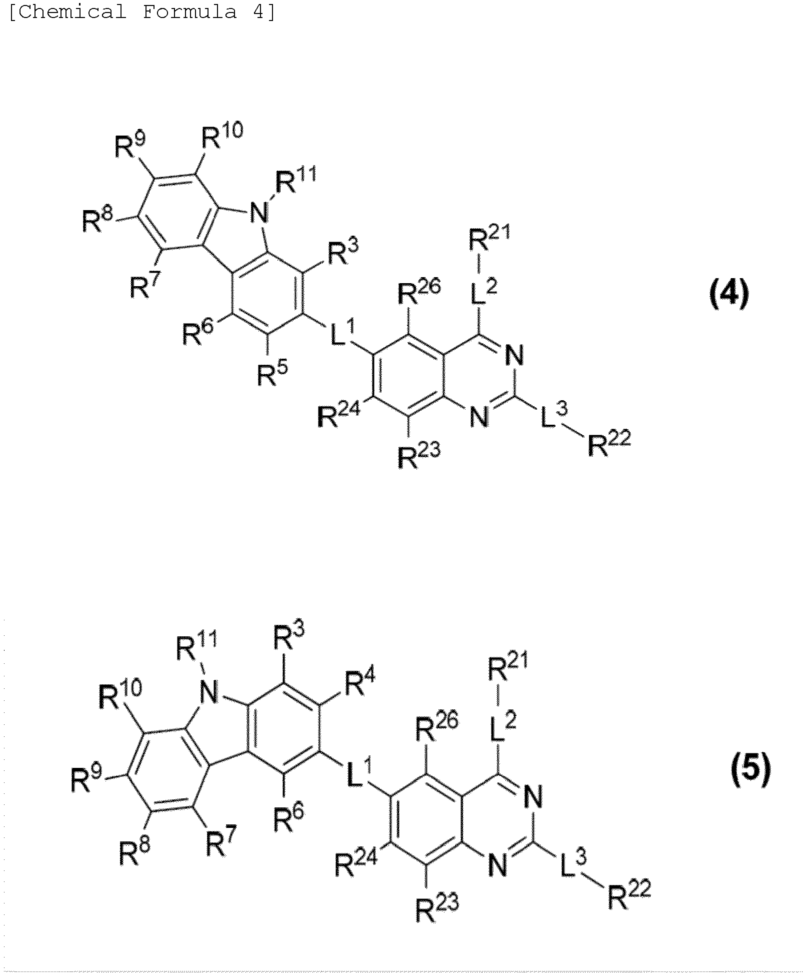 wherein L¹ is a single bond; wherein R³ to R¹⁰ are each a hydrogen atom except at the position that is linked to L¹, R¹¹ is selected from the group consisting of a hydrogen atom, an alkyl group, a cycloalkyl group, a heterocyclic group, an alkenyl group, a cycloalkenyl group, an aryl group, and a heteroaryl group; wherein L² is a single bond, a phenylene group, a naphthalenylene group, or a biphenylene group, R²¹ is a hydrogen atom, a halogen atom, a carbazolyl group, a benzocarbazolyl group, a dibenzofuranyl group, or a dibenzothiophenyl group, L³ is a single bond, a substituted or unsubstituted arylene group, or a substituted or unsubstituted heteroarylene group, and when L³ is a substituted arylene group or a substituted heteroarylene group, 1 the substituent is selected from the group consisting of an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen atom, a cyano group, an amino group, a carbonyl group, a carboxy group, an oxycarbonyl group, a carbamoyl group, and -P(=O)R¹R², and R1 and R² are each an aryl group or a heteroaryl group, and R¹ and R² are optionally condensed to form a ring; R²² to R²⁶ are each independently selected from the group consisting of a hydrogen atom, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen atom, a cyano group, an amino group, a carbonyl group, a carboxy group, an oxycarbonyl group, a carbamoyl group, and -P(=O)R²⁷R²⁸, wherein R²⁷ and R²⁸ are each an aryl group or a heteroaryl group, and R²⁷ and R²⁸ are optionally condensed to form a ring.

### [Advantageous Effects of Invention]

According to the present invention, it is possible to provide an organic thin film luminescent element that is compatible among the high luminous efficiency, low driving voltage, and long durability life, or a photoelectric conversion element having high conversion efficiency and high on/off ratio.

### [Description of Embodiments]

### <Compound Represented by General Formula (1)>

A compound represented by the general formula (1), which does not fall under the scope of the present invention, will be described in detail, which contributes to a better understanding of the present invention.
[Chemical Formula 4]

**Cz-L¹-Q** **(1)**

In the formula (1), Cz is a group represented by the following general formula (2), Q is a group represented by the following general formula (3), and L¹ is a single bond or a substituted or unsubstituted arylene group. When L¹ is a single bond, Cz and Q are directly bonded to each other.

When L¹ is a substituted arylene group, the substituent is selected from the group consisting of an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen atom, a cyano group, an amino group, a carbonyl group, a carboxy group, an oxycarbonyl group, a carbamoyl group, and -P(=O)R¹R².

R¹ and R² are each an aryl group or a heteroaryl group, and R¹ and R² are optionally condensed to form a ring.

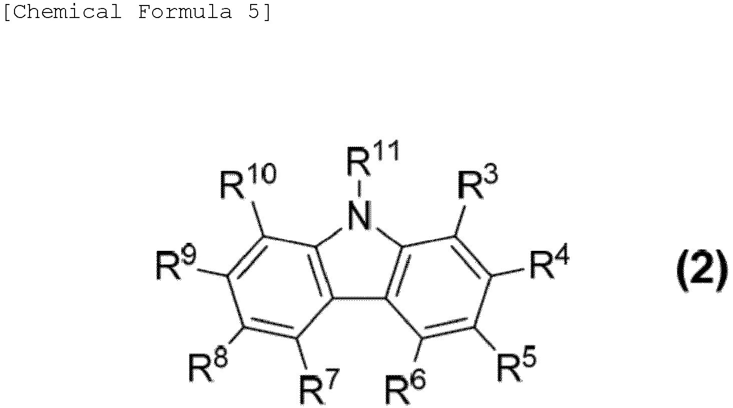

In the formula (2), R³ to R¹⁰ are each independently selected from the group consisting of a hydrogen atom, an alkyl group, a heterocyclic group, an alkenyl group, an oxygen-containing aromatic heterocyclic group, a sulfur-containing aromatic heterocyclic group, a halogen atom, and a cyano group. The group represented by the general formula (2) is linked to L¹ at any one position of R³ to R⁶.

R¹¹ is selected from the group consisting of a hydrogen atom, an alkyl group, a cycloalkyl group, a heterocyclic group, an alkenyl group, a cycloalkenyl group, an aryl group, and a heteroaryl group.

Adjacent substituents of R³ to R¹¹ optionally form a ring with each other.

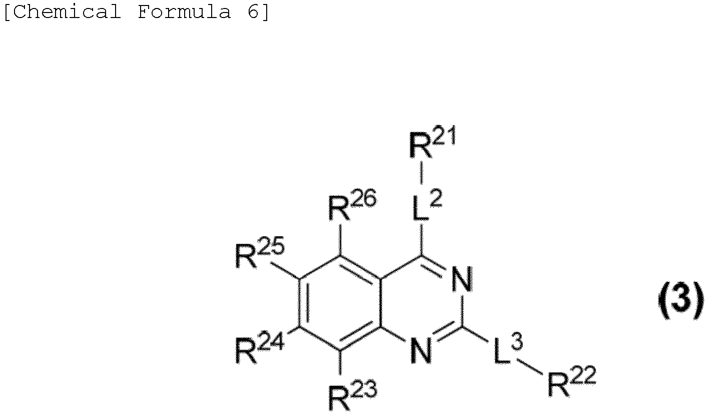

In the formula (3), L² is a single bond, a phenylene group, a naphthalenylene group, or a biphenylene group.

R²¹ is a hydrogen atom, a halogen atom, a carbazolyl group, a benzocarbazolyl group, a dibenzofuranyl group, or a dibenzothiophenyl group.

L³ is a single bond, a substituted or unsubstituted arylene group, or a substituted or unsubstituted heteroarylene group. When L³ is a substituted arylene group or a substituted heteroarylene group, the substituent is independently selected from the same group of substituents as that of a case where L¹ has a substituent.

R²² to R²⁶ are each independently selected from the group consisting of a hydrogen atom, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen atom, a cyano group, an amino group, a carbonyl group, a carboxy group, an oxycarbonyl group, a carbamoyl group, and -P(=O)R²⁷R²⁸. R²⁷ and R²⁸ are each an aryl group or a heteroaryl group, and R²⁷ and R²⁸ are optionally condensed to form a ring. Adjacent substituents of R²³ to R²⁸ optionally form a ring with each other.

The group represented by the general formula (3) is linked to L¹ at any one position of R²³ to R²⁶.

In all of the above groups, hydrogen may be deuterium. In the following description, for example, a "substituted or unsubstituted aryl group having 6 to 40 carbon atoms" has 6 to 40 carbon atoms inclusive of carbon atoms contained in the substituent substituted for the aryl group, and this also applies to other substituents whose number of carbon atoms is defined.

Further, as the substituent in the case of "substituted or unsubstituted", an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen, a cyano group, an amino group, a carbonyl group, a carboxy group, an oxycarbonyl group, and a carbamoyl group are preferable, and specific substituents mentioned as being preferable in the description of each substituent are preferable. In addition, these substituents may be further substituted with the above-mentioned substituents.

In the compound or a partial structure thereof described below, the "substituted or unsubstituted" has the same meaning as described above.

The alkyl group represents, for example, a saturated aliphatic hydrocarbon group such as a methyl group, an ethyl group, an n-propyl group, an isopropyl group, an n-butyl group, a sec-butyl group, or a tert-butyl group, which may or may not have a substituent. There is no particular limitation on the added substituent when the alkyl group is substituted, and examples thereof include an alkyl group, an aryl group, and a heteroaryl group. This point applies also to the following description. There is no particular limitation on the number of carbon atoms of the alkyl group, and the number of carbon atoms is preferably in the range of 1 or more and 20 or less, more preferably 1 or more and 8 or less, in view of availability and costs.

The cycloalkyl group represents, for example, a saturated alicyclic hydrocarbon group such as a cyclopropyl group, a cyclohexyl group, a norbornyl group, or an adamantyl group, which may or may not have a substituent. There is no particular limitation on the number of carbon atoms of the alkyl group moiety, and the number of carbon atoms is preferably in the range of 3 or more and 20 or less.

The heterocyclic group represents, for example, an aliphatic ring having an atom other than carbon in the ring, such as a pyran ring, a piperidine ring, or a cyclic amide, which may or may not have a substituent. The heterocycle may have one or more double bonds in the ring as long as the conjugated system is not linked in a cyclic form to have aromaticity. There is no particular limitation on the number of carbon atoms of the heterocyclic group, and the number of carbon atoms is preferably in the range of 2 or more and 20 or less.

The alkenyl group represents, for example, an unsaturated aliphatic hydrocarbon group having a double bond, such as a vinyl group, an allyl group, or a butadienyl group, which may or may not have a substituent. There is no particular limitation on the number of carbon atoms of the alkenyl group, and the number of carbon atoms is preferably in the range of 2 or more and 20 or less.

The cycloalkenyl group represents, for example, an unsaturated alicyclic hydrocarbon group having a double bond, such as a cyclopentenyl group, a cyclopentadienyl group, or a cyclohexenyl group, which may or may not have a substituent. There is no particular limitation on the number of carbon atoms of the cycloalkenyl group, and the number of carbon atoms is preferably in the range of 4 or more and 20 or less.

The alkynyl group represents, for example, an unsaturated aliphatic hydrocarbon group having a triple bond, such as an ethynyl group, which may or may not have a substituent. There is no particular limitation on the number of carbon atoms of the alkynyl group, and the number of carbon atoms is preferably in the range of 2 or more and 20 or less.

The alkoxy group represents, for example, a functional group in which aliphatic hydrocarbon groups are bonded through an ether bond, such as a methoxy group, an ethoxy group, or a propoxy group, and this aliphatic hydrocarbon group may or may not have a substituent. There is no particular limitation on the number of carbon atoms of the alkoxy group, and the number of carbon atoms is preferably in the range of 1 or more and 20 or less.

The alkylthio group is a group resulting from substitution of an oxygen atom of an ether bond of an alkoxy group with a sulfur atom. A hydrocarbon group of the alkylthio group may or may not have a substituent. There is no particular limitation on the number of carbon atoms of the alkylthio group, and the number of carbon atoms is preferably in the range of 1 or more and 20 or less.

The aryl ether group represents, for example, a functional group in which aromatic hydrocarbon groups are bonded through an ether bond, such as a phenoxy group, and the aromatic hydrocarbon group may or may not have a substituent. There is no particular limitation on the number of carbon atoms of the aryl ether group, and the number of carbon atoms is preferably in the range of 6 or more and 40 or less.

The aryl thioether group is a group resulting from substitution of an oxygen atom of an ether bond of an aryl ether group with a sulfur atom. An aromatic hydrocarbon group of the aryl thioether group may or may not have a substituent. There is no particular limitation on the number of carbon atoms of the aryl ether group, and the number of carbon atoms is preferably in the range of 6 or more and 40 or less.

The aryl group represents, for example, an aromatic hydrocarbon group such as a phenyl group, a naphthyl group, a biphenyl group, a terphenyl group, a phenanthryl group, an anthracenyl group, a pyrenyl group, or a fluoranthenyl group. The aryl group may or may not have a substituent. There is no particular limitation on the number of carbon atoms of the aryl group, and the number of carbon atoms is preferably in the range of 6 or more and 40 or less, more preferably in the range of 6 or more and 24 or less. The aryl group is still more preferably a phenyl group, a 1-naphthyl group, or a 2-naphthyl group.

The heteroaryl group represents a cyclic aromatic group having one or more atoms other than carbon in the ring, such as a furanyl group, a thiophenyl group, a pyridyl group, a quinolinyl group, an isoquinolinyl group, a pyrazinyl group, a pyrimidyl group, a naphthylidyl group, a benzofuranyl group, a benzothiophenyl group, an indolyl group, a dibenzofuranyl group, a dibenzothiophenyl group, or a carbazolyl group, which may or may not be substituted. There is no particular limitation on the number of carbon atoms of the heteroaryl group, and the number of carbon atoms is preferably in the range of 2 or more and 30 or less. More preferably, the heteroaryl group is a pyridyl group, a quinolyl group, a carbazolyl group, a dibenzofuranyl group, or a dibenzothiophenyl group.

The amino group is a substituted or unsubstituted amino group. Examples of the substituent when the amino group is a substituted amino group include an aryl group, a heteroaryl group, a linear alkyl group, and a branched alkyl group. More specific examples of the substituent include a phenyl group, a biphenyl group, a naphthyl group, a pyridyl group, and a methyl group, and these substituents may be further substituted. There is no particular limitation on the number of carbon atoms of the amino group, and the number of carbon atoms is preferably in the range of 6 or more and 40 or less.

The halogen atom represents an atom selected from fluorine, chlorine, bromine, and iodine.

The carbonyl group, the carboxy group, the oxycarbonyl group, the carbamoyl group, and the phosphine oxide group may or may not have a substituent. Examples of the substituent include an alkyl group, a cycloalkyl group, an aryl group, and a heteroaryl group, and these substituents may be further substituted.

The arylene group represents a divalent or higher-valent group derived from an aromatic hydrocarbon group such as benzene, naphthalene, biphenyl, fluorene, and phenanthrene, which may or may not have a substituent. The arylene group is preferably a divalent or trivalent arylene group. Specific examples of the arylene group include a phenylene group, a biphenylene group, a naphthylene group, and a fluorenylene group. More specific examples of the arylene group include a 1,4-phenylene group, a 1,3-phenylene group, a 1,2-phenylene group, a 4,4'-biphenylene group, a 4,3'-biphenylene group, a 3,3'-biphenylene group, a 1,4-naphthalenylene group, a 1,5-naphthalenylene group, a 2,5-naphthalenylene group, a 2,6-naphthalenylene group, a 2,7-naphthalenylene group, and a 1,3,5-phenylene group. More preferable examples of the arylene group include a 1,4-phenylene group, a 1,3-phenylene group, a 4,4'-biphenylene group, and a 4,3'-biphenylene group.

The heteroarylene group represents a divalent or higher-valent group derived from an aromatic group having one or more atoms other than carbon in the ring, such as pyridine, quinoline, pyrimidine, pyrazine, triazine, quinoxaline, quinazoline, dibenzofuran, and dibenzothiophene, which may or may not have a substituent. The heteroarylene group is preferably a divalent or trivalent heteroarylene group. There is no particular limitation on the number of carbon atoms of the heteroarylene group, and the number of carbon atoms is preferably in the range of 2 to 30. Specific examples of the heteroarylene group include a 2,6-pyridylene group, a 2,5-pyridylene group, a 2,4-pyridylene group, a 3,5-pyridylene group, a 3,6-pyridylene group, a 2,4,6-pyridylene group, a 2,4-pyrimidinylene group, a 2,5-pyrimidinylene group, a 4,6-pyrimidinylene group, a 2,4,6-pyrimidinylene group, a 2,4,6-triazinylene group, a 4,6-dibenzofuranylene group, a 2,6-dibenzofuranylene group, a 2,8-dibenzofuranylene group, and a 3,7-dibenzofuranylene group.

The oxygen-containing aromatic heterocyclic group is a monovalent or higher-valent ring structure that includes one or more carbon atoms and one or more oxygen atoms in the ring, and that is considered to be aromatic from the Huckel's rule, such as furan, benzofuran, and dibenzofuran. These may or may not have a substituent. There is no particular limitation on the number of carbon atoms of the oxygen-containing aromatic heterocyclic group, and the number of carbon atoms is preferably in the range of 4 to 20.

The sulfur-containing aromatic heterocyclic group is a monovalent or higher-valent ring structure that includes one or more carbon atoms and one or more sulfur atoms in the ring, and that is considered to be aromatic from the Huckel's rule, such as thiophene, benzothiophene, and dibenzothiophene. These may or may not have a substituent. There is no particular limitation on the number of carbon atoms of the sulfur-containing aromatic heterocyclic group, and the number of carbon atoms is preferably in the range of 4 to 20.

The quinazoline skeleton has two nitrogen atoms having high electronegativity and having an sp² hybridized orbital. Thus, the quinazoline skeleton has high affinity with electrons and high electron mobility. Therefore, when the compound of the present invention is used in an electron injection layer of a luminescent element, electron injection from the negative electrode is likely to occur. In addition, when the compound of the present invention is used in an electron transport layer, the layer exhibits a high electron transport property.

The lowest unoccupied molecular orbital (LUMO) energy of a molecule consisting only of the quinazoline skeleton, however, is too low compared to that of a general luminescent layer, so that it is difficult to inject electrons into the luminescent layer when a compound having the molecule is applied to a luminescent element. Therefore, into the compound of the present invention, a carbazole skeleton having an effect of increasing the LUMO energy and also having electron transport ability is introduced.

Moreover, when the compound of the present invention is used in an electron extraction layer of a photoelectric conversion element, high electron extraction efficiency is achieved since the compound has a lower LUMO energy than that of the n-type material in the photoelectric conversion layer.

Further, the carbazole skeleton and the quinazoline skeleton have high charge mobility in the skeletons. Combination of these two skeletons extend the highest occupied molecular orbital (HOMO) and LUMO in the molecule. When the HOMO and LUMO largely extend, the orbitals largely overlap between adjacent molecules, and the charge transport property is improved.

Therefore, use of the compound of the present invention in any of layers constituting a luminescent element can lower the driving voltage of the luminescent element since electrons generated from the negative electrode and holes generated from the positive electrode can be efficiently transported. As a result, the luminous efficiency of the luminescent element can be improved.

In addition, use of the compound of the present invention in an electron extraction layer of a photoelectric conversion element enables prompt transport of electrons generated in the photoelectric conversion layer without loss, and thus an element having high photoelectric conversion efficiency can be produced.

Further, since the orbitals extend in the molecule, radicals generated upon receipt of the electric charge are stabilized, and the durability life of the luminescent element is improved. In addition, since the carbazole skeleton and the quinazoline skeleton have high stability to electric charge, that is, high electrochemical stability, and can perform reduction by electrons and oxidation by holes rapidly and reversibly, the luminescent element and the photoelectric conversion element containing the compound of the present invention are improved in the durability life.

Since both the carbazole skeleton and the quinazoline skeleton are composed of a plurality of rigid ring structures, a compound having these skeletons exhibits a high glass transition temperature. Moreover, due to the rigid ring structures, a compound having these skeletons does not get intertwined between molecules and stably sublimes. Since the compound has a high glass transition temperature, the heat resistance of the luminescent element is improved, and a good film quality formed by stable sublimation is also maintained, so that the life of the luminescent element and the photoelectric conversion element can be prolonged.

As described above, the compound of the present invention has both a carbazole skeleton and a quinazoline skeleton in the molecule, whereby all of high luminous efficiency, low driving voltage, and long durability life can be achieved.

In particular, the compound of the present invention is preferably used in a luminescent layer or an electron transport layer of a luminescent element because of its characteristics. Above all, the compound of the present invention is particularly preferably used in an electron transport layer.

The positions of bonding of the carbazole skeleton and the quinazoline skeleton are any one position of R³ to R⁶ in the general formula (2) for the carbazole skeleton and any one position of R²³ to R²⁶ in the general formula (3) for the quinazoline skeleton.

The positions of R³ to R⁶ of the carbazole skeleton are positions on a six-membered ring that does not include a nitrogen atom in the carbazole skeleton. When the bond to the quinazoline skeleton is present at any of these positions, the crystallinity of the compound is lowered and the stability of the film is improved compared to the case where the skeletons are bonded through a nitrogen atom.

The positions of R²³ to R²⁶ of the quinazoline skeleton are positions on a six-membered ring that does not include an electron-attracting nitrogen atom in the quinazoline skeleton. When the bond to the carbazole skeleton is present at any of these positions, the distance between the electron-donating nitrogen of the carbazole skeleton and the electron-attracting nitrogen of the quinazoline skeleton increases in the molecule. As the distance increases, polarization in the molecule due to pushing and pulling of electrons decreases. As a result, no electron trap level is formed, and high electron mobility is achieved.

To enhance these effects, it is more preferable that the carbazole skeleton be bonded to L¹ at the position of R⁴ or R⁵ in the general formula (2). Further, it is more preferable that the quinazoline skeleton be linked to L¹ at the position of R²⁴ or R²⁵ in the general formula (3), and it is particularly preferable that the quinazoline skeleton be linked to L¹ at the position of R²⁵.

R³ to R¹⁰ on the carbazole skeleton are each independently selected from the group consisting of a hydrogen atom, an alkyl group, a heterocyclic group, an alkenyl group, an oxygen-containing aromatic heterocyclic group, a sulfur-containing aromatic heterocyclic group, a halogen atom, and a cyano group. Substituents other than a hydrogen atom are introduced to modify the electronic state of the carbazole skeleton. For example, an electron-donating substituent such as an oxygen-containing aromatic heterocyclic group and a sulfur-containing aromatic heterocyclic group has an action of reinforcing the electron-donating property of the carbazole skeleton, and an electron-attracting group such as a cyano group has an action of reducing the electron-donating property of the carbazole skeleton.

As already described, a material consisting only of a quinazoline skeleton has a low energy level of the LUMO orbital and may inhibit electron injection into the luminescent layer, and thus the compound of the present invention contains a carbazolyl group that has an effect of increasing the LUMO level. Accordingly, for the substituent of the carbazole skeleton, a substituent that does not inhibit or that reinforces the electron-donating property of carbazole is preferable. Therefore, among these substituents, R³ to R¹⁰ are more preferably each a hydrogen atom, an oxygen-containing aromatic heterocyclic group, or a sulfur-containing aromatic heterocyclic group, still more preferably a hydrogen atom, a dibenzofuranyl group, or a dibenzothiophenyl group, most preferably a hydrogen atom.

R¹¹ is preferably an aryl group. Specifically, R¹¹ is preferably a phenyl group, a biphenyl group, a naphthyl group, a terphenyl group, a 9,9-dialkylfluorenyl group, an anthracenyl group, or a pyrenyl group, more preferably a phenyl group, a biphenyl group, a naphthyl group, a 9,9-dialkylfluorenyl group, an anthracenyl group, or a pyrenyl group, still more preferably a phenyl group or a naphthyl group, particularly preferably a phenyl group. These groups may have a phenyl group as an additional substituent.

L¹ is a single bond or a substituted or unsubstituted arylene group. Since these groups do not affect the LUMO level of the molecule, they can maintain an appropriate LUMO level that is achieved by the combination of the carbazole skeleton and the quinazoline skeleton as described above.

When L¹ is a heteroarylene group, there is a concern that L¹ may affect the LUMO level of the molecule and make the LUMO level too low. If the LUMO level is too low, an energy difference is generated between the LUMO level of the molecule and the LUMO level of the host material of the luminescent layer, and this may inhibit the injection of electrons into the luminescent layer.

When L¹ is a substituted arylene group, the substituent is as described above. The substituent is preferably an alkyl group, a cycloalkyl group, a heterocyclic group, an aryl ether group, an aryl thioether group, an aryl group, a heteroaryl group, a halogen atom, a cyano group, or an amino group, more preferably a heterocyclic group, an aryl group, a heteroaryl group, a halogen atom, a cyano group, or an amino group, still more preferably an aryl group, a heteroaryl group, or an amino group.

L¹ is more preferably a single bond or an arylene group having 6 to 24 carbon atoms, still more preferably a single bond, a phenyl group, or a biphenyl group.

R²³ to R²⁶ at positions not linked to L¹ are as described above. R²³ to R²⁶ are preferably each a hydrogen atom, a cycloalkyl group, a heterocyclic group, an aryl ether group, an aryl thioether group, an aryl group, a heteroaryl group, a halogen atom, a cyano group, or an amino group, more preferably a hydrogen atom, an aryl group, a heteroaryl group, a halogen atom, a cyano group, or an amino group, still more preferably a hydrogen atom, an aryl group, or a heteroaryl group.

Since L²-R²¹ is linked to a ring having two nitrogen atoms in the quinazoline skeleton, it is preferable to adjust the electronic state of the compound of the present invention with a substituent at this position. Therefore, it is preferable that L² be a phenylene group, a naphthalenylene group, or a biphenylene group, and R²¹ be an electrically neutral hydrogen atom, an electron-attracting halogen atom, or an electron-donating carbazolyl group, benzocarbazolyl group, dibenzofuranyl group, or dibenzothiophenyl group. It is particularly preferable that L²-R²¹ be an electrically neutral phenyl group, biphenyl group, or naphthyl group, or be a fluorophenyl group that has an electron-attracting property by an induction effect as well as an electron-donating property by a resonance effect, particularly a phenyl group.

The position of L³-R²² is the bonding position between two nitrogen atoms in the quinazoline skeleton. Since no hydrogen atom is bonded to these two nitrogen atoms, the substituent bonded to the position of L³-R²² is not so susceptible to distortion of the structure. Thus, when L³-R²² has a conjugated structure, the triplet excitation energy of the molecule tends to be low since the orbital of the quinazoline skeleton and the orbital of the conjugated system of L³-R²² effectively overlap each other. If the triplet excitation energy is low, when the compound of the present invention is used in a luminescent element, the effect of confining, into the luminescent layer, the excitons generated in the luminescent layer is reduced, and consequently the luminous efficiency is lowered due to exciton deactivation.

Therefore, it is preferable to introduce, into the position of L³-R²², an aromatic ring having a small conjugated system and a large triplet excitation energy, such as a phenyl group, or a substituent having a large triplet excitation energy due to a distorted ring structure, such as a carbazolyl group. That is, it is preferable that L³ be a single bond, a phenylene group, or a biphenylene group, and R²² be a phenyl group, a naphthyl group, an anthracenyl group, a pyrenyl group, a terphenyl group, a 9,9-dialkylfluorenyl group, a carbazolyl group, a benzocarbazolyl group, a dibenzofuranyl group, or a dibenzothiophenyl group. It is more preferable that R²² be a phenyl group, a naphthyl group, a terphenyl group, a 9,9-dialkylfluorenyl group, a carbazolyl group, a benzocarbazolyl group, a dibenzofuranyl group, or a dibenzothiophenyl group.

More specifically, it is more preferable that L³ be a single bond, a 1,4-phenylene group, a 1,3-phenylene group, a 4,4'-biphenylene group, or a 4,3'-biphenylene group, and it is particularly preferable that L³ be a single bond, a 1,4-phenylene group, or a 1,3-phenylene group. It is more preferable that R²² be a phenyl group, a 1-naphthyl group, a 2-naphthyl group, an m-terphenyl group, a 9,9-dimethylfluorenyl group, a 3-carbazolyl group, an N-carbazolyl group, an N-benzocarbazolyl group, a 2-dibenzofuranyl group, or a 2-dibenzothiophenyl group, and it is particularly preferable that R²² be a phenyl group, a 9,9-dimethylfluorenyl group, or an m-terphenyl group.

When L³-R²² is an m-terphenyl group having a large triplet excitation energy, the luminescent element tends to be prolonged in life. When L³-R²² is a dibenzofuranyl group having an oxygen atom that has higher electronegativity than nitrogen does and has high electron affinity, the luminescent element tends to have lower voltage. When L³-R²² includes a carbazolyl group having high electron injection efficiency into the luminescent layer, the luminescent element tends to have high efficiency.

At the positions of R³ to R¹⁰ on the carbazole skeleton and the positions of R²³ to R²⁶ on the quinazoline skeleton, adjacent substituents may form a ring. The size of the ring formed by the adjacent substituents is not particularly limited. From the viewpoint of stability of the molecular structure, the ring is preferably a 5-membered ring or a 6-membered ring. Further, the formed ring may be either of an aliphatic ring and an aromatic ring. The ring formed by the adjacent substituents may further have a substituent or may be further condensed. The formed ring may include a heteroatom other than carbon. Examples of the heteroatom other than carbon include a nitrogen atom. In particular, it is preferable that the ring be consisting only of carbon and hydrogen, since the electrochemical stability is improved and this contributes to improvement in durability of the element.

When R³ to R¹⁰ and/or R²³ to R²⁶ are substituents, the number of carbon atoms of each substituent is not particularly limited, but is preferably in the range of 1 or more and 24 or less, more preferably 1 or more and 12 or less.

According to the present invention, the compound represented by the general formula (1) is a compound represented by the general formula (4) or (5).

The electrons of the substituent linked to the position of R²² on the quinazoline skeleton in the general formula (3) are attracted by two nitrogen atoms having high electronegativity in the quinazoline skeleton. As a result, an electric dipole (electric dipole 1) having a partial negative charge on the two nitrogen atoms and a partial positive charge on R²² in the quinazoline skeleton is generated.

Meanwhile, when the quinazoline skeleton is linked to the carbazole skeleton at any position of R²³ to R²⁶, a partial positive charge is generated on the carbazole skeleton due to its electron-donating property, and a partial negative charge is generated on the two nitrogen atoms having high electronegativity in the quinazoline skeleton. An electric dipole (electric dipole 2) formed in this case has a vector component opposite in direction to the electric dipole 1. These electric dipoles having vector components opposite in direction cancel each other in the molecule, and the entire molecule has a smaller electric dipole. As a result, reduction in charge mobility due to generation of the charge trap level is suppressed, and high charge transport ability is achieved.

Among R²³ to R²⁶, the carbazole skeleton is particularly preferably bonded to the position of R²⁵, since the directions of the electric dipole 1 and the electric dipole 2 are closest to the completely opposite directions, and thus the dipoles cancel each other most effectively, and generation of the charge trap level is minimized.

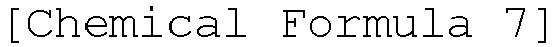

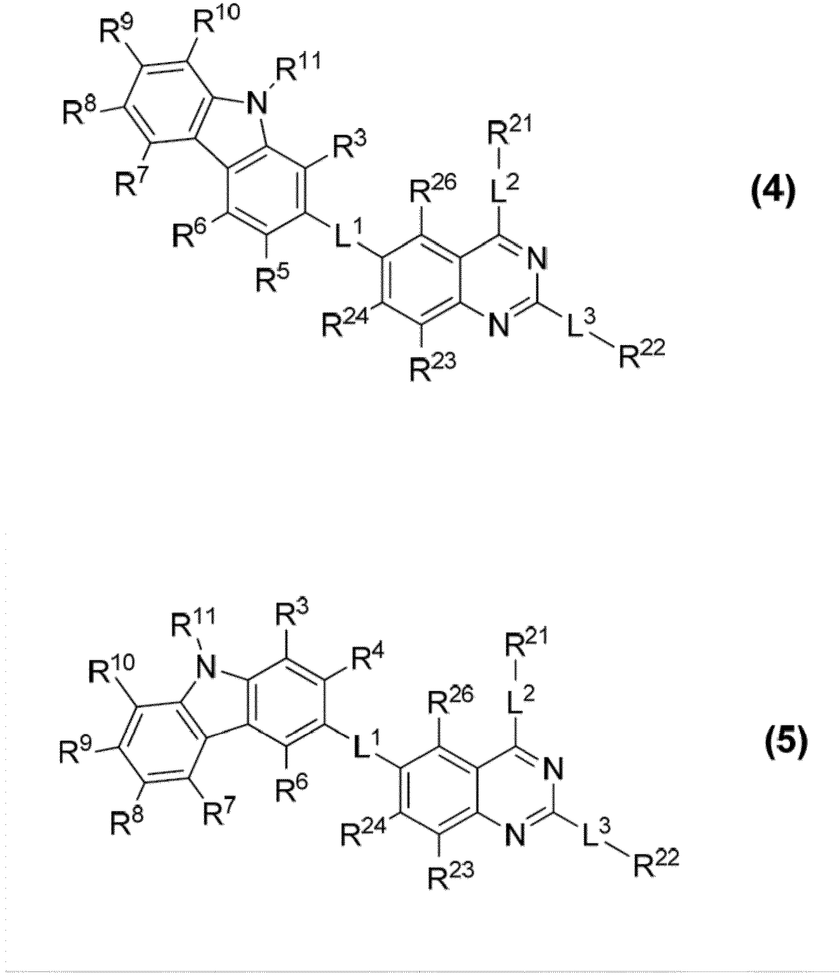

Each R and each L in the formulae are as described above.

R³ to R¹⁰ in the general formulae (4), and (5) are each a hydrogen atom except for the position linked to L¹. When R³ to R¹⁰ are each a hydrogen atom, the intermolecular distance in the film is short, and charge transfer can be efficiently performed. As a result, it is possible to improve the luminous efficiency of the luminescent element by low voltage driving, and to improve the photoelectric conversion efficiency of the photoelectric conversion element. Further, when R³ to R¹⁰ are each a hydrogen atom, the molecular weight can be suppressed, and as a result, the compound can sublime at a lower temperature, and vapor deposition stability is also improved.

For the same reason, it is particularly preferable that R²³ to R²⁶ in the general formulae (3), (4), and (5) be each a hydrogen atom except for the position linked to L¹.

In the general formulae (2), (4), and (5), it is particularly preferable that adjacent substituents of R³ to R¹¹ do not form a ring with each other. When the substituents do not form a ring structure, the crystallinity is not too high, the thin film stability is improved, and as a result, the life of the luminescent element and the photoelectric conversion element is improved. Further, the molecular weight of molecules for forming the ring structure can be suppressed, and as a result, the compound can sublime at a lower temperature, and vapor deposition stability is also improved.

For the same reason, it is preferable that adjacent substituents of R²³ to R²⁶ do not form a ring with each other in the general formulae (3), (4), and (5).

The LUMO energy of the compound represented by the general formula (1) is slightly lower than the LUMO energy of an anthracene skeleton. Therefore, in a luminescent element, it is preferable to use the compound represented by the general formula (1) in a luminescent layer containing a compound having an anthracene skeleton that is in contact with the negative electrode, since injection of electrons into the luminescent layer is likely to occur.

In addition, the compound represented by the general formula (1) is easy to receive electrons from a compound having a phenanthroline skeleton. A compound having a phenanthroline skeleton can transport electrons at a low driving voltage. Therefore, in a luminescent element, it is preferable to use a compound having a phenanthroline skeleton in a layer containing a compound represented by the general formula (1) that is in contact with the negative electrode, since an element capable of being driven at a low voltage can be produced.

In order to distribute the electric charge and speed up the transfer of electrons, it is more preferable that the compound having a phenanthroline skeleton have a plurality of phenanthroline skeletons in the molecule.

In addition, since the electron-accepting nitrogen has an unshared electron pair on the nitrogen atom, it shows a strong coordination property to metal atoms. Therefore, the quinazoline skeleton having two electron-accepting nitrogen atoms has a strong metal coordination property. Therefore, when the compound represented by the general formula (1) is used in an electron extraction layer of a photoelectric conversion element, the conversion efficiency and the on/off ratio of the photoelectric conversion element can be improved since the compound accelerates the extraction of electrons to the negative electrode.

The compound represented by the general formula (1) is not particularly limited, and specific examples thereof include the following compounds falling under the scope of the present invention.

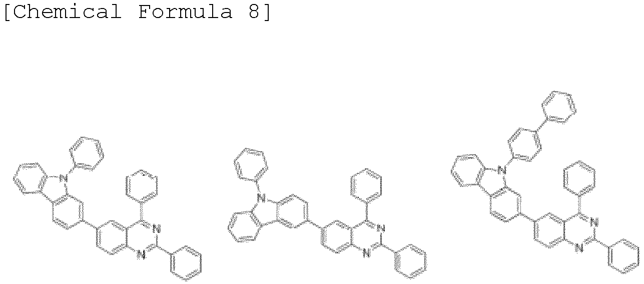

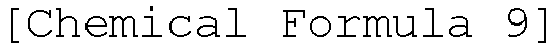

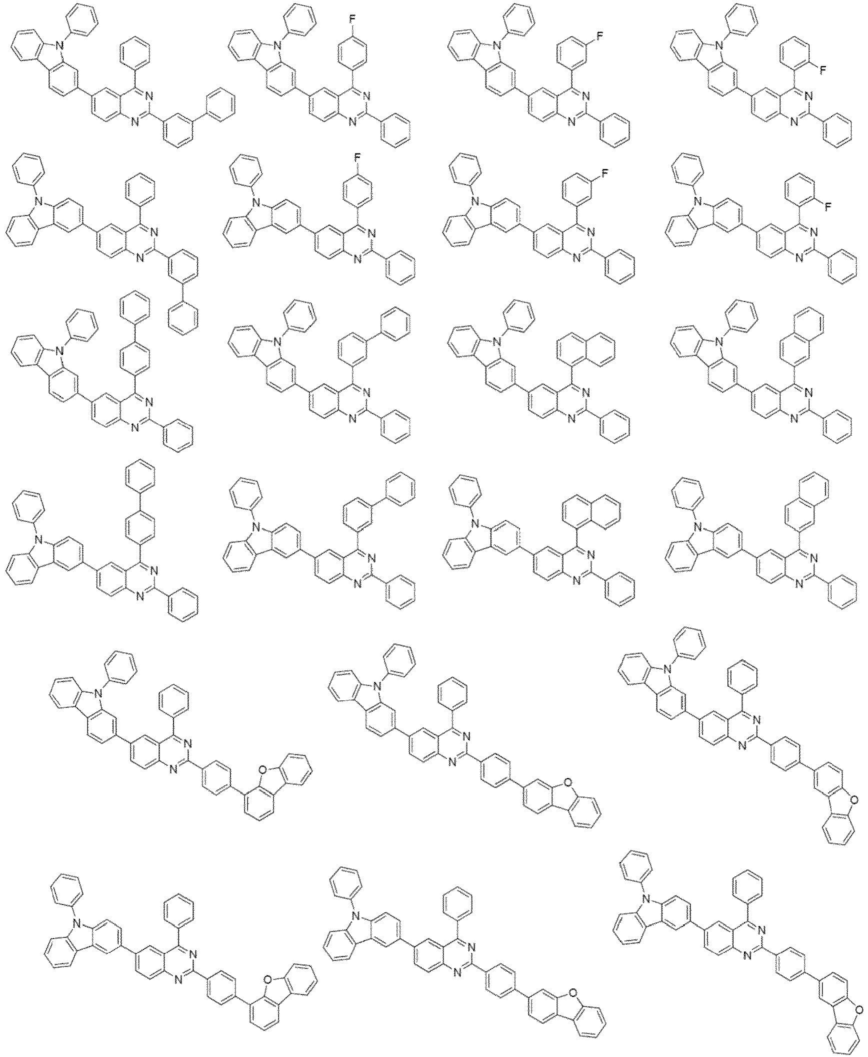

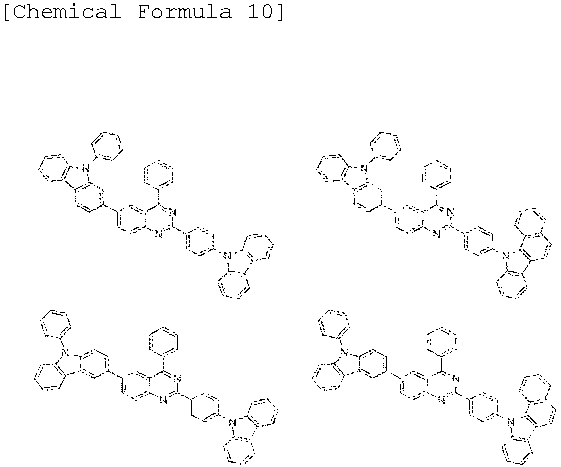

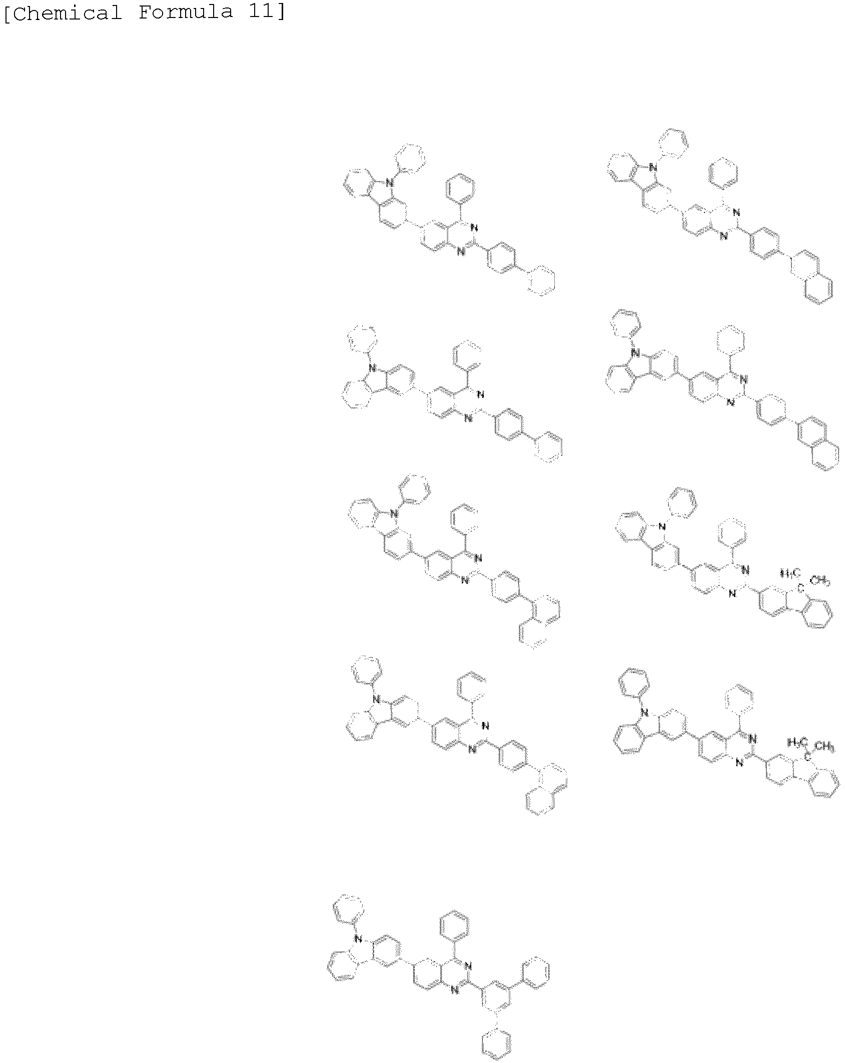

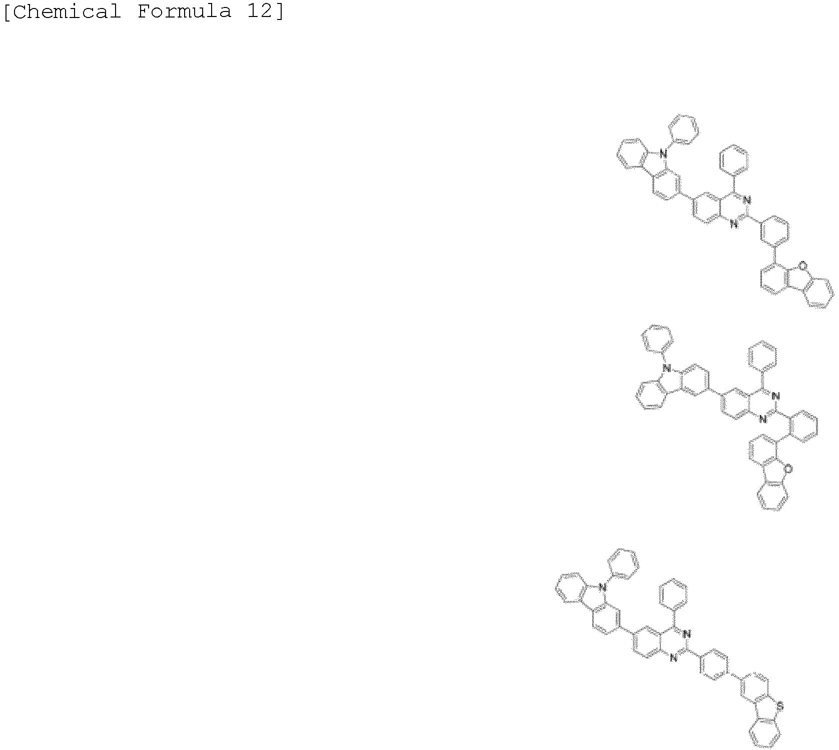

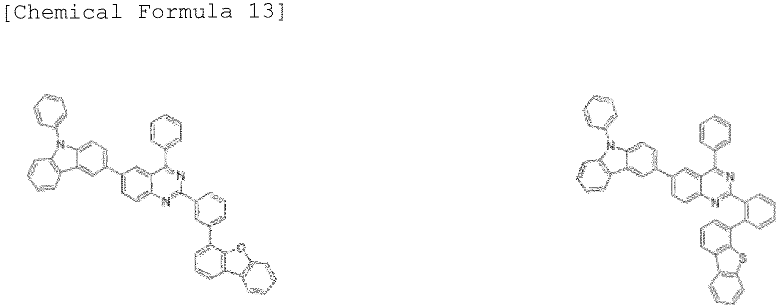

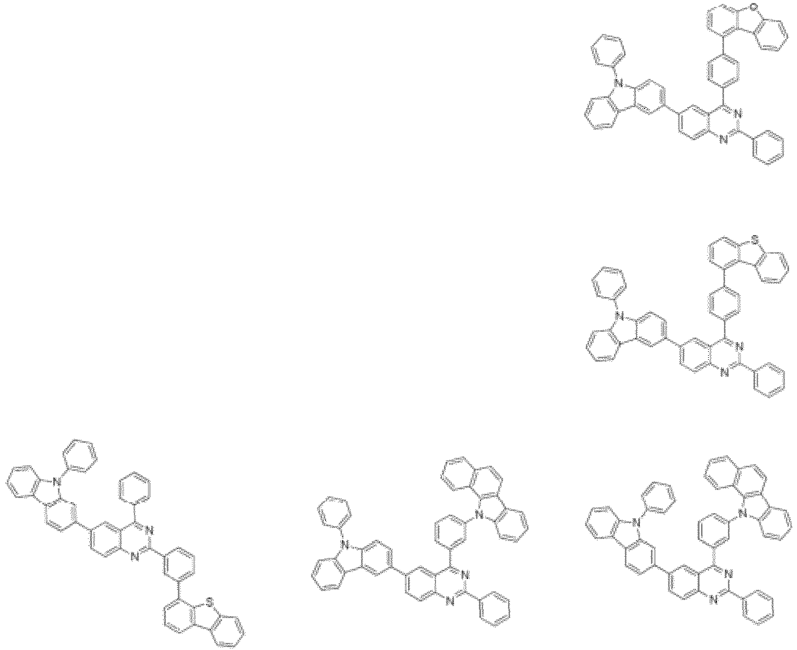

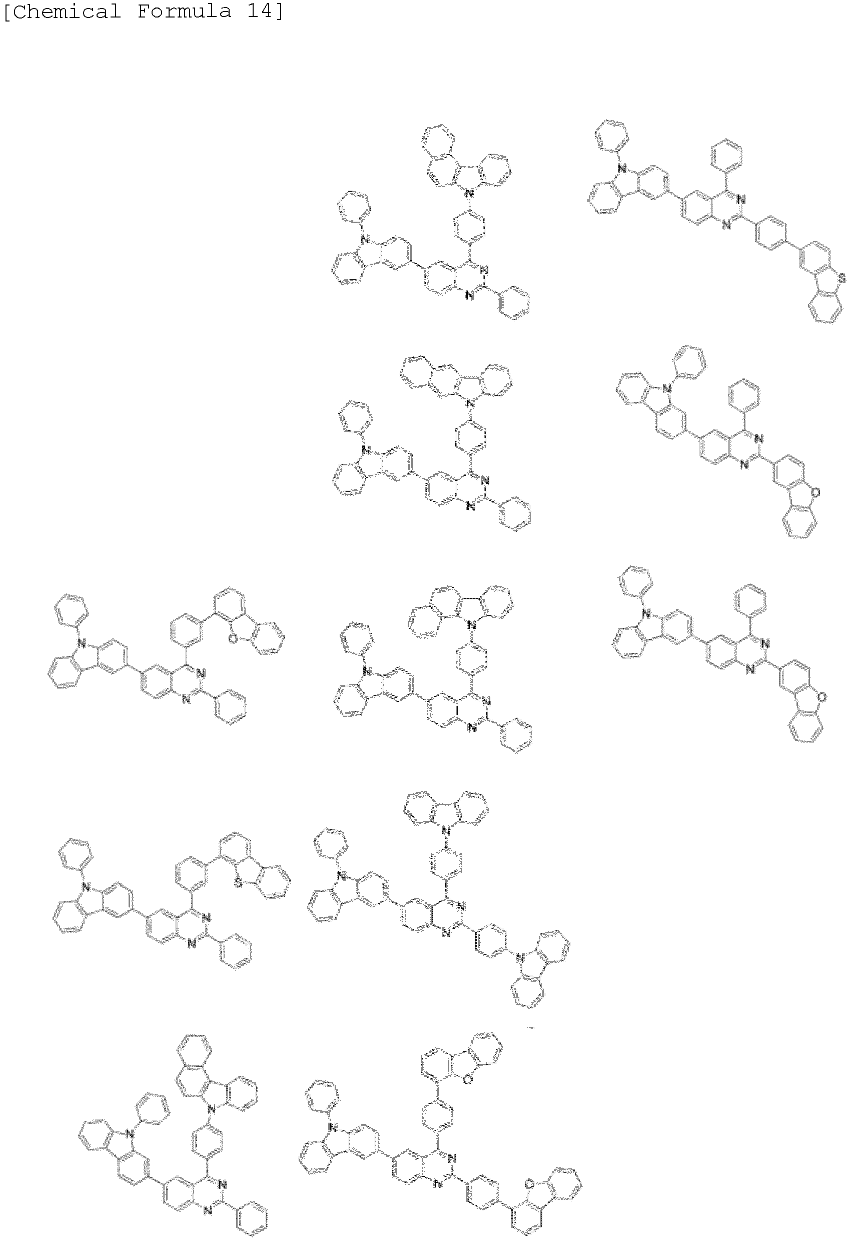

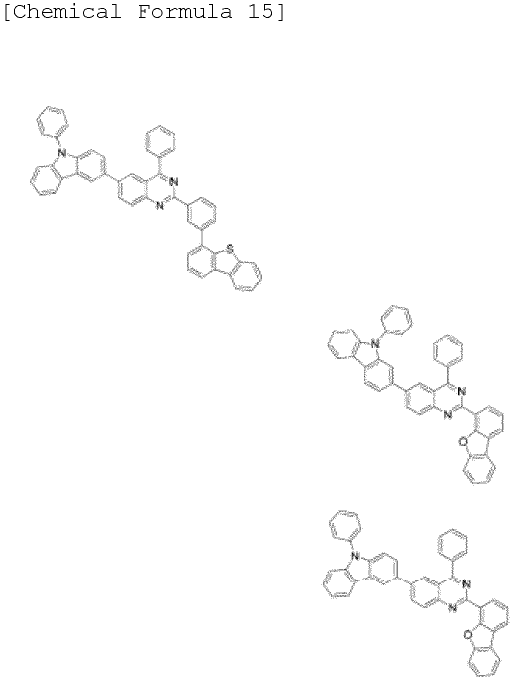

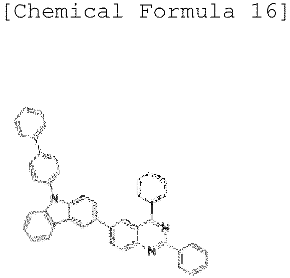

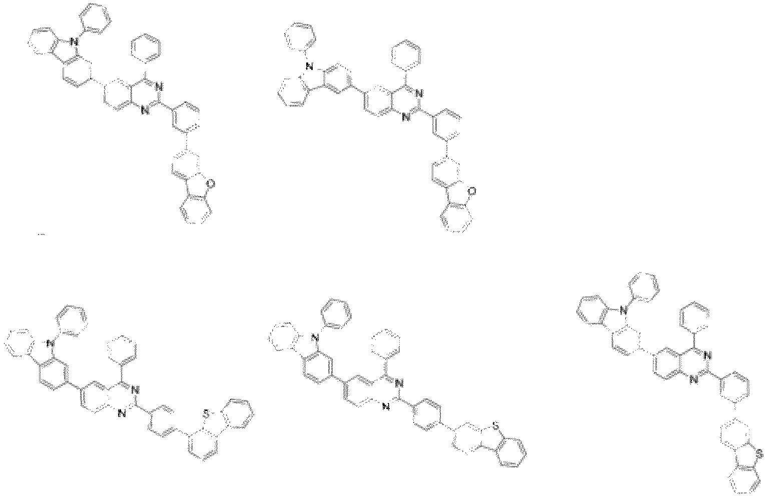

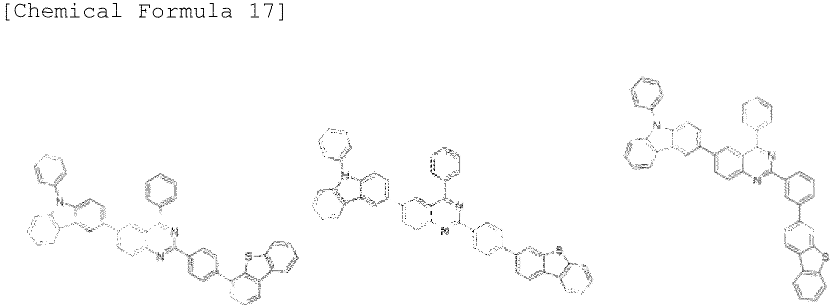

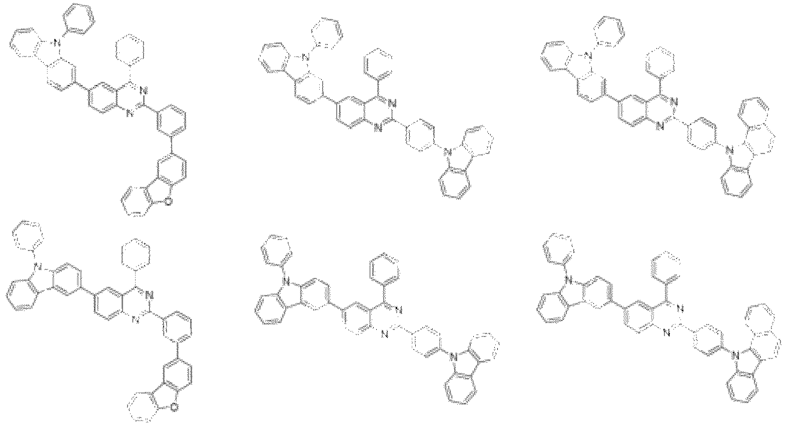

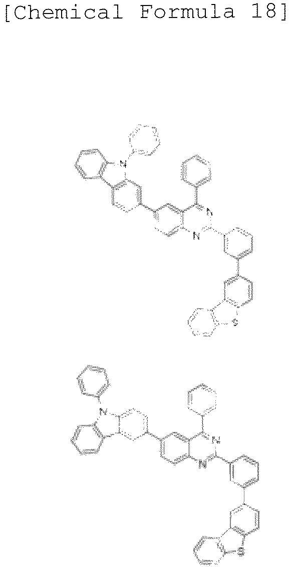

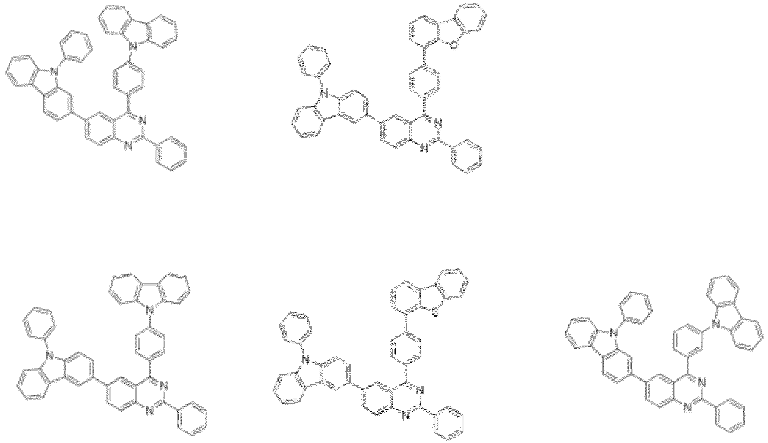

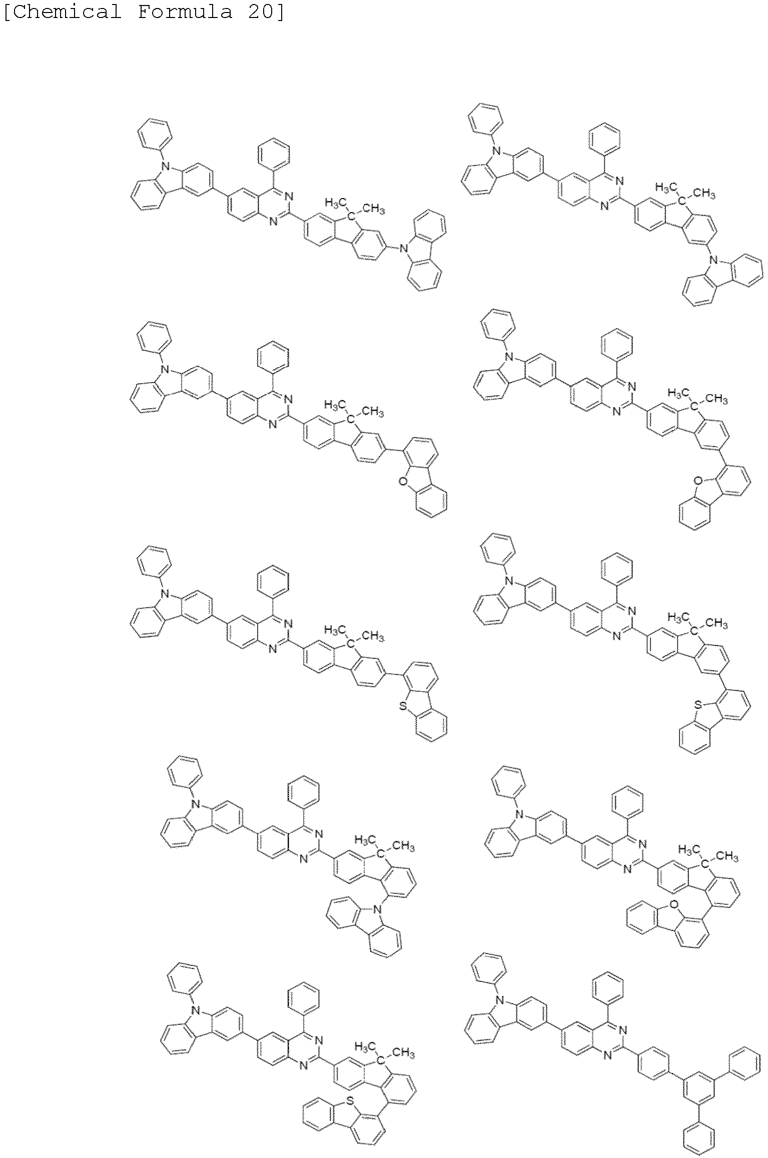

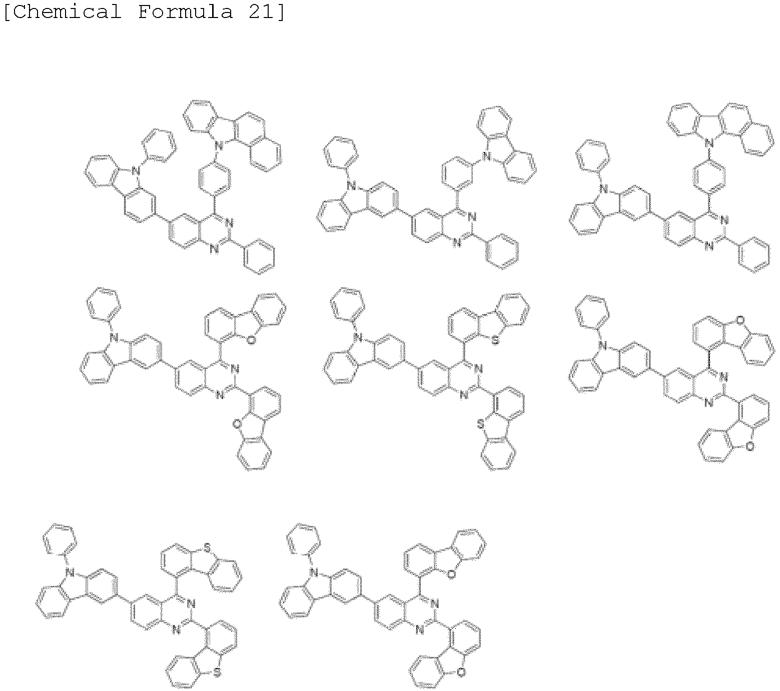

For synthesizing the compound represented by the general formula (1), a known method can be used. For example, a carbazole skeleton to which a halogen atom or boronic acid is bonded is commercially available. Examples of the synthesis method include a method utilizing a coupling reaction between a substituted or unsubstituted carbazolyl boronic acid derivative and a substituted or unsubstituted halogenated quinazoline derivative in the presence of a palladium catalyst or a nickel catalyst, a method utilizing a coupling reaction between a substituted or unsubstituted carbazole derivative and a substituted or unsubstituted halogenated quinazoline derivative, and a coupling reaction between a substituted or unsubstituted halogenated carbazole derivative and a substituted or unsubstituted quinazolinyl boronic acid derivative, but the method is not limited thereto.

A boronic acid ester may be used as the boronic acid, and a halogen atom can be converted into a boronic acid ester by a known method. The quinazoline skeleton can be synthesized by a cyclization reaction from a substituted or unsubstituted 2-aminobenzonitrile derivative and a Grignard reagent using a known method. A commercially available halogenated quinazoline can also be used, but the quinazoline is not limited thereto.

### <Application of Compound Represented by General Formula (1)>

The compound of the present invention is preferably used as an electronic device material in electronic devices such as a luminescent element, a photoelectric conversion element, a lithium ion battery, a fuel cell, and a transistor. In particular, in a luminescent element and a photoelectric conversion element, the compound is preferably used as a luminescent element material or a photoelectric conversion element material.

The luminescent element material is a material used in any layer of a luminescent element, and is a material used in a layer selected from a hole transport layer, a luminescent layer, and an electron transport layer, as well as a material used in a protective layer (cap layer) for an electrode, as described later. Use of the compound of the present invention in any layer of a luminescent element provides a luminescent element having high luminous efficiency as well as low driving voltage and high durability.

### <Photoelectric Conversion Element>

The photoelectric conversion element has an anode and a cathode, and an organic layer interposed between the anode and the cathode. In the organic layer, light energy is converted into an electric signal. It is preferable that the organic layer have at least a photoelectric conversion layer, and it is more preferable that the photoelectric conversion layer contain a p-type material and an n-type material. The p-type material is an electron-donating (donor) material, has a high HOMO energy level, and readily transports holes. The n-type material is an electron-attracting (acceptor) material, has a low LUMO energy level, and readily transports electrons. The p-type material and the n-type material may be laminated or mixed.

As for the lamination structure of the organic layer, in addition to the structure consisting only of the photoelectric conversion layer, the following structures can be mentioned: 1) hole extraction layer/photoelectric conversion layer, 2) photoelectric conversion layer/electron extraction layer, 3) hole extraction layer/photoelectric conversion layer/electron extraction layer, and the like. The electron extraction layer is a layer provided to facilitate extraction of electrons from the photoelectric conversion layer to the cathode, and is usually provided between the photoelectric conversion layer and the cathode. The hole extraction layer is a layer provided to facilitate extraction of holes from the photoelectric conversion layer to the anode, and is usually provided between the anode and the photoelectric conversion layer. Each of the above layers may be either a single layer or a plurality of layers.

The compound of the present invention may be used in any layer in the photoelectric conversion element described above. The compound is, however, preferably used in a photoelectric conversion layer since it has high electron affinity and thin film stability and has strong absorption in the visible light region, and is more preferably used as an n-type material of a photoelectric conversion layer since it has excellent electron transport ability. In addition, since the compound of the present invention has high electron affinity, it can be suitably used also in an electron extraction layer. As a result, the electron extraction efficiency from the photoelectric conversion layer to the negative electrode is enhanced, so that the conversion efficiency can be improved.

The photoelectric conversion element can be used in a photosensor. Further, the photoelectric conversion element in this embodiment can be used in a solar cell. Further, the photoelectric conversion element can be suitably used in an image sensor. The image sensor herein refers to an image pickup element that stores image information by detecting light to generate electric charge and converting the electric charge into an electric signal. Further, the photoelectric conversion element in this embodiment can be used in a solar cell.

### <Luminescent Element>

As described above, the compound of the present invention can also be used as a luminescent element material. Embodiments of the luminescent element of the present invention will be described in detail below. The luminescent element of the present invention has a positive electrode, a negative electrode, and an organic layer interposed between the positive electrode and the negative electrode, wherein the organic layer has at least a luminescent layer and an electron transport layer, and the luminescent layer emits light by electric energy.

As for the lamination structure of the organic layer, in addition to the structure consisting only of the luminescent layer/electron transport layer, the following structures can be mentioned: 1) hole transport layer/luminescent layer/electron transport layer, 2) hole transport layer/luminescent layer/electron transport layer/electron injection layer, 3) hole injection layer/hole transport layer/luminescent layer/electron transport layer/electron injection layer, and the like. Each of the above layers may be either a single layer or a plurality of layers. Further, the luminescent element may be a laminate having a plurality of phosphorescent layers or fluorescent layers, or may be a luminescent element including a fluorescent layer and a phosphorescent layer in combination. Further, luminescent layers exhibiting luminescent colors different from each other can be laminated.

Further, the above-mentioned element structure may be of a tandem type in which a plurality of layers are laminated via an intermediate layer. Among the layers, at least one layer is preferably a phosphorescent layer. The intermediate layer is generally also called an intermediate electrode, an intermediate conductive layer, a charge generating layer, an electron extracting layer, a connecting layer, or an intermediate insulating layer, and can be made with a known material configuration. Specific examples of the tandem type element include a lamination structure including a charge generating layer as an intermediate layer between a positive electrode and a negative electrode, such as: 4) hole transport layer/luminescent layer/electron transport layer/charge generating layer/hole transport layer/luminescent layer/electron transport layer, and 5) hole injection layer/hole transport layer/luminescent layer/electron transport layer/electron injection layer/charge generating layer/hole injection layer/hole transport layer/luminescent layer/electron transport layer/electron injection layer. As materials constituting the intermediate layer, specifically, a pyridine derivative and a phenanthroline derivative are preferably used. In the case of a phenanthroline derivative, the compound is more preferably a compound having two or more phenanthroline skeletons in the molecule.

The compound of the present invention may be used in any layer in the above-mentioned element structure, but is preferably used in a luminescent layer, an electron transport layer, or an intermediate layer of a luminescent element since the compound has high electron injection/transport ability, high fluorescence quantum yield, and high thin film stability. Since the compound has excellent electron injection/transport ability, it is more preferably used in an electron transport layer or an intermediate layer, and is particularly preferably used in an electron transport layer.

### (Electrodes)

In the luminescent element of the present invention, the positive electrode and the negative electrode have a role of supplying a sufficient current for light emission of the element, and at least one of the positive electrode and the negative electrode is preferably transparent or translucent for light extraction. Usually, a transparent electrode is used as a positive electrode to be formed on a substrate.

As a material used in the positive electrode, a material that is capable of efficiently injecting holes into the organic layer and that is transparent or translucent for light extraction can be used without particular limitation, and examples thereof include conductive metal oxides such as tin oxide, indium oxide, indium tin oxide (ITO), and indium zinc oxide (IZO), inorganic conductive substances including metals such as gold, silver, and chromium, and copper iodide and copper sulfide, and conductive polymers such as polythiophene, polypyrrole, and polyaniline. It is particularly preferable to use ITO glass, or Nesa glass having a film mainly containing tin oxide formed on the surface of glass. These electrode materials may be used singly, or a plurality of materials may be laminated or mixed. The resistance of the transparent electrode is not limited as long as the electrode can supply a sufficient current for light emission of the element, but the resistance is preferably low from the viewpoint of the power consumption of the element. For example, an ITO substrate having a resistance of 300 Ω/□ or less functions as an element electrode. At present, however, even a substrate having a resistance of about 10 Ω/□ can be provided, and thus a substrate having a low resistance of 20 Ω/□ or less is particularly preferably used. The thickness of the ITO substrate can be arbitrarily selected according to the resistance value, and usually an ITO substrate having a thickness in the range of 100 to 300 nm is often used.

In addition, in order to maintain the mechanical strength of the luminescent element, it is preferable to form the luminescent element on a substrate. The substrate is suitably a glass substrate such as soda glass or alkali-free glass. A sufficient thickness of the glass substrate is 0.5 mm or more, since the glass substrate has only to have a thickness sufficient for maintaining the mechanical strength. As for the material of the glass, the alkali-free glass is preferable since it is preferable that less ions be eluted from the glass. Alternatively, commercially available soda-lime glass coated with a barrier coat such as SiO₂ can also be used. If a first electrode stably functions, there is no need that the substrate is made from glass and, for example, the positive electrode may be formed on a plastic substrate. The method of forming the ITO film is not particularly limited as long as it is a method capable of forming an ITO film, such as an electron beam method, a sputtering method, and a chemical reaction method.

The material used in the negative electrode is not particularly limited as long as it is a substance capable of efficiently injecting electrons into the luminescent layer. In general, metals such as platinum, gold, silver, copper, iron, tin, aluminum, and indium, and alloys or multilayers of these metals with a low work function metal such as lithium, sodium, potassium, calcium, and magnesium are preferable. Among them, aluminum, silver, and magnesium are preferable as the main component from the viewpoint of electric resistance value, ease of film formation, film stability, luminous efficiency, and the like. In particular, the negative electrode is preferably made from magnesium and silver since electron injection into the electron transport layer and the electron injection layer in the present invention is facilitated, and low voltage driving becomes possible.

In addition, for protection of the negative electrode, metals such as platinum, gold, silver, copper, iron, tin, aluminum, and indium, alloys containing these metals, inorganic substances such as silica, titania, and silicon nitride, and organic polymer compounds such as polyvinyl alcohol, polyvinyl chloride, and a hydrocarbon-based polymer compound are preferably laminated on the negative electrode as a protective film layer. The compound of the present invention can also be used as the protective film layer (cap layer). In the case of an element structure in which light is extracted from the negative electrode side (top emission structure), however, the material of the protective film layer is selected from materials having light permeability in the visible light region. The method of producing the electrode is not particularly limited, and examples thereof include resistance heating, electron beam, sputtering, ion plating, and coating.

### (Hole Transport Layer)

The hole transport layer is formed by a method of laminating or mixing one or more hole transport materials, or a method of using a mixture of a hole transport material and a polymer binder. The hole transport material is required to efficiently transport, between electrodes to which an electric field is applied, holes from the positive electrode, and it is preferable that the hole transport material have high hole injection efficiency and be capable of efficiently transporting the injected holes. For this purpose, it is required to use, as the hole transport material, a substance that has an appropriate ionization potential, high hole mobility, and excellent stability, and that hardly produces impurities as a trap at the time of production and use. Substances satisfying such conditions are not particularly limited, and preferable examples thereof include benzidine derivatives, such as 4,4'-bis(N-(3-methylphenyl)-N-phenylamino)biphenyl (TPD), 4,4'-bis(N-(1-naphthyl)-N-phenylamino)biphenyl (NPD), 4,4'-bis(N,N-bis(4-biphenylyl)amino)biphenyl (TBDB), and bis(N,N'-diphenyl-4-aminophenyl)-N,N-diphenyl-4,4'-diamino-1,1'-biphenyl (TPD232), a group of materials called starburst arylamines, such as 4,4',4"-tris(3-methylphenyl(phenyl)amino)triphenylamine (m-MTDATA) and 4,4',4"-tris(1-naphthyl(phenyl)amino)triphenylamine (1-TNATA), materials having a carbazole skeleton, above all, carbazole multimers, specifically, derivatives of carbazole dimers such as bis(N-arylcarbazole) and bis(N-alkylcarbazole), derivatives of carbazole trimers, and derivatives of carbazole tetramers, heterocyclic compounds such as triphenylene compounds, pyrazoline derivatives, stilbene compounds, hydrazone compounds, benzofuran derivatives, thiophene derivatives, oxadiazole derivatives, phthalocyanine derivatives, and porphyrin derivatives, fullerene derivatives, and polymers having the above-mentioned monomers in the side chain, such as polycarbonate, styrene derivatives, polythiophene, polyaniline, polyfluorene, polyvinylcarbazole, and polysilane. Further, inorganic compounds such as p-type Si and p-type SiC can also be used. Since the compound of the present invention is also excellent in electrochemical stability, it can be used as a hole transport material.

For the hole transport layer, a compound having an excellent electron blocking property is preferably used. Above all, a compound containing a carbazole skeleton is preferable because it has an excellent electron blocking property and contributes to high efficiency of the luminescent element. Further, it is preferable that the compound containing a carbazole skeleton contain a carbazole dimer, carbazole trimer, or carbazole tetramer skeleton. This is because such a compound combines a good electron blocking property with a good hole injection/transport property.

Further, when a compound containing a carbazole skeleton is used in the hole transport layer, it is more preferable that the luminescent layer to be combined therewith contain a phosphorescent material described later. This is because the compound having a carbazole skeleton also has a high triplet exciton blocking function, and can achieve high luminous efficiency when the compound is combined with a phosphorescent material.

Alternatively, a compound containing a triphenylene skeleton which is excellent in that it has high hole mobility is preferably used in the hole transport layer since the compound has an effect of improving the carrier balance as well as improving the luminous efficiency and durability life. It is more preferable that the compound containing a triphenylene skeleton have two or more diarylamino groups.

Each of these compound containing a carbazole skeleton and compound containing a triphenylene skeleton may be used alone in a hole transport layer, or a mixture of these compounds may be used in a hole transport layer. Further, other materials may be mixed as long as the effect of the present invention is not impaired. In the case where the hole transport layer is composed of a plurality of layers, it is sufficient that a compound containing a carbazole skeleton or a compound containing a triphenylene skeleton is contained in any one of the layers.

### (Hole Injection Layer)

A hole injection layer may be provided between the positive electrode and the hole transport layer. Providing the hole injection layer lowers the driving voltage of the luminescent element and improves the durability life of the luminescent element. For the hole injection layer, a material having a smaller ionization potential than that of a material usually used in the hole transport layer is preferably used. Specifically, besides the above-mentioned benzidine derivatives such as TPD232, and the group of starburst arylamine materials, phthalocyanine derivatives and the like can be used. It is also preferable that the hole injection layer be consisting only of an acceptor compound, or that another hole transport material doped with an acceptor compound be used. Examples of the acceptor compound include metal chlorides such as iron(III) chloride, aluminum chloride, gallium chloride, indium chloride, and antimony chloride, metal oxides such as molybdenum oxide, vanadium oxide, tungsten oxide, and ruthenium oxide, and charge transfer complexes such as tris(4-bromophenyl)aminium hexachloroantimonate (TBPAH). Organic compounds having a nitro group, a cyano group, a halogen, or a trifluoromethyl group in the molecule, quinone compounds, acid anhydride compounds, fullerenes and the like are also suitably used. Specific examples of these compounds include hexacyanobutadiene, hexacyanobenzene, tetracyanoethylene, tetracyanoquinodimethane (TCNQ), tetrafluorotetracyanoquinodimethane (F₄-TCNQ), 2,3,6,7,10,11-hexacyano-1,4,5,8,9,12-hexaazatriphenylene (HAT-CN₆), p-fluoranil, p-chloranil, p-bromanyl, p-benzoquinone, 2,6-dichlorobenzoquinone, 2,5-dichlorobenzoquinone, tetramethylbenzoquinone, 1,2,4,5-tetracyanobenzene, o-dicyanobenzene, p-dicyanobenzene, 1,4-dicyano-2,3,5,6-tetrafluorobenzene, 2,3-dichloro-5,6-dicyanobenzoquinone, o-dinitrobenzene, m-dinitrobenzene, p-dinitrobenzene, o-cyanonitrobenzene, m-cyanonitrobenzene, p-cyanonitrobenzene, 1,4-naphthoquinone, 2,3-dichloro-1,4-naphthoquinone, 1-nitronaphthalene, 2-nitronaphthalene, 1,3-dinitronaphthalene, 1,5-dinitronaphthalene, 9-cyanoanthracene, 9-nitroanthracene, 9,10-anthraquinone, 1,3,6,8-tetranitrocarbazole, 2,4,7-trinitro-9-fluorenone, 2,3,5,6-tetracyanopyridine, maleic anhydride, phthalic anhydride, C₆₀, and C₇₀.

Among them, metal oxides and cyano group-containing compounds are preferable because they are easy to handle and are easily vapor-deposited, so that the above-mentioned effects can be easily obtained. Examples of preferable metal oxides include molybdenum oxide, vanadium oxide, and ruthenium oxide. Among the cyano group-containing compounds, the following compounds are more preferable since they serve as a strong electron acceptor: (a) a compound having at least one electron-accepting nitrogen atom in the molecule in addition to the nitrogen atom of the cyano group, (b) a compound having both a halogen and a cyano group in the molecule, (c) a compound having both a carbonyl group and a cyano group in the molecule, and (d) a compound having both a halogen and a cyano group in the molecule, and further having at least one electron-accepting nitrogen atom in addition to the nitrogen atom of the cyano group. Specific examples of such compounds include the following compounds.

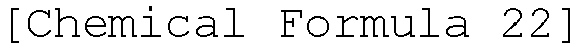

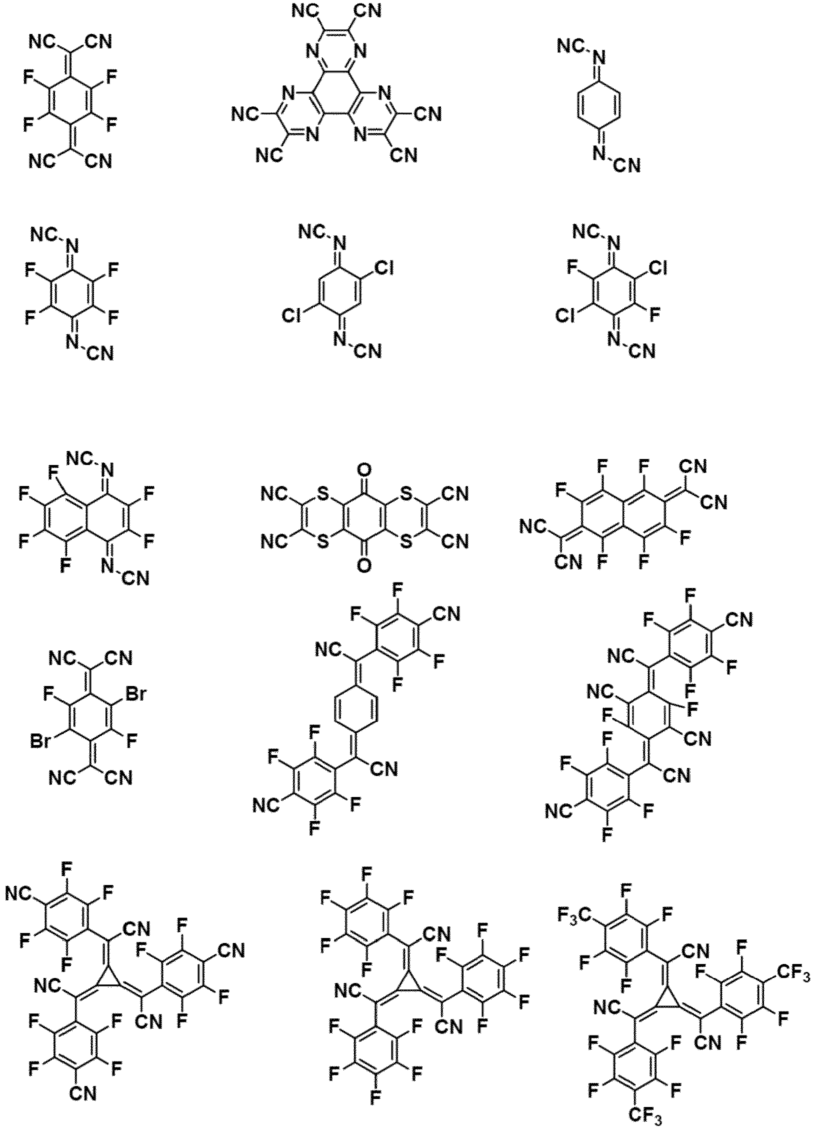

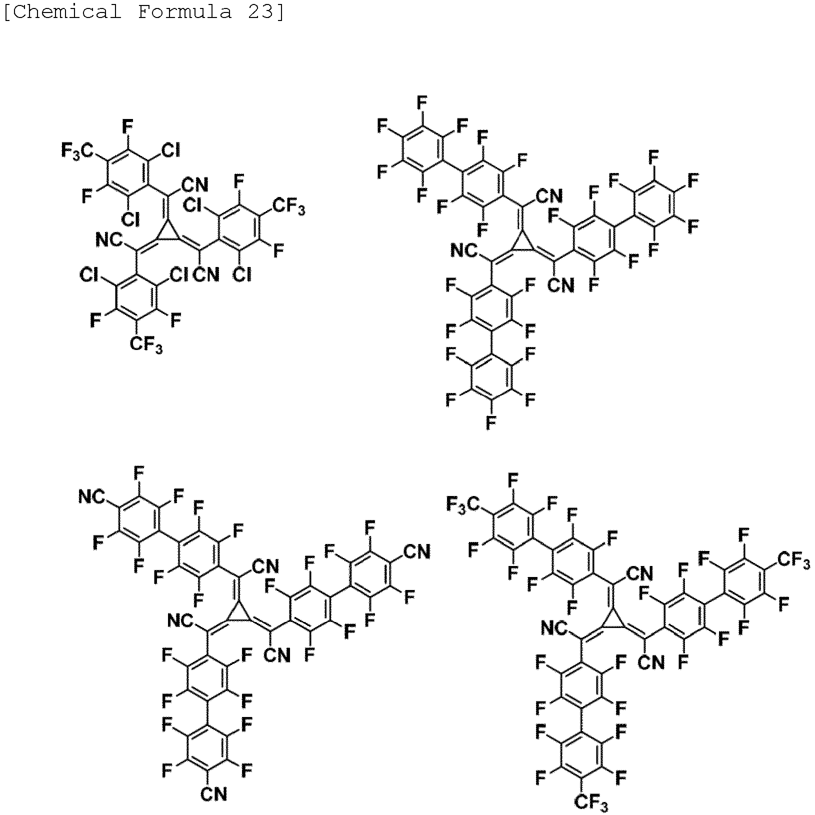

In either of the case where the hole injection layer is consisting only of an acceptor compound, or the hole injection layer is doped with an acceptor compound, the hole injection layer may be composed of one layer or a laminate of a plurality of layers. In addition, from the viewpoint that the hole injection barrier to the hole transport layer can be reduced, it is more preferable that the hole injection material used in combination when the hole injection layer is doped with an acceptor compound be the same compound as the compound used in the hole transport layer.

### (Luminescent Layer)

The luminescent layer may be either a single layer or a plurality of layers. Each luminescent layer is formed of a luminescent material (a host material and a dopant material), which may be a mixture of a host material and a dopant material, or a host material alone. That is, in the luminescent element of the present invention, in each luminescent layer, only one of the host material and the dopant material may emit light, or both the host material and the dopant material may emit light. From the viewpoint of efficiently utilizing the electric energy and obtaining light emission of high color purity, the luminescent layer is preferably made from a mixture of a host material and a dopant material.

Further, each of the host material and the dopant material may be of one type or a combination of a plurality of types. The dopant material may be contained in the entire host material or may be partially contained therein. The dopant material may be either laminated or dispersed. The dopant material is capable of controlling the luminescent color. The amount of the dopant material is preferably 20% by weight or less, more preferably 10% by weight or less relative to the host material, since too large an amount thereof may cause a concentration quenching phenomenon. The dopant material can be applied by a method of co-deposition with a host material. Alternatively, the dopant material may be preliminarily mixed with the host material and simultaneously vapor-deposited with the host material.

The luminescent material is not particularly limited, and specific examples thereof include condensed ring derivatives such as anthracene and pyrene, which have been previously known as luminous bodies, metal chelated oxinoid compounds such as tris(8-quinolinolate)aluminum(III), bisstyryl derivatives such as bisstyrylanthracene derivatives and distyrylbenzene derivatives, tetraphenyl butadiene derivatives, indene derivatives, coumarin derivatives, oxadiazole derivatives, pyrrolopyridine derivatives, perinone derivatives, cyclopentadiene derivatives, oxadiazole derivatives, thiadiazolopyridine derivatives, dibenzofuran derivatives, carbazole derivatives, indolocarbazole derivatives, and polymers such as polyphenylenevinylene derivatives, polyparaphenylene derivatives, and polythiophene derivatives.

The host material contained in the luminescent material is not particularly limited, and examples thereof include compounds having a condensed aryl ring and derivatives thereof, such as naphthalene, anthracene, phenanthrene, pyrene, chrysene, naphthacene, triphenylene, perylene, fluoranthene, fluorene, and indene, aromatic amine derivatives such as N,N'-dinaphthyl-N,N'-diphenyl-4,4'-diphenyl-1,1'-diamine, metal chelated oxinoid compounds such as tris(8-quinolinolate)aluminum(III), bistyryl derivatives such as distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, carbazole derivatives, indolocarbazole derivatives, triazine derivatives, and polymers such as polyphenylenevinylene derivatives, polyparaphenylene derivatives, polyfluorene derivatives, polyvinylcarbazole derivatives, and polythiophene derivatives.

When the compound of the present invention is used as an electron transport material, a compound having an anthracene skeleton is more preferable. The dopant material is not particularly limited, and examples thereof include compounds having a fused aryl ring, such as naphthalene, anthracene, phenanthrene, pyrene, chrysene, triphenylene, perylene, fluoranthene, fluorene, and indene, and derivatives thereof (for example, 2-(benzothiazol-2-yl)-9,10-diphenylanthracene and 5,6,11,12-tetraphenylnaphthacene), compounds having a heteroaryl ring and derivatives thereof, such as furan, pyrrole, thiophene, silole, 9-silafluorene, 9,9'-spirobisilafluorene, benzothiophene, benzofuran, indole, dibenzothiophene, dibenzofuran, imidazopyridine, phenanthroline, pyridine, pyrazine, naphthyridine, quinoxaline, pyrrolopyridine, and thioxanthene, borane derivatives, distyrylbenzene derivatives, aminostyryl derivatives such as 4,4'-bis(2-(4-diphenylaminophenyl)ethenyl)biphenyl and 4,4'-bis(N-(stilben-4-yl)-N-phenylamino)stilbene, aromatic acetylene derivatives, tetraphenylbutadiene derivatives, stilbene derivatives, aldazine derivatives, pyrromethene derivatives, diketopyrrolo[3,4-c]pyrrole derivatives, coumarin derivatives such as 2,3,5,6-1H,4H-tetrahydro-9-(2'-benzothiazolyl)quinolizino[9,9a,1-gh]coumarin, azole derivatives such as imidazole, thiazole, thiadiazole, carbazole, oxazole, oxadiazole, and triazole, and metal complexes thereof, and aromatic amine derivatives typified by N,N'-diphenyl-N,N'-di(3-methylphenyl)-4,4'-diphenyl-1,1'-diamine.

Further, a phosphorescent material may be contained in the luminescent layer. The phosphorescent material is a material that exhibits phosphorescence even at room temperature. When a phosphorescent material is used as a dopant, basically phosphorescence should be obtained at room temperature, but the phosphorescent material is not particularly limited. The phosphorescent material is preferably an organometallic complex compound containing at least one metal selected from the group consisting of iridium (Ir), ruthenium (Ru), rhodium (Rh), palladium (Pd), platinum (Pt), osmium (Os), and rhenium (Re). In particular, an organometallic complex containing iridium or platinum is more preferable from the viewpoint of having a high phosphorescence yield even at room temperature.

Examples of the host suitably used in combination with the phosphorescent dopant include indole derivatives, carbazole derivatives, indolocarbazole derivatives, nitrogen-containing aromatic compound derivatives having a pyridine, pyrimidine, or triazine skeleton, aromatic hydrocarbon compound derivatives such as polyarylbenzene derivatives, spirofluorene derivatives, truxene derivatives, and triphenylene derivatives, compounds containing a chalcogen element, such as dibenzofuran derivatives and dibenzothiophene derivatives, and organometallic complexes such as a beryllium quinolinol complex. The host is not particularly limited as long as it has basically a higher triplet energy than that of the dopant used, and electrons and holes are smoothly injected and transported from each transport layer.

The luminescent layer may contain two or more triplet luminescent dopants or two or more host materials. Further, the luminescent layer may contain one or more triplet luminescent dopants and one or more fluorescent dopants. When a phosphorescent material is used in the luminescent layer, the compound of the present invention is suitably used as an electron transport material.

A preferable phosphorescent host or dopant is not particularly limited, and specific examples thereof include the following compounds.

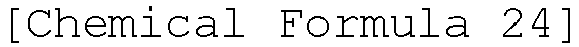

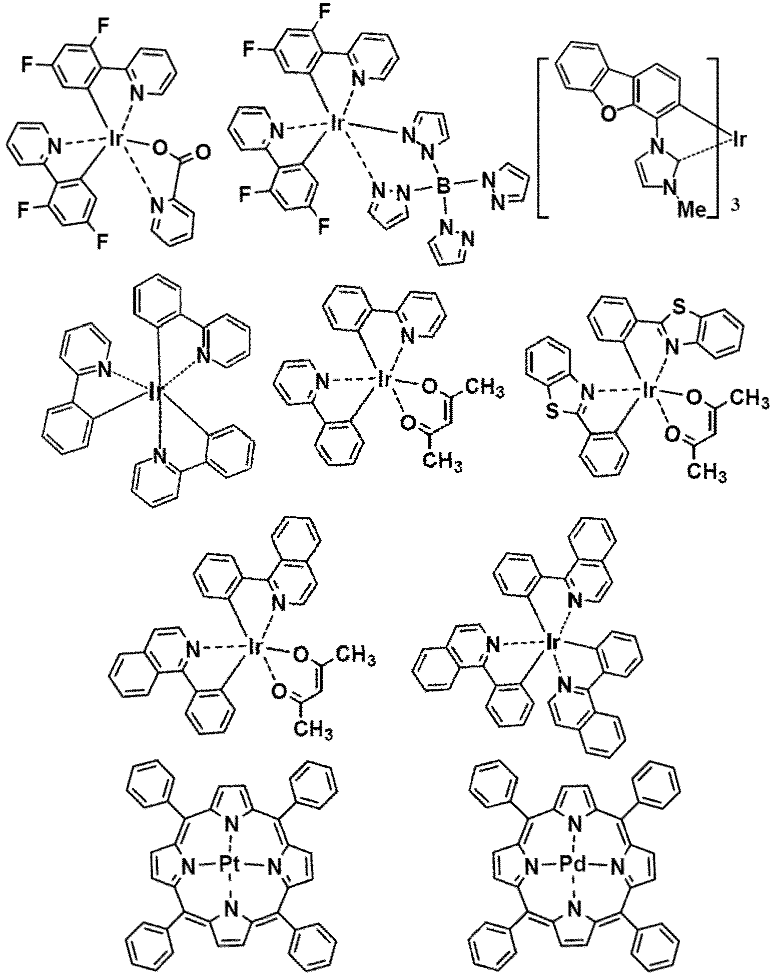

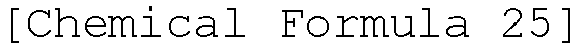

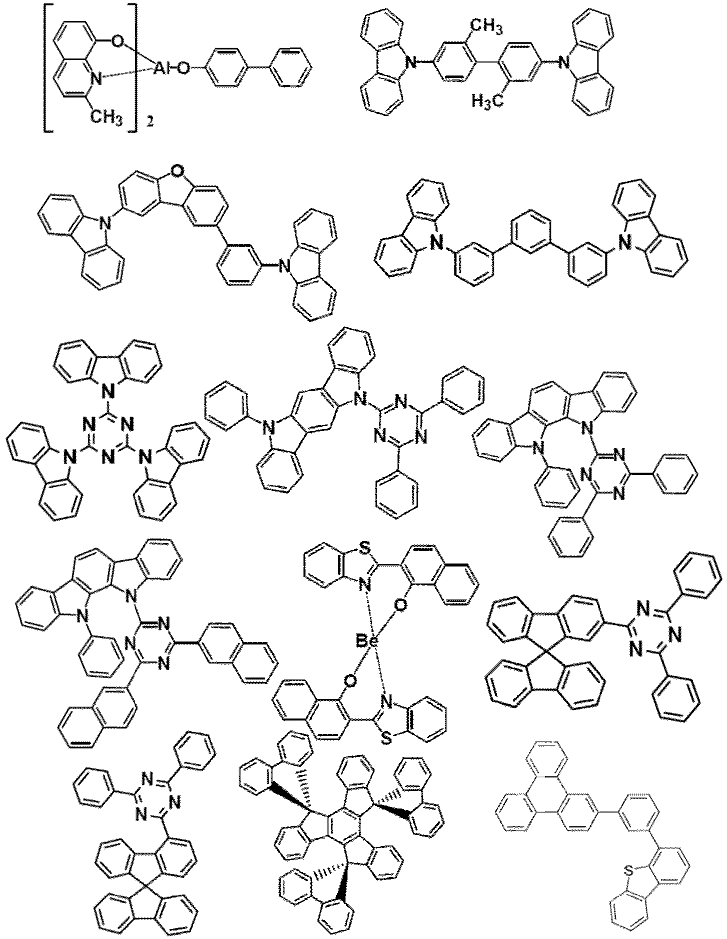

In addition, the luminescent layer may contain a thermally activated delayed fluorescence material. Generally, a thermally activated delayed fluorescence material is also called a TADF material, and is a material that promotes reverse intersystem crossing from the triplet excited state to the singlet excited state and improves the singlet exciton generation probability by lowering the energy gap between the energy level of the singlet excited state and the energy level of the triplet excited state. The thermally activated delayed fluorescence material may be a single material that exhibits thermally activated delayed fluorescence or a plurality of materials that exhibit thermally activated delayed fluorescence. When the thermally activated delayed fluorescence material is composed of a plurality of materials, it may be used as a mixture or used in a laminated structure. Known materials can be used as the thermally activated delayed fluorescence material. Specific examples of the material include benzonitrile derivatives, triazine derivatives, disulfoxide derivatives, carbazole derivatives, indolocarbazole derivatives, dihydrophenazine derivatives, thiazole derivatives, and oxadiazole derivatives, but the thermally activated delayed fluorescence material is not limited thereto.

The compound of the present invention also has high luminescence performance, so that the compound can be used as a luminescent material. Since the compound of the present invention exhibits strong light emission in the blue to green region (the region of 400 to 600 nm), it can be suitably used as blue and green luminescent materials. Since the compound of the present invention has high fluorescence quantum yield, it is suitably used as a fluorescent dopant material. In addition, the carbazole skeleton and the quinazoline skeleton each have a high triplet excitation energy level, and can be suitably used as a phosphorescent host. In particular, the carbazole skeleton and the quinazoline skeleton can be suitably used in a green phosphorescent host and a red phosphorescent host.

### (Electron Transport Layer)

In the present invention, the electron transport layer is a layer located between the negative electrode and the luminescent layer. The electron transport layer may be a single layer or a plurality of layers, and may be in contact with or not in contact with the negative electrode or the luminescent layer.

The electron transport layer is required to have high electron injection efficiency from the negative electrode, transport the injected electrons with high efficiency, and have high electron injection efficiency into the luminescent layer. Therefore, it is preferable that the electron transport layer be made from a substance that has high electron affinity, high electron mobility, and excellent stability, and that hardly produces impurities as a trap at the time of production and use.

In consideration of the balance of transportation between holes and electrons, however, if the electron transport layer mainly plays the role of efficiently preventing holes from the positive electrode from flowing to the negative electrode without recombination, the effect of improving the luminous efficiency is equivalent to the case where the electron transport layer is made from a material having high electron transport ability even if the electron transport layer is made from a material whose electron transport ability is not so high. Therefore, the "electron transport layer" in the present invention also encompasses a hole blocking layer capable of efficiently preventing the movement of holes.

Examples of the electron transport material used in the electron transport layer include condensed polycyclic aromatic hydrocarbon derivatives such as naphthalene and anthracene, styryl aromatic ring derivatives typified by 4,4'-bis(diphenylethenyl)biphenyl, quinone derivatives such as anthraquinone and diphenoquinone, phosphorus oxide derivatives, quinolinol complexes such as tris(8-quinolinolate)aluminum(III), and various metal complexes such as benzoquinolinol complexes, hydroxyazole complexes, azomethine complexes, tropolone metal complexes, and flavonol metal complexes. From the viewpoint of reducing the driving voltage and obtaining high-efficiency light emission, it is preferable to use a compound having a heteroaryl ring structure that is formed of an element selected from carbon, hydrogen, nitrogen, oxygen, silicon, and phosphorus, and that contains electron-accepting nitrogen.

An aromatic heterocycle having electron-accepting nitrogen has high electron affinity. The electron transport material having electron-accepting nitrogen makes it easier to receive electrons from the negative electrode having high electron affinity and enables driving at lower voltage. In addition, since the supply of electrons to the luminescent layer is increased and the recombination probability is increased, the luminous efficiency is improved.

Examples of the aromatic heterocycle having electron-accepting nitrogen include a pyridine ring, a pyrazine ring, a pyrimidine ring, a quinoline ring, a quinoxaline ring, a naphthyridine ring, a pyrimidopyrimidine ring, a benzoquinoline ring, a phenanthroline ring, an imidazole ring, an oxazole ring, an oxadiazole ring, a triazole ring, a thiazole ring, a thiadiazole ring, a benzoxazole ring, a benzothiazole ring, a benzimidazole ring, and a phenanthroimidazole ring.

Preferable examples of the compound having such aromatic heterocycle having electron-accepting nitrogen include benzimidazole derivatives, benzoxazole derivatives, benzthiazole derivatives, oxadiazole derivatives, thiadiazole derivatives, triazole derivatives, pyrazine derivatives, phenanthroline derivatives, quinoxaline derivatives, quinoline derivatives, benzoquinoline derivatives, oligopyridine derivatives such as bipyridine and terpyridine, and naphthyridine derivatives. Among them, those preferably used from the viewpoint of electron transport ability include: imidazole derivatives such as tris(N-phenylbenzimidazol-2-yl)benzene, oxadiazole derivatives such as 1,3-bis[(4-tert-butylphenyl)1,3,4-oxadiazolyl]phenylene, triazole derivatives such as N-naphthyl-2,5-diphenyl-1,3,4-triazole, phenanthroline derivatives such as bathocuproine and 1,3-bis(1,10-phenanthroline-9-yl)benzene, benzoquinoline derivatives such as 2,2'-bis(benzo[h]quinolin-2-yl)-9,9'-spirobifluorene, bipyridine derivatives such as 2,5-bis(6'-(2',2"-bipyridyl))-1,1-dimethyl-3,4-diphenylsilole, terpyridine derivatives such as 1,3-bis(4'-(2,2':6'2"-terpyridinyl)benzene, and naphthyridine derivatives such as bis(1-naphthyl)-4-(1,8-naphthyridin-2-yl)phenylphosphine oxide.

In addition, these derivatives more preferably have a condensed polycyclic aromatic skeleton, since the glass transition temperature is increased and the electron mobility is also increased, so that a large effect of lowering the voltage of the luminescent element can be obtained. Furthermore, in consideration of the improved element durability life and ease of synthesis and availability of raw materials, it is particularly preferable that the condensed polycyclic aromatic skeleton be an anthracene skeleton, a pyrene skeleton, or a phenanthroline skeleton.

Each of the above-mentioned electron transport materials may be used alone, but it is also possible that two or more of the above-mentioned electron transport materials are used as a mixture, or one or more other electron transport materials are mixed with the above-mentioned electron transport materials.

A preferable electron transport material is not particularly limited, and specific examples thereof include the following compounds.

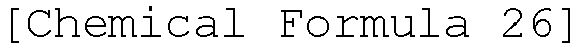

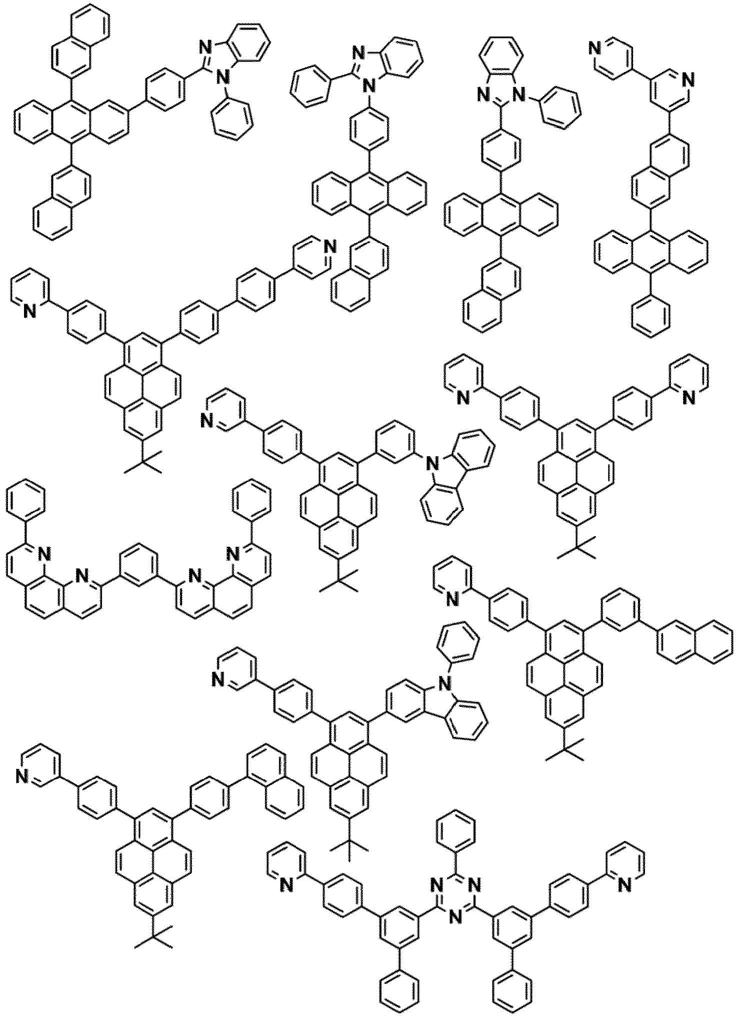

In addition to these, it is also possible to use electron transport materials disclosed in, for example, International Publications WO 2004/63159 and WO 2003/60956, "Applied Physics Letters", (USA), 1999, Vol. 74, No. 6, pp. 865-867, "Organic Electronics", (the Netherlands), 2003, Vol. 4, No. 2-3, pp. 113-121, and International Publications WO 2010/113743 and WO 2010/1817.

Further, since the compound of the present invention also has high electron injection/transport ability, it can be used as an electron transport material. When the compound of the present invention is used, it is not limited to only one kind thereof, but a mixture of a plurality of compounds of the present invention may be used, or one or more kinds of other electron transport materials may be mixed with the compound of the present invention as long as the effect of the present invention is not impaired.

The electron transport material that can be mixed is not particularly limited, and examples thereof include compounds having a condensed aryl ring and derivatives thereof, such as naphthalene, anthracene, and pyrene, styryl aromatic ring derivatives typified by 4,4'-bis(diphenylethenyl)biphenyl, perylene derivatives, perinone derivatives, coumarin derivatives, naphthalimide derivatives, quinone derivatives such as anthraquinone and diphenoquinone, phosphorus oxide derivatives, carbazole derivatives, indole derivatives, quinolinol complexes such as lithium quinolinol and tris(8-quinolinolate)aluminum(III), hydroxyazole complexes such as hydroxyphenyloxazole complexes, azomethine complexes, tropolone metal complexes, and flavonol metal complexes.

Each of the above-mentioned electron transport materials may be used alone, but it is also possible that two or more of the above-mentioned electron transport materials are used as a mixture, or one or more other electron transport materials are mixed with the above-mentioned electron transport materials. The electron transport material may also contain a donor material. The donor material is a compound that facilitates electron injection into the electron transport layer from the negative electrode or the electron injection layer by reduction of the electron injection barrier, and further improves the electric conductivity of the electron transport layer.

Preferable examples of the donor material in the present invention include alkali metals, inorganic salts containing an alkali metal, complexes of an alkali metal and an organic substance, alkaline earth metals, inorganic salts containing an alkaline earth metal, and complexes of an alkaline earth metal and an organic substance. Preferable examples of the alkali metal and alkaline earth metal include alkali metals such as lithium, sodium, and cesium, and alkaline earth metals such as magnesium and calcium, that have a low work function and a large effect of improving the electron transport ability.

In addition, the donor material is preferably an inorganic salt or in a state of a complex with an organic substance rather than a simple metal, since such a material is easily vapor-deposited in vacuum and is excellent in handleability. Further, it is more preferable that the donor material be in a state of a complex with an organic substance in view of ease of handling in the atmosphere and ease of control of the concentration of addition. Examples of the inorganic salt include oxides such as LiO and Li₂O, nitrides, fluorides such as LiF, NaF, and KF, and carbonates such as Li₂CO₃, Na₂CO₃, K₂CO₃, Rb₂CO₃, and CS₂CO₃. Preferable examples of the alkali metal or alkaline earth metal include lithium from the viewpoint of inexpensiveness of the raw material and ease of synthesis. Preferable examples of the organic substance in the complex with an organic substance include quinolinol, benzoquinolinol, flavonol, hydroxyimidazopyridine, hydroxybenzazole, and hydroxytriazole. Above all, a complex of an alkali metal and an organic substance is preferable, a complex of lithium and an organic substance is more preferable, and lithium quinolinol is particularly preferable. Two or more of these donor materials may be used as a mixture.

The suitable doping concentration differs depending on the material and the film thickness of the doping region. For example, when the donor material is an inorganic material such as an alkali metal or an alkaline earth metal, the electron transport material and the donor material are preferably codeposited at a deposition rate ratio in the range of 10,000 : 1 to 2 : 1 so as to form an electron transport layer. The deposition rate ratio is more preferably 100 : 1 to 5 : 1, further preferably 100 : 1 to 10 : 1. When the donor material is a complex of a metal and an organic substance, co-deposition is preferably performed so that the deposition rate ratio between the electron transport material and the donor material falls within the range of 100 : 1 to 1 : 100 to form the electron transport layer. The deposition rate ratio is more preferably 10 : 1 to 1 : 10, further preferably 7 : 3 to 3 : 7.

In addition, the electron transport layer containing the compound of the present invention doped with the donor material as described above may be used as a charge generating layer in a tandem structure type element including a plurality of luminescent elements connected to each other. In particular, when the compound of the present invention is doped with an alkali metal or an alkaline earth metal as a donor material, the layer can be suitably used as a charge generating layer.

The method of doping the electron transport layer with the donor material to improve the electron transport ability is particularly effective when the thin film layer is thick. The method is particularly preferable when the total film thickness of the electron transport layer and the luminescent layer is 50 nm or more. For example, there is a method of utilizing the interference effect to improve the luminous efficiency, which matches the phase of the light directly emitted from the luminescent layer with the phase of the light reflected by the negative electrode to improve the light extraction efficiency. Although the optimum condition of this method varies depending on the emission wavelength of light, the total film thickness of the electron transport layer and the luminescent layer is 50 nm or more, and in the case of long-wavelength light emission as in red light, the film may have a large thickness near 100 nm.

The region of the electron transport layer to be doped may be either a part or the whole of the electron transport layer. When a part of the electron transport layer is doped, it is desirable to provide a doping region at least at the interface of the electron transport layer/negative electrode. Even the doping near the negative electrode interface can provide the effect of lowering the voltage. On the other hand, if the donor material is in direct contact with the luminescent layer, it may adversely affect and lower the luminous efficiency. In that case, it is preferable to provide a non-doped region at the luminescent layer/electron transport layer interface.

### (Electron Injection Layer)

In the present invention, an electron injection layer may be provided between the negative electrode and the electron transport layer. In general, the electron injection layer is inserted for the purpose of assisting the injection of electrons from the negative electrode into the electron transport layer. When the electron injection layer is inserted, a compound having a heteroaryl ring structure containing electron-accepting nitrogen may be used, or a layer containing the above-mentioned donor material may be used. The compound of the present invention may be contained in the electron injection layer. An insulator or semiconductor inorganic substance can also be used in the electron injection layer. Use of these materials is preferable since a short circuit of the luminescent element can be effectively prevented and electron injectability can be improved.

As such an insulator, it is preferable to use at least one metal compound selected from the group consisting of alkali metal chalcogenides, alkaline earth metal chalcogenides, alkali metal halides, and alkaline earth metal halides. The electron injection layer is more preferably made from such alkali metal chalcogenide or the like because the electron injectability can be further improved. Specifically, preferable examples of alkali metal chalcogenides include Li₂O, Na₂S, and Na₂Se, and preferable examples of alkaline earth metal chalcogenides include CaO, BaO, SrO, BeO, BaS, and CaSe. Preferable examples of alkali metal halides include LiF, NaF, KF, LiCl, KCl, and NaCl. Preferable examples of alkaline earth metal halides include fluorides such as CaF₂, BaF₂, SrF₂, MgF₂, and BeF₂, and halides other than fluorides. Complexes of organic substances and metals are also suitably used.

A complex of an organic substance and a metal is more preferably used in the electron injection layer since the film thickness can be easily adjusted. As an example of such an organometallic complex, preferable examples of the organic substance in the complex with the organic substance include quinolinol, benzoquinolinol, pyridylphenol, flavonol, hydroxyimidazopyridine, hydroxybenzazole, and hydroxytriazole. Above all, a complex of an alkali metal and an organic substance is preferable, a complex of lithium and an organic substance is more preferable, and lithium quinolinol is particularly preferable.

The method of forming each of the layers constituting the luminescent element is not particularly limited, and examples thereof include resistance heating evaporation, electron beam evaporation, sputtering, a molecular lamination method, and a coating method. Usually, resistance heating evaporation or electron beam evaporation is preferable from the viewpoint of element characteristics.

The thickness of the organic layer cannot be limited because it depends on the resistance value of the luminescent substance, but it is preferably 1 to 1000 nm. The thickness of each of the luminescent layer, the electron transport layer, and the hole transport layer is preferably 1 nm or more and 200 nm or less, more preferably 5 nm or more and 100 nm or less.

The luminescent element of the present invention has a function of converting electric energy into light. As the electric energy, a direct current is mainly used, but it is also possible to use a pulse current or an alternating current. The current value and the voltage value are not particularly limited, however, in view of the power consumption and life of the element, the current value and the voltage value should be selected so as to obtain the maximum luminance with as low energy as possible.

The luminescent element of the present invention is suitably used, for example, as a display of a matrix and/or segment system.

In the matrix system, pixels for display are arranged two-dimensionally, as in a lattice shape or a mosaic shape, and characters or images are displayed by a set of pixels. The shape and size of the pixels depend on the application. For example, tetragonal pixels 300 µm or less on a side are usually used in image and character display of personal computers, monitors, and televisions, and in the case of a large display such as a display panel, pixels on a millimeter order on a side are used. In the case of monochrome display, pixels of the same color may be arranged, whereas in the case of color display, pixels of red, green, and blue are displayed side by side. In this case, there are typically delta type and stripe type arrangements. The method of driving the matrix may be either a line sequential driving method or an active matrix driving method. Although the line sequential driving is simpler in the structure, the active matrix may be superior in some cases in consideration of operating characteristics, and thus it is also necessary to select the driving method properly depending on the application.

In the segment system according to the present invention, a pattern is formed so as to display predetermined information, and a designated region is caused to emit light depending on the arrangement of the pattern. Examples of the system include time and temperature indication in a digital watch or a thermometer, operation state indication in an audio device and an electromagnetic cooker, and a panel display of an automobile. The matrix display and the segment display may coexist in one panel.

The luminescent element of the present invention is also preferably used as a backlight of various devices and the like. The backlight is used mainly for the purpose of improving the visibility of a display device that does not emit light on its own, and is used in liquid crystal display devices, clocks, audio devices, automobile panels, display boards, signs, and the like. In particular, the luminescent element of the present invention is preferably used in a liquid crystal display device, particularly a backlight for a personal computer that is studied to be thin, and can provide a thinner and lighter backlight than conventional ones.

### [Examples]

The present invention will be described below by way of examples, but the present invention is not limited to these examples.

### Synthesis Example 1

### Synthesis of intermediate [B]

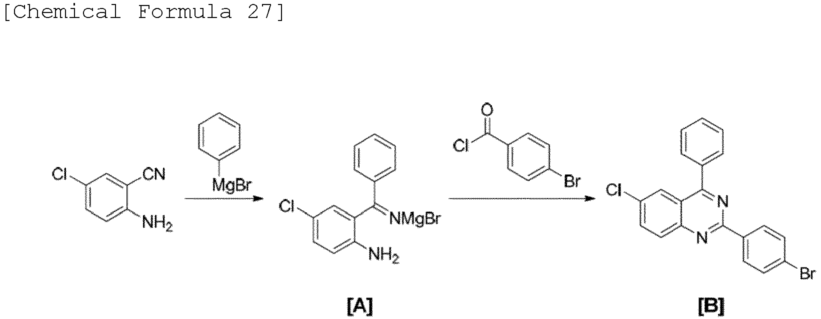

The intermediate B was synthesized by applying the method described in International Publication WO 2006/049013. That is, 100 ml (100 mmol) of a 1 M tetrahydrofuran solution of phenylmagnesium bromide was charged into a 500 ml three-necked flask, 100 ml of dry tetrahydrofuran was added thereto, and the mixture was heated under reflux in an oil bath at 45°C. To the mixture was added dropwise a solution of 7.63 g (50 mmol) of 2-amino-5-chlorobenzonitrile in 50 ml of dry tetrahydrofuran over 30 minutes. After further refluxing for 1.5 hours, the mixture was cooled to 0°C in an ice water bath. Subsequently, a solution of 13.2 g (60 mmol) of 4-bromobenzoyl chloride in 100 ml of dry tetrahydrofuran was added dropwise over 10 minutes, and the mixture was heated under reflux in an oil bath at 45°C for 2 hours. After completion of the reaction, the reaction mixture was cooled to 0°C in an ice water bath, and a saturated aqueous ammonium chloride solution was added thereto. The precipitate was collected by filtration, washed with a small amount of methanol, and dried under vacuum to give 8.11 g of the intermediate [B]. The obtained intermediate [B] was purified by silica gel column chromatography using a heptane-toluene mixed developing solvent.

Apart from the synthesis of the intermediate [B], an intermediate [C] was synthesized in the following manner.

### Synthesis of intermediate [C]

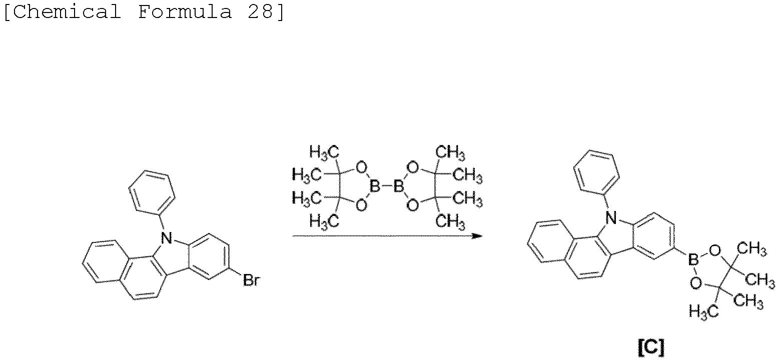

To a 100 ml three-necked flask, 3.72 g (10.0 mmol) of 8-bromo-11-phenyl-11H-benzo[a]carbazole, 2.79 g (11.0 mmol) of bis(pinacolato)diboron, and 2.94 g (30.0 mmol) of potassium acetate were charged, then 50 mL of dimethylformamide was added thereto, and the interior of the vessel was purged with nitrogen. To this mixture was added 0.073 g (0.1 mmol) of a [1,1'-bis(diphenylphosphino)ferrocene]palladium(II) dichloride-dichloromethane complex, and the mixture was heated to 100°C. After 1 hour, the mixture was cooled to room temperature, then 250 mL of ethyl acetate, 250 mL of toluene, and 250 mL of water were added, and the mixture was separated. The aqueous layer was extracted with 200 mL of ethyl acetate and 200 mL of toluene, combined with the above organic layer, and washed three times with 500 mL of water. The organic layer was dried over magnesium sulfate, and the solvent was distilled off. The residue was purified by silica gel column chromatography, the solvent was distilled off from the eluate, and the resultant was dried under vacuum to give 3.40 g of the intermediate [C].

### Synthesis of intermediate [D]

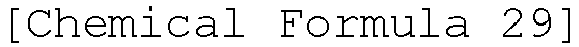

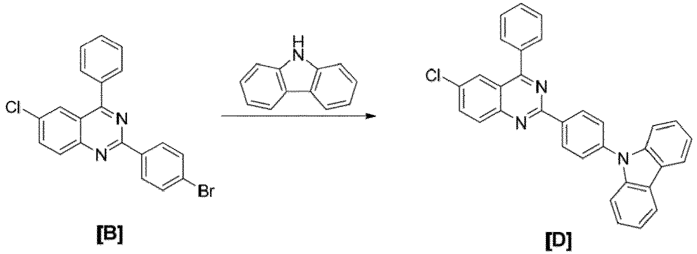

To a 100 ml three-necked flask, 6.00 g (15.2 mmol) of the intermediate [B], 2.66 g (15.9 mmol) of carbazole, and 2.05 g (21.3 mmol) of sodium tert-butoxide were charged, then 30 ml of o-xylene was added thereto, and the interior of the vessel was purged with nitrogen. To this mixture was added 0.173 g (0.15 mmol) of tetrakis(triphenylphosphine)palladium(0), and the mixture was heated under reflux in an oil bath at 90°C for 3 hours. After completion of the reaction, the reaction mixture was cooled to room temperature, and the precipitated solid was collected by filtration to give 6.65 g of the intermediate [D]. The obtained solid was dissolved in toluene, purified by silica gel column chromatography, dissolved in 130 mL of butyl acetate by heating, cooled to recrystallize, collected by filtration, and dried under vacuum to give 4.86 g of the intermediate [D].

### Synthesis of compound [1]

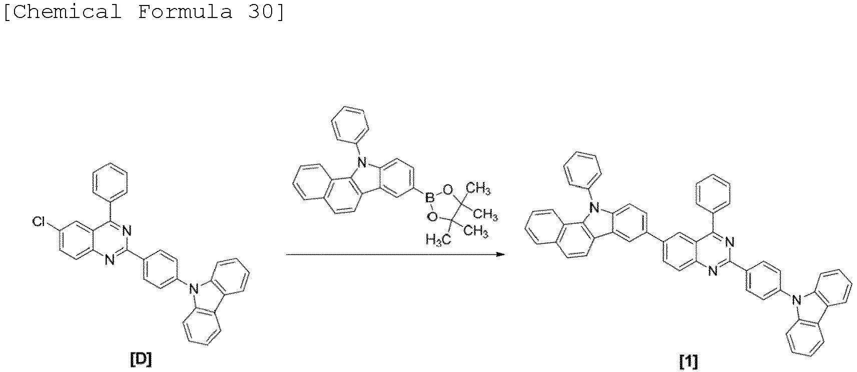

To a 200 ml three-necked flask, 4.00 g (10.1 mmol) of the intermediate [B], 8.89 g (21.2 mmol) of the intermediate [C], 50 ml of 1,2-dimethoxyethane, and 15.2 ml (3 equivalents) of a 2 M aqueous sodium carbonate solution were charged, and the interior of the vessel was purged with nitrogen. Further, 0.233 g (0.20 mmol) of tetrakis(triphenylphosphine)palladium(0) was added thereto, and the mixture was heated under reflux in an oil bath at 100°C for 3 hours. After completion of the reaction, the reaction mixture was cooled to room temperature, and the precipitated solid was collected by filtration to give 6.72 g of the compound [1]. The obtained solid was dissolved in toluene, purified by silica gel column chromatography, dissolved in 120 mL of butyl acetate by heating, cooled to recrystallize, collected by filtration, and dried under vacuum to give 5.02 g of a yellow-green solid of the compound [1].

The obtained yellow-green solid was confirmed to be the compound [1] by mass spectrometry (JMS-Q1000TD manufactured by JEOL Ltd.).

Further, the compound [1] was subjected to sublimation purification at about 360°C under a pressure of 1 × 10⁻³ Pa using an oil diffusion pump, and then used as a luminescent element material.

### Synthesis Example 2

### Synthesis of intermediate [F]

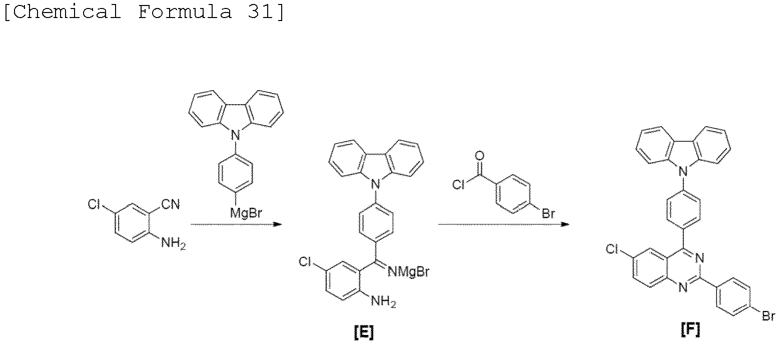

The intermediate [F] was synthesized in the same manner as in synthesis of the intermediate [B] of Synthesis Example 1 except that 2.43 g of magnesium was allowed to act on 32.2 g (100 mmol) of 9-(4-bromophenyl)carbazole and the resultant was used as a Grignard reagent instead of 100 ml (100 mmol) of the 1 M tetrahydrofuran solution of phenylmagnesium bromide, and purification was carried out in the same manner as in Synthesis Example 1.

Apart from the synthesis of the intermediate [F], an intermediate [G] was synthesized in the following manner.

### Synthesis of intermediate [G]

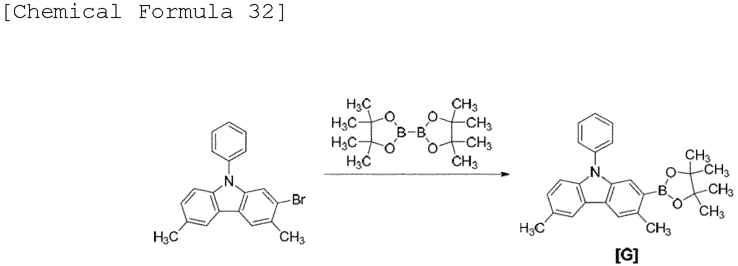

The intermediate [G] was synthesized in the same manner as in synthesis of the intermediate [C] of Synthesis Example 1 except that 3.50 g (10.0 mmol) of 2-bromo-3,6-dimethyl-9-phenylcarbazole was used instead of 3.72 g (10.0 mmol) of 8-bromo-11-phenyl-11H-benzo[a]carbazole, and extraction and purification were carried out in the same manner as in Synthesis Example 1.

### Synthesis of intermediate [H]

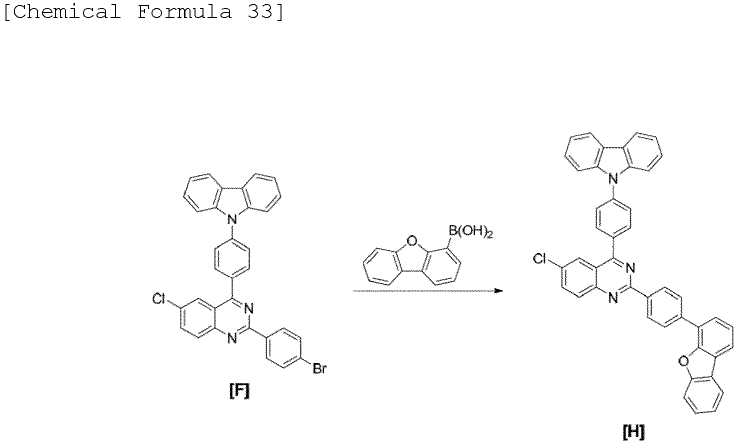

To a 200 ml three-necked flask, 6.00 g (10.7 mmol) of the intermediate [F] and 2.38 g (11.2 mmol) of 4-dibenzofuran boronic acid were charged, then 55 ml of dimethyl ether and 7.5 ml of a 2 M aqueous sodium carbonate solution were added, and the interior of the vessel was purged with nitrogen. To this mixture was added 0.127 g (0.11 mmol) of tetrakis(triphenylphosphine)palladium(0), and the mixture was heated under reflux in an oil bath at 80°C for 3 hours. After completion of the reaction, the reaction mixture was cooled to room temperature, and the precipitated solid was collected by filtration to give 6.38 g of the intermediate [H]. The obtained solid was dissolved in toluene, purified by silica gel column chromatography, dissolved in 130 mL of butyl acetate by heating, cooled to recrystallize, collected by filtration, and dried under vacuum to give 4.59 g of the intermediate [H].

### Synthesis of compound [2]

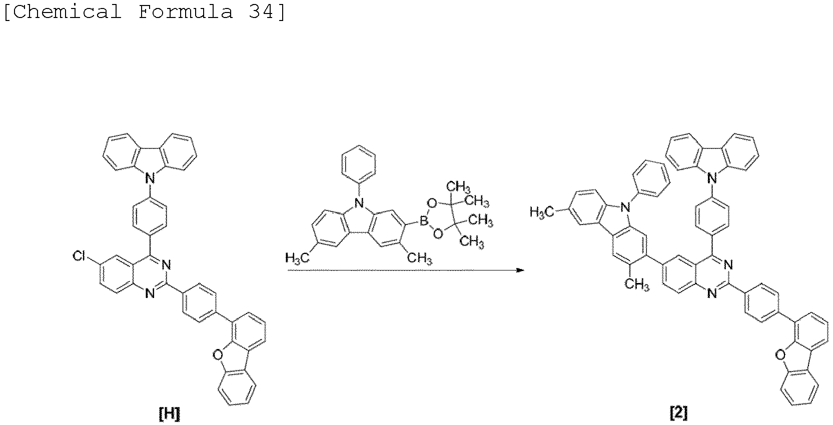

To a 100 ml three-necked flask, 4.50 g (6.94 mmol) of the intermediate [H], 3.03 g (7.64 mmol) of the intermediate [G], 35 ml of 1,4-dioxane, and 4.86 ml of a 2 M aqueous sodium carbonate solution were charged, and the interior of the vessel was purged with nitrogen. Further, 0.080 g (0.069 mmol) of tetrakis(triphenylphosphine)palladium(0) was added thereto, and the mixture was heated under reflux in an oil bath at 100°C for 3 hours. After completion of the reaction, the reaction mixture was cooled to room temperature, and the precipitated solid was collected by filtration to give 5.69 g of the compound [2]. The obtained solid was dissolved in toluene, purified by silica gel column chromatography, dissolved in 120 mL of butyl acetate by heating, cooled to recrystallize, collected by filtration, and dried under vacuum to give 3.98 g of a yellow-green solid of the compound [2].

The obtained yellow-green solid was confirmed to be the compound [2] by mass spectrometry (JMS-Q1000TD manufactured by JEOL Ltd.).

Further, the compound [2] was subjected to sublimation purification at about 360°C under a pressure of 1 × 10⁻³ Pa using an oil diffusion pump, and then used as a luminescent element material.

Compounds [3] to [30] used in the following examples can also be synthesized and purified from the corresponding raw material substances using methods similar to the methods described in Synthesis Examples 1 and 2.

### Example 1

A glass substrate (manufactured by GEOMATEC Co., Ltd., 11 Ω/□, sputtered product) on which 165 nm of an ITO transparent conductive film was deposited was cut into a size of 38 × 46 mm, followed by etching. The obtained substrate was subjected to ultrasonic cleaning with "Semico Clean 56" (trade name, manufactured by Furuuchi Chemical Corporation) for 15 minutes, and then washed with ultrapure water. Immediately before the production of an element, this substrate was subjected to a UV ozone treatment for 1 hour. The substrate was placed in a vacuum vapor deposition device, and the inside of the device was evacuated until the degree of vacuum reached 5 × 10⁻⁴ Pa or less. First, a HAT-CN₆ layer having a thickness of 5 nm as a hole injection layer and a HT-1 layer having a thickness of 50 nm as a hole transport layer were vapor-deposited by a resistance heating method. Then, as a luminescent layer, a host material H-1 and a dopant material D-1 were vapor-deposited each to a thickness of 20 nm so that the doping concentration would be 5% by weight. Then, the compound [1] was vapor-deposited and laminated to a thickness of 35 nm as an electron transport layer. Then, lithium fluoride was vapor-deposited to a thickness of 0.5 nm, and aluminum was vapor-deposited to a thickness of 1000 nm to form a negative electrode, thereby producing an element having a size of 5 mm × 5 mm. The film thickness referred to herein is an indicated value of a crystal oscillation type thickness monitor. Characteristics of this luminescent element at a luminance of 1000 cd/m² were a driving voltage of 4.63 V and an external quantum efficiency of 4.22%. Further, when the initial luminance was set to 1000 cd/m² and constant current driving was performed, the time until the luminance lowered by 20% (durability) was 1650 hours. The compounds [1], HAT-CN₆, HT-1, H-1, and D-1 are compounds shown below.

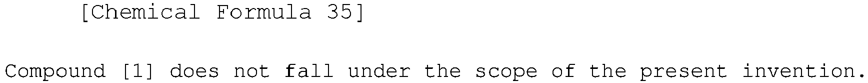

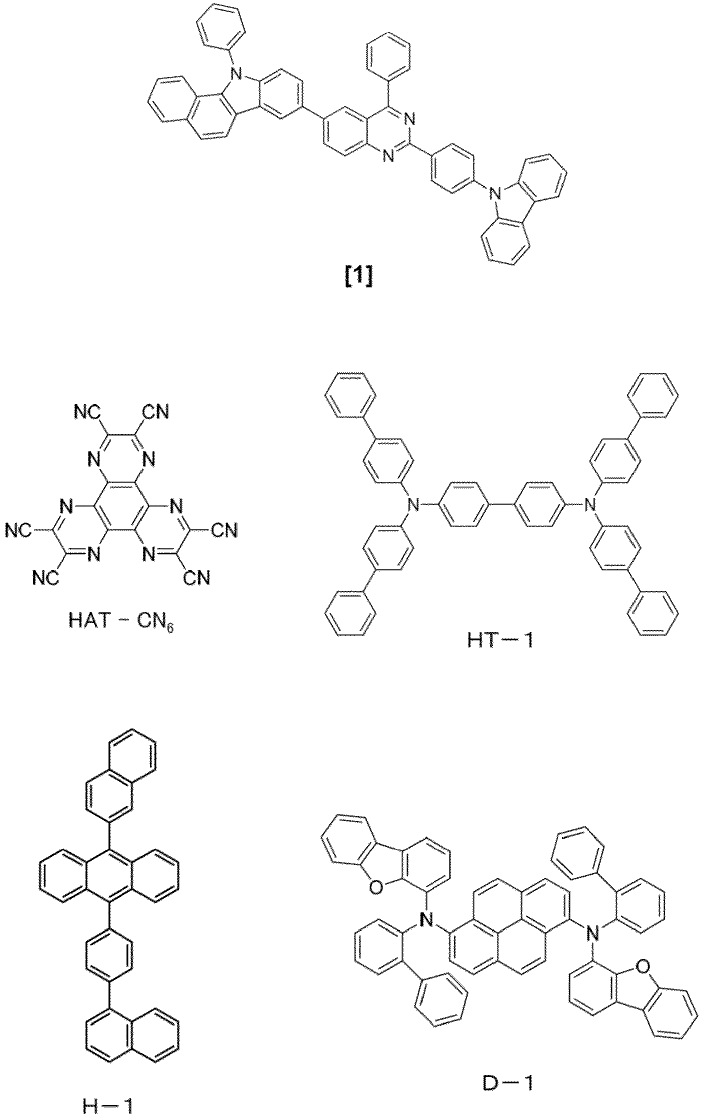

### Reference Examples : 1 to 6, 9, ; 12, 29 to 32, 41 and 42 and Examples : 7, 8, 10, 11, 13 to 28, 33 to 40 and 43

A luminescent element was produced and evaluated in the same manner as in Example 1, except that the compounds listed in Table 1 were used in the host material of the luminescent layer and the electron transport layer. The results are shown in Table 1. Note that H-2 and the compounds [2] to [42] are compounds shown below.

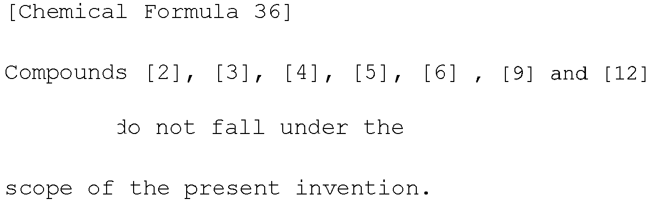

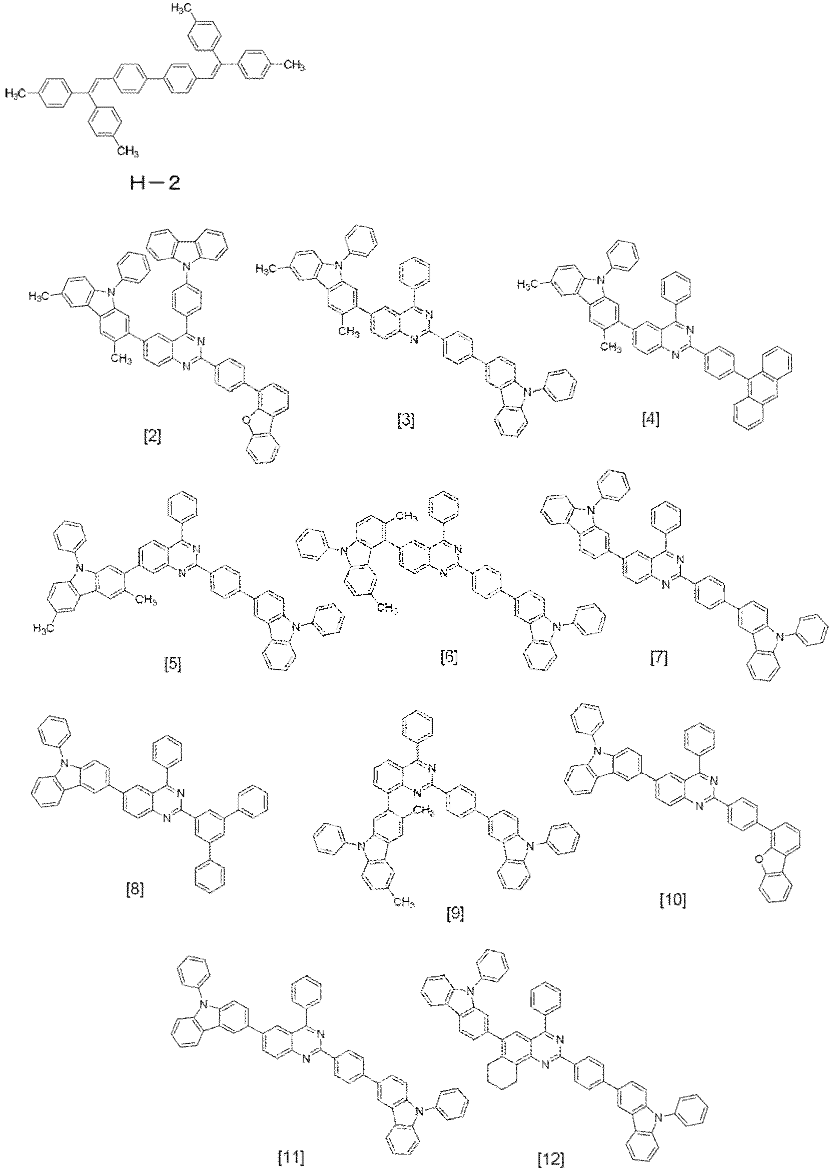

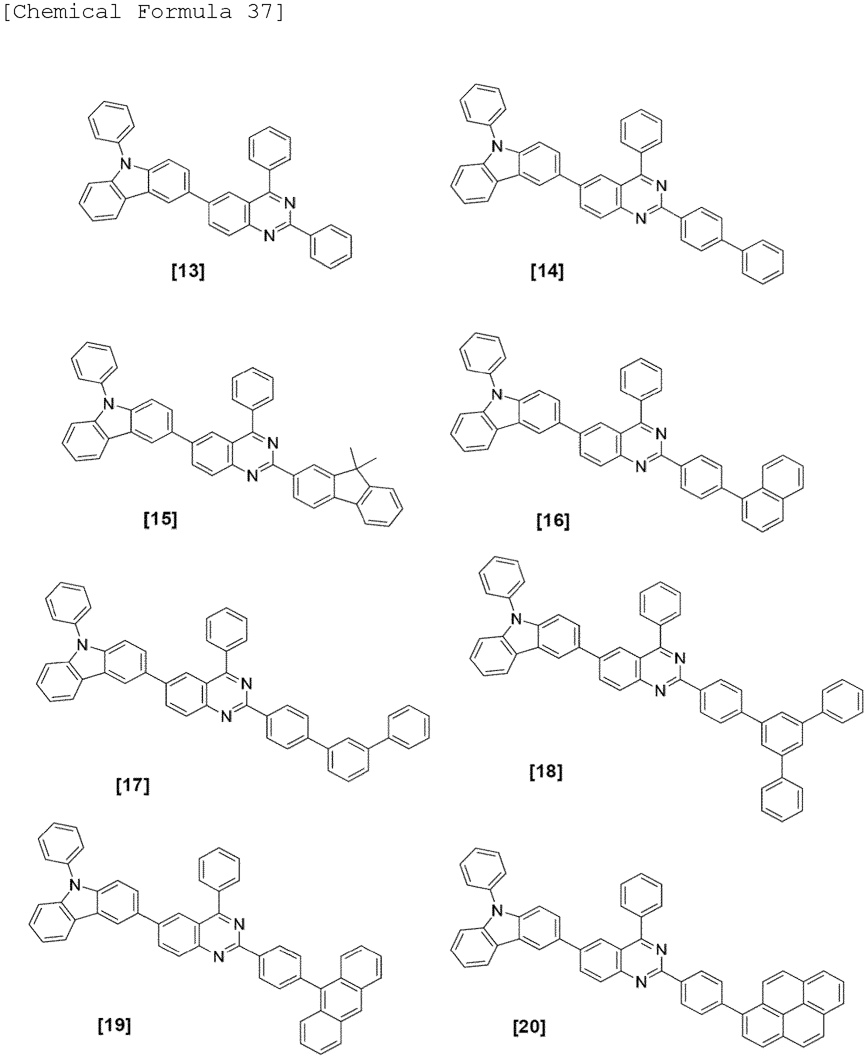

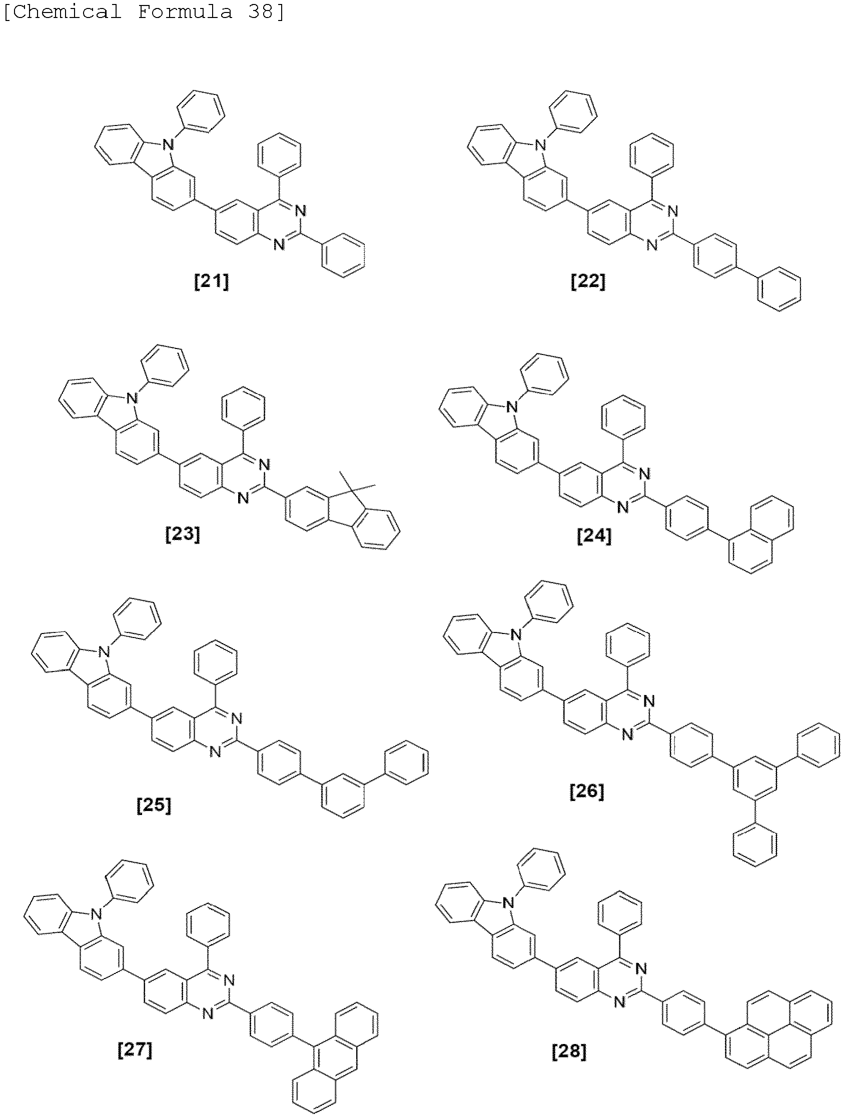

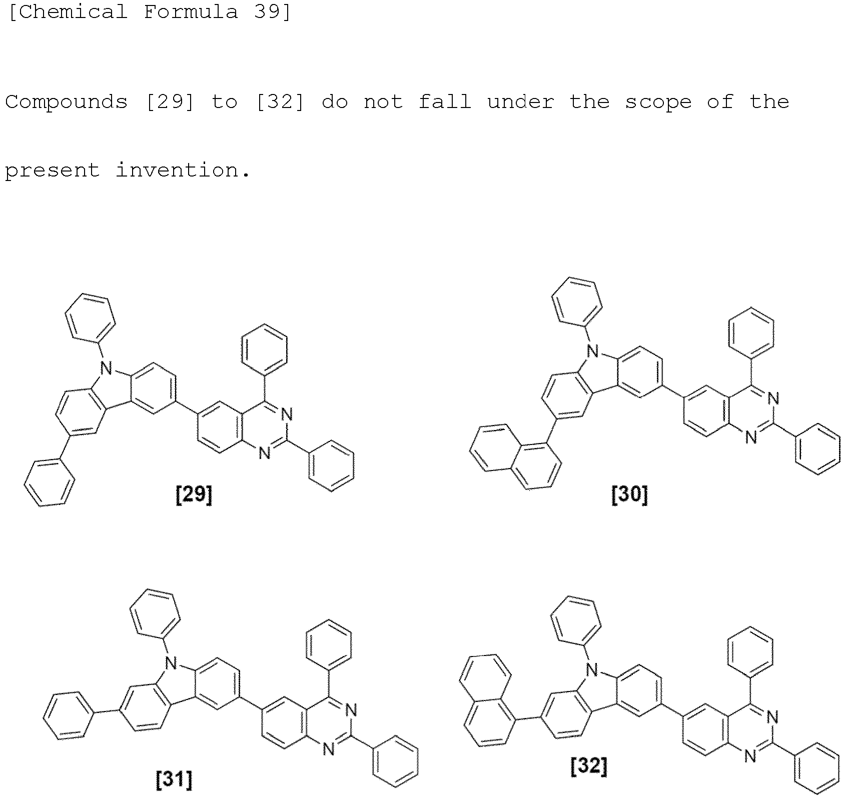

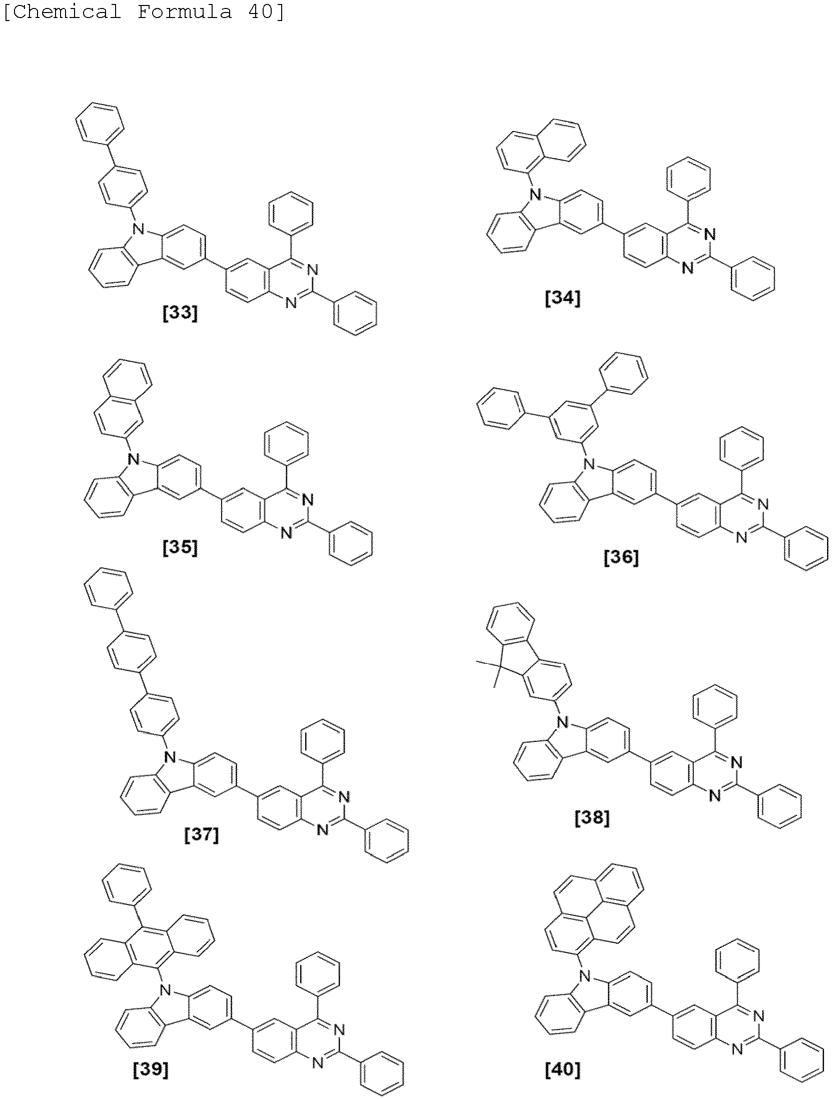

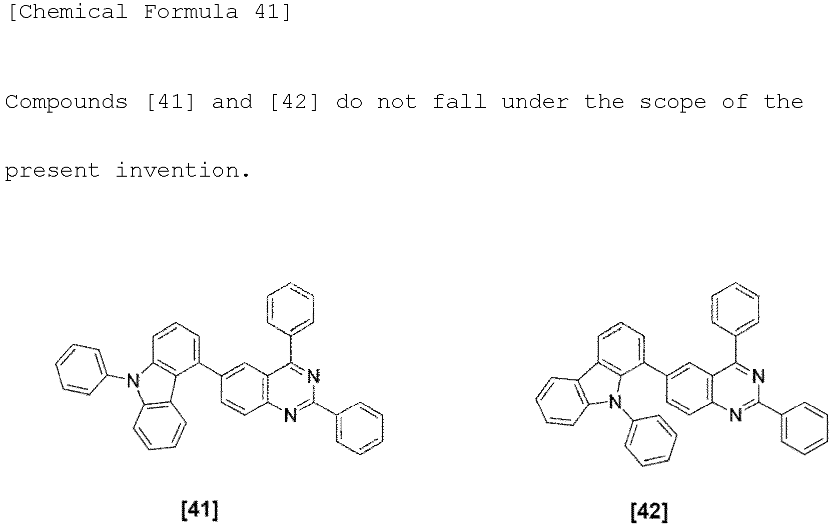

### Comparative Examples 1 to 18

A luminescent element was produced and evaluated in the same manner as in Example 1, except that the compound shown in Table 2 was used in the electron transport layer. The results are shown in Table 2. E-1 to E-8 and E-20 to E-29 are compounds shown below.

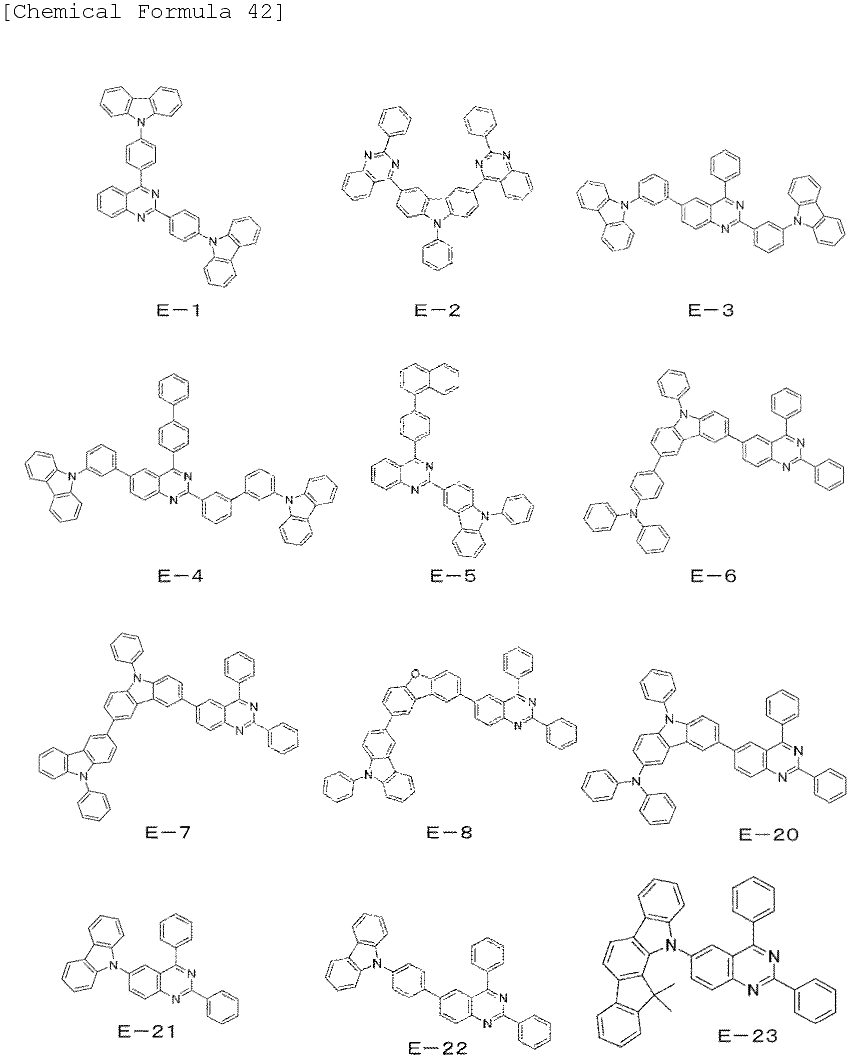

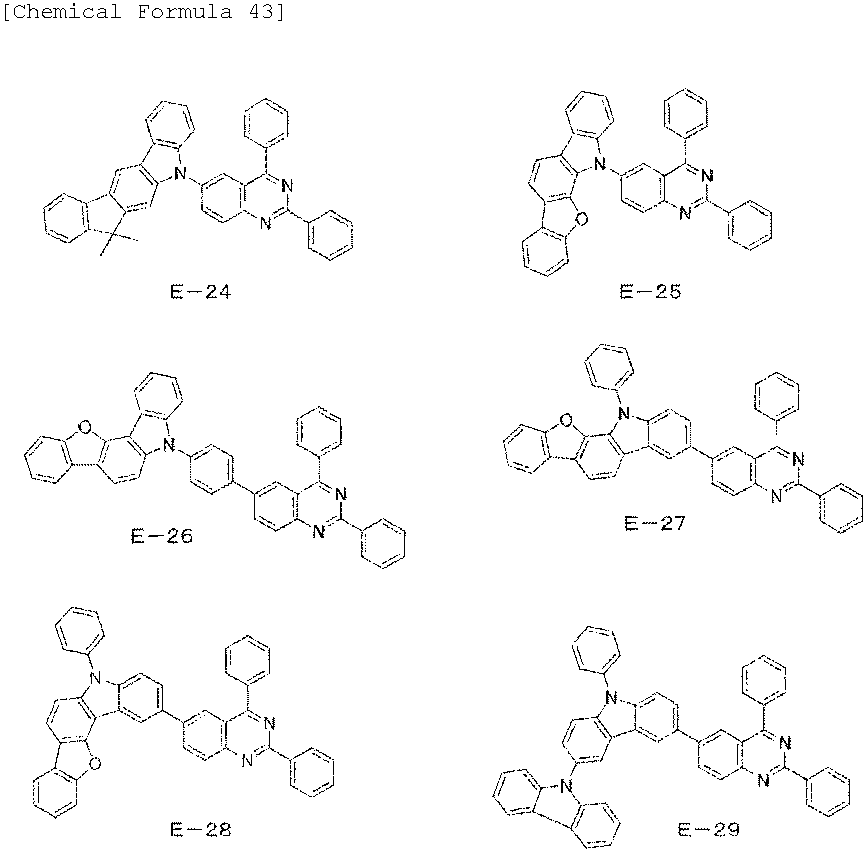

### Example 44

A glass substrate (manufactured by GEOMATEC Co., Ltd., 11 Ω/□, sputtered product) on which 165 nm of an ITO transparent conductive film was deposited was cut into a size of 38 × 46 mm, followed by etching. The obtained substrate was subjected to ultrasonic cleaning with "Semico Clean 56" (trade name, manufactured by Furuuchi Chemical Corporation) for 15 minutes, and then washed with ultrapure water. Immediately before the production of an element, this substrate was subjected to a UV ozone treatment for 1 hour. The substrate was placed in a vacuum vapor deposition device, and the inside of the device was evacuated until the degree of vacuum reached 5 × 10⁻⁴ Pa or less. First, a HAT-CN₆ layer having a thickness of 5 nm as a hole injection layer and a HT-1 layer having a thickness of 50 nm as a hole transport layer were vapor-deposited by a resistance heating method. Then, as a luminescent layer, a host material H-1 and a dopant material D-1 were vapor-deposited each to a thickness of 20 nm so that the doping concentration would be 5% by weight. Then, the compound [1] was vapor-deposited and laminated to a thickness of 25 nm as a first electron transport layer. Further, a second electron transport layer having a thickness of 10 nm was laminated using the compound [1] as an electron transport material and lithium as a donor material so that the deposition rate ratio between the compound [1] and lithium would be 20 : 1. Then, lithium fluoride was vapor-deposited to a thickness of 0.5 nm, and aluminum was vapor-deposited to a thickness of 1000 nm to form a negative electrode, thereby producing an element having a size of 5 mm × 5 mm. The film thickness referred to herein is an indicated value of a crystal oscillation type thickness monitor. Characteristics of this luminescent element at a luminance of 1000 cd/m² were a driving voltage of 3.87 V and an external quantum efficiency of 4.94%. Further, when the initial luminance was set to 1000 cd/m² and constant current driving was performed, the time until the luminance lowered by 20% was 1630 hours.

### Reference Examples 45 to 49 and 52 and Examples 50, 51 and 53 to 65

A luminescent element was produced and evaluated in the same manner as in Example 44, except that the compounds listed in Table 3 were used in the first electron transport layer and the second electron transport layer. The results are shown in Table 3. E-9 and E-10 are compounds shown below.

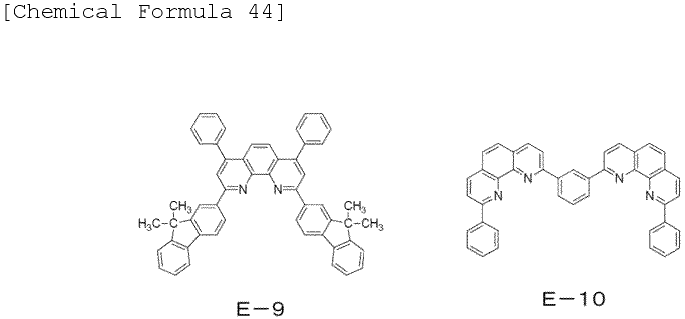

### Comparative Examples 19 to 40

A luminescent element was produced and evaluated in the same manner as in Example 44, except that the compounds listed in Table 3 were used in the first electron transport layer and the second electron transport layer. The results are shown in Table 3.

### Example 66

A glass substrate (manufactured by GEOMATEC Co., Ltd., 11 Ω/□, sputtered product) on which 165 nm of an ITO transparent conductive film was deposited was cut into a size of 38 × 46 mm, followed by etching. The obtained substrate was subjected to ultrasonic cleaning with "Semico Clean 56" (trade name, manufactured by Furuuchi Chemical Corporation) for 15 minutes, and then washed with ultrapure water. Immediately before the production of an element, this substrate was subjected to a UV ozone treatment for 1 hour. The substrate was placed in a vacuum vapor deposition device, and the inside of the device was evacuated until the degree of vacuum reached 5 × 10⁻⁴ Pa or less. First, a HAT-CN₆ layer having a thickness of 5 nm as a hole injection layer and a HT-1 layer having a thickness of 50 nm as a hole transport layer were vapor-deposited by a resistance heating method. Then, as a luminescent layer, a host material H-1 and a dopant material D-1 were vapor-deposited each to a thickness of 20 nm so that the doping concentration would be 5% by weight. Further, an electron transport layer having a thickness of 35 nm was laminated using the compound [1] as an electron transport material and 2E-1 as a donor material so that the deposition rate ratio between the compound [1] and 2E-1 would be 1 : 1. This electron transport layer is shown as a second electron transport layer in Table 3. Then, lithium fluoride was vapor-deposited to a thickness of 0.5 nm, and magnesium and silver were co-deposited to a thickness of 1000 nm to form a negative electrode, thereby producing an element having a size of 5 mm × 5 mm. The film thickness referred to herein is an indicated value of a crystal oscillation type thickness monitor. Characteristics of this luminescent element at a luminance of 1000 cd/m² were a driving voltage of 4.25 V and an external quantum efficiency of 5.39%. Further, when the initial luminance was set to 1000 cd/m² and constant current driving was performed, the time until the luminance lowered by 20% was 4020 hours.

### Examples 67 to 81

A luminescent element was produced and evaluated in the same manner as in Example 66, except that the compounds listed in Table 4 were used in the electron transport layer and the donor material. The results are shown in Table 4. 2E-1 is a compound shown below.

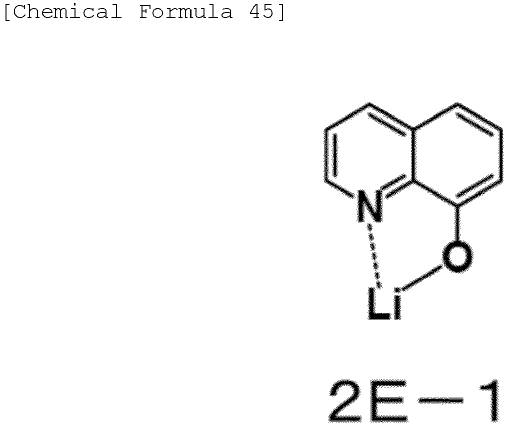

### Comparative Examples 41 to 55

A luminescent element was produced and evaluated in the same manner as in Example 28, except that the compounds listed in Table 4 were used in the electron transport layer and the donor material. The results are shown in Table 4.

### Example 82

A glass substrate (manufactured by GEOMATEC Co., Ltd., 11 Ω/□, sputtered product) on which 165 nm of an ITO transparent conductive film was deposited was cut into a size of 38 × 46 mm, followed by etching. The obtained substrate was subjected to ultrasonic cleaning with "Semico Clean 56" (trade name, manufactured by Furuuchi Chemical Corporation) for 15 minutes, and then washed with ultrapure water. Immediately before the production of an element, this substrate was subjected to a UV ozone treatment for 1 hour. The substrate was placed in a vacuum vapor deposition device, and the inside of the device was evacuated until the degree of vacuum reached 5 × 10⁻⁴ Pa or less. First, a HAT-CN₆ layer having a thickness of 5 nm as a hole injection layer and a HT-1 layer having a thickness of 50 nm as a hole transport layer were vapor-deposited by a resistance heating method. This hole transport layer is shown as a first hole transport layer in Table 5. Then, as a luminescent layer, a host material H-3 and a dopant material D-2 were vapor-deposited each to a thickness of 20 nm so that the doping concentration would be 10% by weight. Then, the compound [11] was vapor-deposited and laminated to a thickness of 35 nm as an electron transport layer. Then, lithium fluoride was vapor-deposited to a thickness of 0.5 nm, and aluminum was vapor-deposited to a thickness of 1000 nm to form a negative electrode, thereby producing an element having a size of 5 mm × 5 mm. The film thickness referred to herein is an indicated value of a crystal oscillation type thickness monitor. Characteristics of this luminescent element at a luminance of 4000 cd/m² were a driving voltage of 3.75 V and an external quantum efficiency of 10.51%. Further, when the initial luminance was set to 4000 cd/m² and constant current driving was performed, the time until the luminance lowered by 20% was 1680 hours. H-3 and D-2 are compounds shown below.

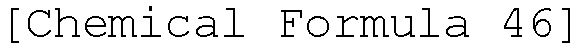

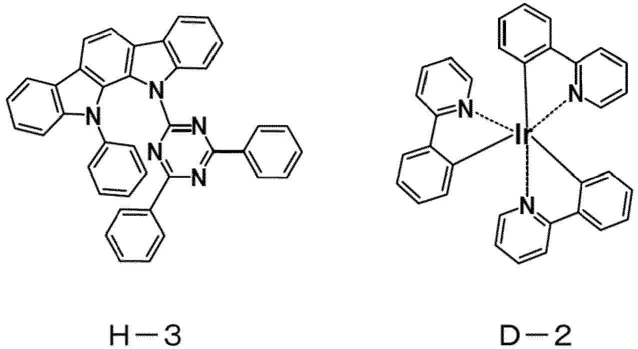

### Comparative Example 56

A luminescent element was produced and evaluated in the same manner as in Example 82, except that the compound shown in Table 5 was used in the electron transport layer. The results are shown in Table 5.

### Example 83

A glass substrate (manufactured by GEOMATEC Co., Ltd., 11 Ω/□, sputtered product) on which 165 nm of an ITO transparent conductive film was deposited was cut into a size of 38 × 46 mm, followed by etching. The obtained substrate was subjected to ultrasonic cleaning with "Semico Clean 56" (trade name, manufactured by Furuuchi Chemical Corporation) for 15 minutes, and then washed with ultrapure water. Immediately before the production of an element, this substrate was subjected to a UV ozone treatment for 1 hour. The substrate was placed in a vacuum vapor deposition device, and the inside of the device was evacuated until the degree of vacuum reached 5 × 10⁻⁴ Pa or less. First, a HAT-CN₆ layer having a thickness of 5 nm as a hole injection layer and a HT-1 layer having a thickness of 40 nm as a first hole transport layer were vapor-deposited by a resistance heating method. HT-2 was further vapor-deposited to a thickness of 10 nm as a second hole transport layer. Then, as a luminescent layer, a host material H-3 and a dopant material D-2 were vapor-deposited each to a thickness of 20 nm so that the doping concentration would be 10% by weight. Then, the compound [11] was vapor-deposited and laminated to a thickness of 35 nm as an electron transport layer. Then, lithium fluoride was vapor-deposited to a thickness of 0.5 nm, and aluminum was vapor-deposited to a thickness of 1000 nm to form a negative electrode, thereby producing an element having a size of 5 mm × 5 mm. The film thickness referred to herein is an indicated value of a crystal oscillation type thickness monitor. Characteristics of this luminescent element at a luminance of 4000 cd/m² were a driving voltage of 3.81 V and an external quantum efficiency of 14.36%. Further, when the initial luminance was set to 4000 cd/m² and constant current driving was performed, the time until the luminance lowered by 20% was 2060 hours. HT-2 is a compound shown below.

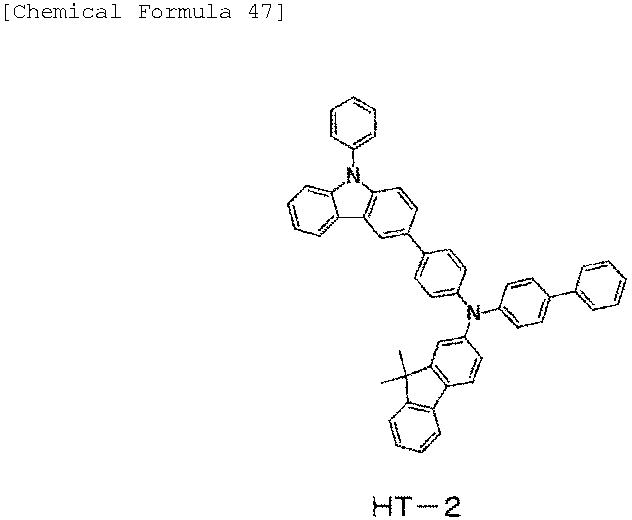

### Examples 84 to 89

An element was produced and evaluated in the same manner as in Example 83, except that the compound shown in Table 5 was used in the second hole transport layer. The results are shown in Table 5. HT-3 and HT-4 are compounds shown below.

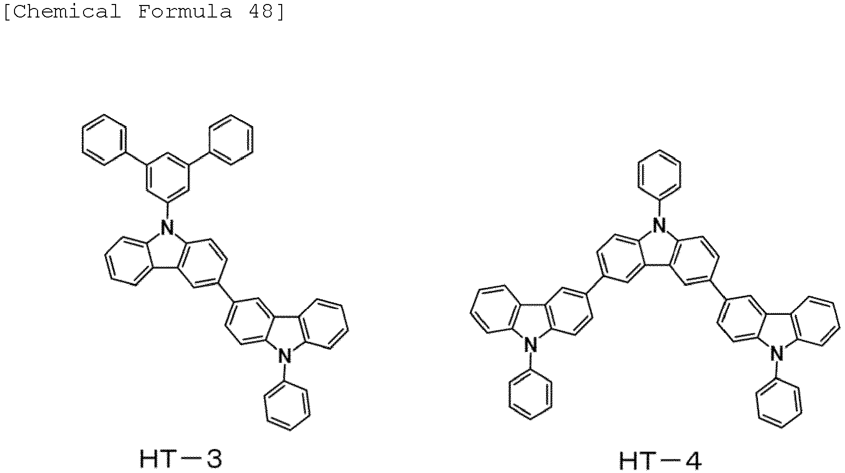

### Comparative Examples 57 to 66

An element was produced and evaluated in the same manner as in Example 83, except that the compounds listed in Table 5 were used in the second hole transport layer and the electron transport layer. The results are shown in Table 5.

### Example 90

A glass substrate (manufactured by GEOMATEC Co., Ltd., 11 Ω/□, sputtered product) on which 165 nm of an ITO transparent conductive film was deposited was cut into a size of 38 × 46 mm, followed by etching. The obtained substrate was subjected to ultrasonic cleaning with "Semico Clean 56" (trade name, manufactured by Furuuchi Chemical Corporation) for 15 minutes, and then washed with ultrapure water. Immediately before the production of an element, this substrate was subjected to a UV ozone treatment for 1 hour. The substrate was placed in a vacuum vapor deposition device, and the inside of the device was evacuated until the degree of vacuum reached 5 × 10⁻⁴ Pa or less. First, a HAT-CN₆ layer having a thickness of 5 nm as a hole injection layer and a HT-1 layer having a thickness of 50 nm as a hole transport layer were vapor-deposited by a resistance heating method. Then, as a luminescent layer, a host material H-1 and a dopant material D-1 were vapor-deposited each to a thickness of 20 nm so that the doping concentration would be 5% by weight. Then, the compound [11] was vapor-deposited and laminated to a thickness of 20 nm as a first electron transport layer. Then, the compound [E-10] as an electron transport material was vapor-deposited and laminated to a thickness of 20 nm as a second electron transport layer. Then, a charge generating layer having a thickness of 10 nm was laminated using lithium as a donor material so that the deposition rate ratio between the compound [E-10] and lithium would be 20 : 1. Then, lithium fluoride was vapor-deposited to a thickness of 0.5 nm, and aluminum was vapor-deposited to a thickness of 1000 nm to form a negative electrode, thereby producing an element having a size of 5 mm × 5 mm. The film thickness referred to herein is an indicated value of a crystal oscillation type thickness monitor. Characteristics of this luminescent element at a luminance of 1000 cd/m² were a driving voltage of 3.98 V and an external quantum efficiency of 5.64%. Further, when the initial luminance was set to 1000 cd/m² and constant current driving was performed, the time until the luminance lowered by 20% was 1930 hours.

### Comparative Example 67

A luminescent element was produced and evaluated in the same manner as in Example 90, except that the compound shown in Table 6 was used in the first electron transport layer. The results are shown in Table 6.

### Example 91

A luminescent element was produced and evaluated in the same manner as in Example 90, except that the element was produced without laminating the second electron transport layer. The results are shown in Table 6.

### Comparative Example 68

A luminescent element was produced and evaluated in the same manner as in Example 90, except that the element was produced without laminating the first electron transport layer. The results are shown in Table 6.

### Comparative Example 69

A luminescent element was produced and evaluated in the same manner as in Comparative Example 67, except that the element was produced without laminating the second electron transport layer. The results are shown in Table 6.

### Comparative Examples 70 to 76

A luminescent element was produced and evaluated in the same manner as in Example 90, except that the compounds listed in Table 6 were used in the first electron transport layer and the second electron transport layer. The results are shown in Table 6.

### Example 92

A glass substrate (manufactured by GEOMATEC Co., Ltd., 11 Ω/□, sputtered product) on which 165 nm of an ITO transparent conductive film was deposited was cut into a size of 38 × 46 mm, followed by etching. The obtained substrate was subjected to ultrasonic cleaning with "Semico Clean 56" (trade name, manufactured by Furuuchi Chemical Corporation) for 15 minutes, and then washed with ultrapure water. Immediately before the production of an element, this substrate was subjected to a UV ozone treatment for 1 hour. The substrate was placed in a vacuum vapor deposition device, and the inside of the device was evacuated until the degree of vacuum reached 5 × 10⁻⁴ Pa or less. First, a HAT-CN₆ layer having a thickness of 5 nm as a hole injection layer and a HT-1 layer having a thickness of 50 nm as a hole transport layer were vapor-deposited by a resistance heating method. Then, as a luminescent layer, a host material H-1 and a dopant material D-1 were vapor-deposited each to a thickness of 20 nm so that the doping concentration would be 5% by weight. Then, the compound [11] was vapor-deposited and laminated to a thickness of 20 nm as a first electron transport layer. Further, the compound [E-11] was laminated to a thickness of 35 nm as a second electron transport layer. Then, lithium fluoride was vapor-deposited to a thickness of 0.5 nm, and magnesium and silver were co-deposited to a thickness of 1000 nm to form a negative electrode, thereby producing an element having a size of 5 mm × 5 mm. The film thickness referred to herein is an indicated value of a crystal oscillation type thickness monitor. Characteristics of this luminescent element at a luminance of 1000 cd/m² were a driving voltage of 3.58 V and an external quantum efficiency of 6.11%. Further, when the initial luminance was set to 1000 cd/m² and constant current driving was performed, the time until the luminance lowered by 20% was 4140 hours. E-11 is a compound shown below.

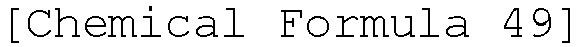

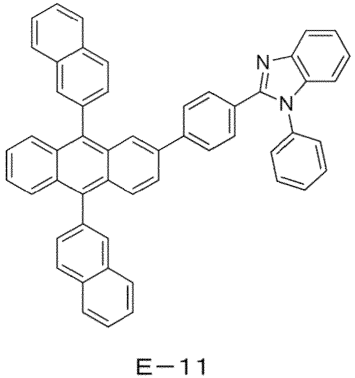

### Comparative Examples 77, 79, 81, 83, 85, and 87

A luminescent element was produced and evaluated in the same manner as in Example 92, except that the compound shown in Table 7 was used in the first electron transport layer. The results are shown in Table 7.

### Example 93

A glass substrate (manufactured by GEOMATEC Co., Ltd., 11 Ω/□, sputtered product) on which 165 nm of an ITO transparent conductive film was deposited was cut into a size of 38 × 46 mm, followed by etching. The obtained substrate was subjected to ultrasonic cleaning with "Semico Clean 56" (trade name, manufactured by Furuuchi Chemical Corporation) for 15 minutes, and then washed with ultrapure water. Immediately before the production of an element, this substrate was subjected to a UV ozone treatment for 1 hour. The substrate was placed in a vacuum vapor deposition device, and the inside of the device was evacuated until the degree of vacuum reached 5 × 10⁻⁴ Pa or less. First, a HAT-CN₆ layer having a thickness of 5 nm as a hole injection layer and a HT-1 layer having a thickness of 50 nm as a hole transport layer were vapor-deposited by a resistance heating method. Then, as a luminescent layer, a host material H-1 and a dopant material D-1 were vapor-deposited each to a thickness of 20 nm so that the doping concentration would be 5% by weight. Then, the compound [11] was vapor-deposited and laminated to a thickness of 20 nm as a first electron transport layer. Further, a second electron transport layer having a thickness of 35 nm was laminated using the compound [E-11] as an electron transport material and 2E-1 as a donor material so that the deposition rate ratio between the compound [E-11] and 2E-1 would be 1 : 1. Then, lithium fluoride was vapor-deposited to a thickness of 0.5 nm, and magnesium and silver were co-deposited to a thickness of 1000 nm to form a negative electrode, thereby producing an element having a size of 5 mm × 5 mm. The film thickness referred to herein is an indicated value of a crystal oscillation type thickness monitor. Characteristics of this luminescent element at a luminance of 1000 cd/m² were a driving voltage of 4.05 V and an external quantum efficiency of 6.07%. Further, when the initial luminance was set to 1000 cd/m² and constant current driving was performed, the time until the luminance lowered by 20% was 4570 hours.

### Comparative Examples 78, 80, 82, 84, 86, and 88

A luminescent element was produced and evaluated in the same manner as in Example 93, except that the compound shown in Table 7 was used in the first electron transport layer. The results are shown in Table 7.

### Examples 94 to 117

A luminescent element was produced and evaluated in the same manner as in Example 1, except that the compounds listed in Table 8 were used in the host material of the luminescent layer and the electron transport layer. The results are shown in Table 8. H-3 is a compound shown below.

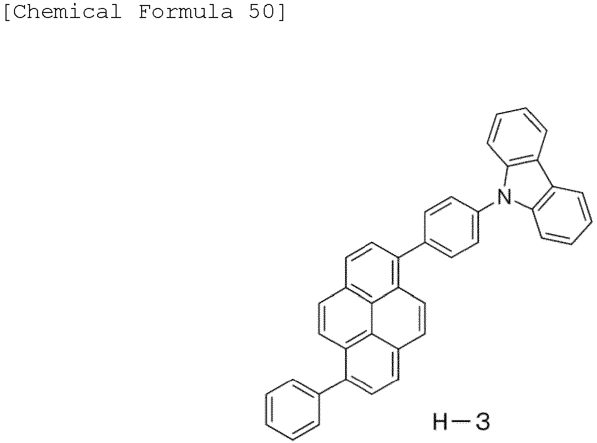

**[Table 1] In Table 1, Examples 1 to 6, 9, 12, 29 to 32, 41 and 42 are Reference Examples.**

| | Luminescent material | | | Electron transport layer | Negative electrode | External quantum efficiency (%) | Driving voltage (V) | Durability (h) |
|---|---|---|---|---|---|---|---|---|
| | Host material | Dopant material | Luminescent color | Compound type | Metal | | | |
| Example 1 | | | Blue color | [1] | Al | 4.22 | 4.63 | 1650 |
| Example 2 | | | Blue color | [2] | Al | 3.78 | 4.79 | 1440 |
| Example 3 | | | Blue color | [3] | Al | 4.07 | 4.87 | 1530 |
| Example 4 | | | Blue color | [4] | Al | 3.77 | 4.86 | 1510 |
| Example 5 | | | Blue color | [5] | Al | 3.64 | 5.04 | 1330 |
| Example 6 | | | Blue color | [6] | Al | 3.20 | 5.21 | 1260 |
| Example 7 | | | Blue color | [7] | Al | 4.51 | 4.54 | 1710 |
| Example 8 | | | Blue color | [8] | Al | 4.65 | 4.38 | 1910 |
| Example 9 | | | Blue color | [9] | Al | 3.35 | 5.19 | 1220 |
| Example 10 | | | Blue color | [10] | Al | 4.64 | 4.30 | 1830 |
| Example 11 | | | Blue color | [11] | Al | 4.82 | 4.37 | 1840 |
| Example 12 | | | Blue color | [12] | Al | 4.06 | 4.78 | 1460 |
| Example 13 | | | Blue color | [13] | Al | 5.12 | 4.11 | 2230 |
| Example 14 | | | Blue color | [14] | Al | 5.07 | 4.12 | 2220 |
| Example 15 | | | Blue color | [15] | Al | 5.08 | 4.12 | 2230 |
| Example 16 | | | Blue color | [16] | Al | 4.34 | 4.13 | 1770 |
| Example 17 | | | Blue color | [17] | Al | 5.08 | 4.12 | 2200 |
| Example 18 | | | Blue color | [18] | Al | 5.1 | 4.12 | 2190 |
| Example 19 | | | Blue color | [19] | Al | 4.13 | 4.24 | 1730 |
| Example 20 | | | Blue color | [20] | Al | 4.12 | 4.23 | 1720 |
| Example 21 | H-1 | D-1 | Blue color | [21] | Al | 5.13 | 4.12 | 2190 |
| Example 22 | | | Blue color | [22] | Al | 5.1 | 4.12 | 2200 |
| Example 23 | | | Blue color | [23] | Al | 5.07 | 4.13 | 2210 |
| Example 24 | | | Blue color | [24] | Al | 4.34 | 4.14 | 1670 |
| Example 25 | | | Blue color | [25] | Al | 5.07 | 4.12 | 2200 |
| Example 26 | | | Blue color | [26] | Al | 5.09 | 4.12 | 2180 |
| Example 27 | | | Blue color | [27] | Al | 4.12 | 4.23 | 1750 |
| Example 28 | | | Blue color | [28] | Al | 4.13 | 4.24 | 1730 |
| Example 29 | | | Blue color | [29] | Al | 4.81 | 4.62 | 2100 |
| Example 30 | | | Blue color | [30] | Al | 4.34 | 4.73 | 1770 |
| Example 31 | | | Blue color | [31] | Al | 4.84 | 4.61 | 2120 |
| Example 32 | | | Blue color | [32] | Al | 4.35 | 4.72 | 1760 |
| Example 33 | | | Blue color | [33] | Al | 5.13 | 4.11 | 2220 |
| Example 34 | | | Blue color | [34] | Al | 5.1 | 4.12 | 2020 |
| Example 35 | | | Blue color | [35] | Al | 5.09 | 4.12 | 2030 |
| Example 36 | | | Blue color | [36] | Al | 5.13 | 4.11 | 2030 |
| Example 37 | | | Blue color | [37] | Al | 5.01 | 4.13 | 2190 |
| Example 38 | | | Blue color | [38] | Al | 5.09 | 4.12 | 2210 |
| Example 39 | | | Blue color | [39] | Al | 4.21 | 4.22 | 1740 |
| Example 40 | | | Blue color | [40] | Al | 4.22 | 4.23 | 1730 |
| Example 41 | | | Blue color | [41] | Al | 4.81 | 4.47 | 1600 |
| Example 42 | | | Blue color | [42] | Al | 4.78 | 4.49 | 1550 |
| Example 43 | H-2 | D-1 | Blue color | [10] | Al | 4.51 | 4.36 | 1770 |

**[Table 2]**

| | Luminescent material | | | Electron transport layer | Negative electrode | External quantum efficiency (%) | Driving voltage (V) | Durability (h) |
|---|---|---|---|---|---|---|---|---|
| | Host material | Dopant material | Luminescent color | Compound type | Metal | | | |
| Comparative Example 1 | | | Blue color | E-1 | Al | 1.43 | 6.21 | 350 |
| Comparative Example 2 | | | Blue color | E-2 | Al | 1.38 | 6.29 | 400 |
| Comparative Example 3 | | | Blue color | E-3 | Al | 1.44 | 6.25 | 270 |
| Comparative Example 4 | | | Blue color | E-4 | Al | 1.62 | 6.23 | 280 |
| Comparative Example 5 | | | Blue color | E-5 | Al | 1.36 | 6.14 | 220 |
| Comparative Example 6 | | | Blue color | E-6 | Al | 1.53 | 6.25 | 350 |
| Comparative Example 7 | | | Blue color | E-7 | Al | 1.55 | 6.18 | 330 |
| Comparative Example 8 | | | Blue color | E-8 | Al | 1.47 | 6.27 | 360 |
| Comparative Example 9 | H-1 | D-1 | Blue color | E-20 | Al | 1.52 | 6.23 | 390 |
| Comparative Example 10 | | | Blue color | E-21 | Al | 1.46 | 6.24 | 370 |
| Comparative Example 11 | | | Blue color | E-22 | Al | 1.43 | 6.19 | 360 |
| Comparative Example 12 | | | Blue color | E-23 | Al | 1.42 | 6.24 | 370 |
| Comparative Example 13 | | | Blue color | E-24 | Al | 1.48 | 6.21 | 390 |
| Comparative Example 14 | | | Blue color | E-25 | Al | 1.47 | 6.31 | 350 |
| Comparative Example 15 | | | Blue color | E-26 | Al | 1.51 | 6.27 | 320 |
| Comparative Example 16 | | | Blue color | E-27 | Al | 1.52 | 6.25 | 370 |
| Comparative Example 17 | | | Blue color | E-28 | Al | 1.47 | 6.23 | 290 |
| Comparative Example 18 | | | Blue color | E-29 | Al | 1.48 | 6.24 | 310 |

In Table 3, Examples 44 to 49, 52 and 55 are Reference Examples.

**[Table 3]**

| | Luminescent material | | | First electron transport layer | Second electron transport layer | | Negative electrode | External quantum efficiency | Driving voltage (V) | Durability (h) |
|---|---|---|---|---|---|---|---|---|---|---|
| | Host material | Dopant material | Luminescent color | Compound type | Compound type | Donor compound type | Metal | (%) | | |
| Example 44 | | | Blue color | [1] | [1] | Li | Al | 4.94 | 3.87 | 1630 |
| Example 45 | | | Blue color | [2] | [2] | Li | Al | 4.43 | 3.99 | 1480 |
| Example 46 | | | Blue color | [3] | [3] | Li | Al | 4.77 | 4.05 | 1580 |
| Example 47 | | | Blue color | [4] | [4] | Li | Al | 4.42 | 4.07 | 1560 |
| Example 48 | | | Blue color | [5] | [5] | Li | Al | 4.26 | 4.21 | 1380 |
| Example 49 | | | Blue color | [6] | [6] | Li | Al | 3.75 | 4.37 | 1290 |
| Example 50 | | | Blue color | [7] | [7] | Li | Al | 5.28 | 3.79 | 1670 |
| Example 51 | | | Blue color | [8] | [8] | Li | Al | 5.45 | 3.66 | 1880 |
| Example 52 | | | Blue color | [9] | [9] | Li | Al | 3.92 | 4.34 | 1310 |
| Example 53 | | | Blue color | [10] | [10] | Li | Al | 5.43 | 3.59 | 1740 |
| Example 54 | H-1 | D-1 | Blue color | [11] | [11] | Li | Al | 5.62 | 3.65 | 1730 |
| Example 55 | | | Blue color | [12] | [12] | Li | Al | 4.75 | 4.01 | 1650 |
| Example 56 | | | Blue color | [10] | E-9 | Li | Al | 4.46 | 9.11 | 1430 |
| Example 57 | | | Blue color | [10] | E-10 | Li | Al | 4.93 | 3.91 | 1570 |
| Example 58 | | | Blue color | [13] | [13] | Li | Al | 5.53 | 3.60 | 2320 |
| Example 59 | | | Blue color | [14] | [14] | Li | Al | 5.47 | 3.61 | 2290 |
| Example 60 | | | Blue color | [15] | [15] | Li | Al | 5.51 | 3.62 | 2280 |
| Example 61 | | | Blue color | [17] | [17] | Li | Al | 5.41 | 3.63 | 2210 |
| Example 62 | | | Blue color | [13] | E-10 | Li | Al | 5.82 | 3.48 | 2520 |
| Example 63 | | | Blue color | [14] | E-10 | Li | Al | 5.83 | 3.48 | 2530 |
| Example 64 | | | Blue color | [15] | E-10 | Li | Al | 5.81 | 3.49 | 2520 |
| Example 65 | | | Blue color | [17] | E-10 | Li | Al | 5.80 | 3.48 | 2530 |
| Comparative Example 19 | | | Blue color | E-1 | E-1 | Li | Al | 2.41 | 5.97 | 500 |
| Comparative Example 20 | | | Blue color | E-2 | E-2 | Li | Al | 2.33 | 6.05 | 530 |
| Comparative Example 21 | | | Blue color | E-3 | E-3 | Li | Al | 2.42 | 6.01 | 390 |
| Comparative Example 22 | | | Blue color | E-4 | E-4 | Li | Al | 2.73 | 5.99 | 400 |
| Comparative Example 23 | | | Blue color | E-5 | E-5 | Li | Al | 2.30 | 5.91 | 310 |
| Comparative Example 24 | | | Blue color | E-6 | E-6 | Li | Al | 2.57 | 6.01 | 500 |
| Comparative Example 25 | | | Blue color | E-7 | E-7 | Li | Al | 2.61 | 5.94 | 470 |
| Comparative Example 26 | | | Blue color | E-8 | E-8 | Li | Al | 2.47 | 6.03 | 520 |
| Comparative Example 27 | | | Blue color | E-9 | E-9 | Li | Al | 2.34 | 5.90 | 510 |
| Comparative Example 28 | | | Blue color | E-10 | E-10 | Li | Al | 2.46 | 5.92 | 490 |
| Comparative Example 29 | H-1 | D-1 | Blue color | E-20 | E-20 | Li | Al | 2.35 | 5.87 | 440 |
| Comparative Example 30 | | | Blue color | E-21 | E-21 | Li | Al | 2.37 | 5.79 | 460 |
| Comparative Example 31 | | | Blue color | E-22 | E-22 | Li | Al | 2.37 | 5.75 | 430 |
| Comparative Example 32 | | | Blue color | E-23 | E-23 | Li | Al | 2.41 | 5.83 | 450 |
| Comparative Example 33 | | | Blue color | E-24 | E-24 | Li | Al | 2.34 | 5.81 | 470 |
| Comparative Example 34 | | | Blue color | E-27 | E-27 | Li | Al | 2.36 | 5.77 | 450 |
| Comparative Example 35 | | | Blue color | E-29 | E-29 | Li | Al | 2.41 | 5.86 | 490 |
| Comparative Example 36 | | | Blue color | E-6 | E-10 | Li | Al | 2.60 | 5.10 | 520 |
| Comparative Example 37 | | | Blue color | E-7 | E-10 | Li | Al | 2.61 | 5.11 | 500 |
| Comparative Example 38 | | | Blue color | E-20 | E-20 | Li | Al | 2.64 | 5.13 | 470 |
| Comparative Example 39 | | | Blue color | E-27 | E-10 | Li | Al | 2.60 | 5.13 | 470 |
| Comparative Example 40 | | | Blue color | E-29 | E-10 | Li | Al | 2.59 | 5.15 | 500 |

In Table 4, Examples 66 to 71, 74 and 77 are Reference Examples.

**[Table 4]**

| | Luminescent material | | | First electron transport layer | Second electron transport layer | | Negative electrode | External quantum efficiency | Driving voltage (V) | Durability (h) |
|---|---|---|---|---|---|---|---|---|---|---|
| | Host material | Dopant material | Luminescent color | Compound type | Compound type | Donor compound type | Metal | (%) | | |
| Example 66 | | | Blue color | None | [1] | 2E-1 | Mg/Ag | 5.39 | 4.25 | 4020 |
| Example 67 | | | Blue color | None | [2] | 2E-1 | Mg/Ag | 4.83 | 4.39 | 3520 |
| Example 68 | | | Blue color | None | [3] | 2E-1 | Mg/Ag | 5.20 | 4.44 | 3690 |
| Example 69 | | | Blue color | Hone | [4] | 2E-1 | Mg/Ag | 4.82 | 4.47 | 3670 |
| Example 70 | | | Blue color | None | [5] | 2E-1 | Mg/Ag | 4.63 | 4.60 | 3230 |
| Example 71 | | | Blue color | None | [6] | 2E-1 | Mg/Ag | 4.09 | 4.76 | 3060 |
| Example 72 | | | Blue color | None | [7] | 2E-1 | Mg/Ag | 5.76 | 4.17 | 4170 |
| Example 73 | H-1 | D-1 | Blue color | None | [8] | 2E-1 | Mg/Ag | 5.94 | 4.03 | 4660 |
| Example 74 | | | Blue color | None | [9] | 2E-1 | Mg/Ag | 4.26 | 4.75 | 2970 |
| Example 75 | | | Blue color | None | [10] | 2E-1 | Mg/Ag | 5.93 | 3.95 | 4460 |
| Example 76 | | | Blue color | None | [11] | 2E-1 | Mg/Ag | 6.13 | 4.01 | 4480 |
| Example 77 | | | Blue color | None | [12] | 2E-1 | Mg/Ag | 5.19 | 4.39 | 3550 |
| Example 78 | | | Blue color | None | [13] | 2E-1 | Mg/Ag | 6.34 | 4.02 | 4720 |
| Example 79 | | | Blue color | None | [14] | 2E-1 | Mg/Ag | 6.34 | 4.03 | 4740 |
| Example 80 | | | Blue color | None | [15] | 2E-1 | Mg/Ag | 6.32 | 4.02 | 4700 |
| Example 81 | | | Blue color | None | [17] | 2E-1 | Mg/Ag | 6.34 | 4.01 | 4730 |
| Comparative Example 41 | | | Blue color | None | E-1 | 2E-1 | Mg/Ag | 2.85 | 6.22 | 950 |
| Comparative Example 42 | | | Blue color | None | E-2 | 2E-1 | Mg/Ag | 2.75 | 6.28 | 990 |
| Comparative Example 43 | | | Blue color | None | E-3 | 2E-1 | Mg/Ag | 2.87 | 6.26 | 770 |
| Comparative Example 44 | | | Blue color | None | E-4 | 2E-1 | Mg/Ag | 3.23 | 6.23 | 780 |
| Comparative Example 45 | | | Blue color | None | E-5 | 2E-1 | Mg/Ag | 2.71 | 6.16 | 760 |
| Comparative Example 46 | | | Blue color | None | E-6 | 2E-1 | Mg/Ag | 2.96 | 6.24 | 970 |
| Comparative Example 47 | | | Blue color | None | E-7 | 2E-1 | Mg/Ag | 2.99 | 6.20 | 910 |
| Comparative Example 48 | H-1 | D-1 | Blue color | None | E-8 | 2E-1 | Mg/Ag | 2.84 | 6.45 | 930 |
| Comparative Example 49 | | | Blue color | None | E-20 | 2E-1 | Mg/Ag | 3.01 | 6.31 | 900 |
| Comparative Example 50 | | | Blue color | None | E-21 | 2E-1 | Mg/Ag | 2.95 | 6.32 | 940 |
| Comparative Example 51 | | | Blue color | None | E-22 | 2E-1 | Mg/Ag | 2.97 | 6.43 | 930 |
| Comparative Example 52 | | | Blue color | None | E-23 | 2E-1 | Mg/Ag | 2.98 | 6.35 | 920 |
| Comparative Example 53 | | | Blue color | None | E-24 | 2E-1 | Mg/Ag | 2.94 | 6.33 | 930 |
| Comparative Example 54 | | | Blue color | None | E-27 | 2E-1 | Mg/Ag | 2.91 | 6.32 | 950 |
| Comparative Example 55 | | | Blue color | None | E-29 | 2E-1 | Mg/Ag | 2.95 | 6.38 | 940 |

**[Table 5]**

| | Hole injection layer | First hole transport layer | Second hole transport layer | Luminescent layer | | Electron transport layer | External quantum efficiency (%) | Driving voltage (V) | Durability (h) |
|---|---|---|---|---|---|---|---|---|---|
| | | | | Host material | Dopant material | | | | |
| Example 82 | HAT-CN6 | HT-1 | None | H-3 | D-2 | [11] | 10.51 | 3.75 | 1680 |
| Example 83 | | | HT-2 | | | | 14.36 | 3.81 | 2060 |
| Example 84 | | | HT-3 | | | | 17.90 | 3.85 | 2870 |
| Example 85 | | | HT-4 | | | | 18.84 | 3.83 | 3240 |
| Example 86 | | | HT-4 | | | [13] | 18.97 | 3.82 | 3250 |
| Example 87 | | | HT-4 | | | [14] | 18.94 | 3.83 | 3230 |
| Example 88 | | | HT-4 | | | [15] | 18.93 | 3.82 | 3230 |
| Example 89 | | | HT-4 | | | [17] | 18.96 | 3.84 | 3210 |
| Comparative Example 56 | HAT-CN6 | HT-1 | None | H-3 | D-2 | E-1 | 6.14 | 7.72 | 340 |
| Comparative Example 57 | | | HT-2 | | | | 6.33 | 7.73 | 610 |
| Comparative Example 58 | | | HT-3 | | | | 6.37 | 7.76 | 830 |
| Comparative Example 59 | | | HT-4 | | | | 6.36 | 7.7 | 820 |
| Comparative Example 60 | | | HT-4 | | | E-20 | 6.4 | 7.69 | 840 |
| Comparative Example 61 | | | HT-4 | | | E-21 | 6.39 | 7.68 | 820 |
| Comparative Example 62 | | | HT-4 | | | E-22 | 6.38 | 7.71 | 810 |
| Comparative Example 63 | | | HT-4 | | | E-23 | 6.39 | 7.72 | 830 |
| Comparative Example 64 | | | HT-4 | | | E-24 | 6.41 | 7.68 | 840 |
| Comparative Example 65 | | | HT-4 | | | E-27 | 6.41 | 7.71 | 940 |
| Comparative Example 66 | | | HT-4 | | | E-29 | 6.38 | 7.70 | 820 |

**[Table 6]**

| | Luminescent material | | | First electron transport layer | Second electron transport layer | Charge generating layer | | Negative electrode | External quantum efficiency (%) | Driving voltage (V) | Durability (h) |
|---|---|---|---|---|---|---|---|---|---|---|---|
| | Host material | Dopant material | Luminescent color | Compound type | Compound type | Compound type | Donor compound type | Metal | | | |
| Example 90 | H-1 | D-1 | Blue color | [11] | E-10 | E-10 | Li | Al | 5.64 | 3.98 | 1930 |
| Example 91 | | | Blue color | [11] | None | E-10 | Li | Al | 5.47 | 3.67 | 1640 |
| Comparative Example 67 | H-1 | D-1 | Blue color | E-1 | E-10 | E-10 | Li | Al | 2.44 | 5.98 | 530 |
| comparative Example 68 | | | Blue color | None | E-10 | E-10 | Li | Al | 2.10 | 5.66 | 340 |
| Comparative Example 69 | | | Blue color | E-1 | None | E-10 | Li | Al | 2.07 | 6.11 | 370 |
| Comparative Example 70 | | | Blue color | E-2 | E-10 | E-10 | Li | Al | 2.31 | 6.05 | 490 |
| Comparative Example 71 | | | Blue color | E-3 | E-10 | E-10 | Li | Al | 2.45 | 6.02 | 410 |
| Comparative Example 72 | | | Blue color | E-4 | E-10 | E-10 | Li | Al | 2.72 | 6.01 | 420 |
| Comparative Example 73 | | | Blue color | E-5 | E-10 | E-10 | Li | Al | 2.34 | 5.93 | 330 |
| Comparative Example 74 | | | Blue color | E-6 | E-10 | E-10 | Li | Al | 2.54 | 6.03 | 510 |
| Comparative Example 75 | | | Blue color | E-7 | E-10 | E-10 | Li | Al | 2.56 | 5.97 | 480 |
| Comparative Example 76 | | | Blue color | E-8 | E-10 | E-10 | Li | Al | 2.46 | 6.06 | 520 |

**[Table 7]**

| | Luminescent material | | | First electron transport layer | Second electron transport layer | | Negative electrode | External quantum efficiency (% ) | Driving voltage (V) | Durability (h) |
|---|---|---|---|---|---|---|---|---|---|---|
| | Host material | Dopant material | Luminescent color | Compound type | Compound type | Donor compound type | Metal | | | |
| Example 92 | H-1 | D-1 | Blue color | [11] | E-11 | None | Mg/Ag | 6.11 | 3.58 | 4140 |
| Example 93 | | | Blue color | | E-11 | 2E-1 | Mg/Ag | 6.07 | 4.05 | 4570 |
| Comparative Example 77 | H-1 | D-1 | Blue color | None | E-11 | None | Mg/Ag | 2.55 | 6.02 | 570 |
| Comparative Example 78 | | | Blue color | | E-11 | 2E-1 | Mg/Ag | 2.53 | 6.86 | 730 |
| Comparative Example 79 | | | Blue color | E-1 | E-11 | None | Mg/Ag | 2.46 | 6.01 | 730 |
| Comparative Example 80 | | | Blue color | | E-11 | 2E-1 | Mg/Ag | 2.38 | 6.83 | 980 |
| Comparative Example 81 | | | Blue color | E-2 | E-11 | None | Mg/Ag | 2.37 | 6.08 | 720 |
| Comparative Example 82 | | | Blue color | | E-11 | 2E-1 | Mg/Ag | 2.29 | 6.84 | 990 |
| Comparative Example 83 | | | Blue color | E-3 | E-11 | None | Mg/Ag | 2.47 | 6.05 | 680 |
| Comparative Example 84 | | | Blue color | | E-11 | 2E-1 | Mg/Ag | 2.36 | 6.03 | 870 |
| Comparative Example 85 | | | Blue color | E-5 | E-11 | None | Mg/Ag | 2.33 | 6.02 | 670 |
| Comparative Example 86 | | | Blue color | | E-11 | 2E-1 | Mg/Ag | 2.18 | 6.77 | 890 |
| Comparative Example 87 | | | Blue color | E-6 | E-11 | None | Mg/Ag | 2.56 | 6.07 | 740 |
| Comparative Example 88 | | | Blue color | | E-11 | 2E-1 | Mg/Ag | 2.42 | 6.82 | 970 |

**[Table 8]**

| | Luminescent material | | | Electron transport layer | Negative electrode | External quantum efficiency (%) | Driving voltage (V) | Durability (h) |
|---|---|---|---|---|---|---|---|---|
| | Host material | Dopant material | Luminescent color | Compound type | Metal | | | |
| Example 94 | H-3 | D-1 | Blue color | [13] | Al | 4.76 | 4.21 | 1870 |
| Example 95 | | | Blue color | [14] | Al | 4.77 | 4.19 | 1890 |
| Example 96 | | | Blue color | [15] | Al | 4.74 | 4.18 | 1880 |
| Example 97 | | | Blue color | [16] | Al | 4.12 | 4.19 | 1530 |
| Example 98 | | | Blue color | [17] | Al | 4.76 | 4.2 | 1860 |
| Example 99 | | | Blue color | [18] | Al | 4.72 | 4.22 | 1810 |
| Example 100 | | | Blue color | [21] | Al | 4.71 | 4.2 | 1850 |
| Example 101 | | | Blue color | [22] | Al | 4.69 | 4.23 | 1830 |
| Example 102 | | | Blue color | [23] | Al | 4.73 | 4.23 | 1830 |
| Example 103 | | | Blue color | [24] | Al | 4.09 | 4.21 | 1460 |
| Example 104 | | | Blue color | [25] | Al | 4.76 | 4.23 | 1880 |
| Example 105 | | | Blue color | [26] | Al | 4.72 | 4.2 | 1840 |
| Example 106 | H-2 | D-1 | Blue color | [13] | Al | 4.63 | 4.2 | 1760 |
| Example 107 | | | Blue color | [14] | Al | 4.64 | 4.2 | 1740 |
| Example 108 | | | Blue color | [15] | Al | 4.62 | 4.18 | 1780 |
| Example 109 | | | Blue color | [16] | Al | 4.03 | 4.21 | 1760 |
| Example 110 | | | Blue color | [17] | Al | 4.65 | 4.21 | 1750 |
| Example 111 | | | Blue color | [18] | Al | 4.64 | 4.23 | 1720 |
| Example 112 | | | Blue color | [21] | Al | 4.62 | 4.21 | 1730 |
| Example 113 | | | Blue color | [22] | Al | 4.6 | 4.21 | 1710 |
| Example 114 | | | Blue color | [23] | Al | 4.61 | 4.22 | 1750 |
| Example 115 | | | Blue color | [24] | Al | 4 | 4.21 | 1760 |
| Example 116 | | | Blue color | [25] | Al | 4.61 | 4.21 | 1740 |
| Example 117 | | | Blue color | [26] | Al | 4.64 | 4.19 | 1730 |

## Claims

1. A compound represented by the following general formula (4) or (5) : 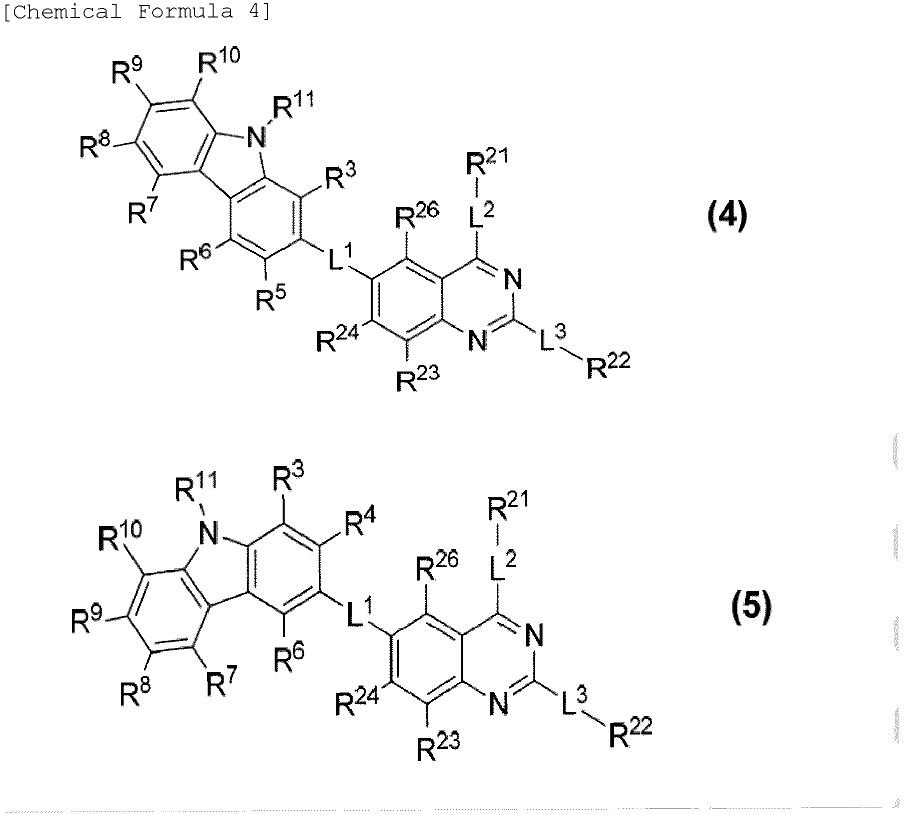
wherein L¹ is a single bond
wherein R³ to R¹⁰ are each a hydrogen atom except at the position that is linked to L¹,
R¹¹ is selected from the group consisting of a hydrogen atom, an alkyl group, a cycloalkyl group, a heterocyclic group, an alkenyl group, a cycloalkenyl group, an aryl group, and a heteroaryl group,
wherein L² is a single bond, a phenylene group, a naphthalenylene group, or a biphenylene group,
R²¹ is a hydrogen atom, a halogen atom, a carbazolyl group, a benzocarbazolyl group, a dibenzofuranyl group, or a dibenzothiophenyl group,
L³ is a single bond, a substituted or unsubstituted arylene group, or a substituted or unsubstituted heteroarylene group, and when L³ is a substituted arylene group or a substituted heteroarylene group, the substituent is selected from the group consisting of an a group, a cycloalkyl group, a heterocyclic group, an alkenyl group, a cycloalkenyl group, an alkynyl group, an alkoxy group, an alkylthio group, an aryl ether group an aryl thioether group, an aryl group, a heteroaryl group, a halogen atom, a cyano group, an amino group, a carbonyl group, a carboxy group, an oxycarbonyl group, a carbamoyl group, and - P(=O)R¹R², and R¹ and R² are each an aryl group or a heteroaryl group, and R¹ and R² are optionally condensed to form a ring;
R²² to R²⁶ are each independently selected from the group consisting of a hydrogen atom, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, a halogen atom, a cyano group, an amino group, a carbonyl group, a carboxy group, an oxycarbonyl group, a carbamoyl group, and - P(=O)R²⁷R²⁸, wherein R²⁷ and R²⁸ are each an aryl group or a heteroaryl group, and R²⁷ and R²⁸ are optionally condensed to form a ring.

2. The compound according to claim 1, wherein L²-R²¹ is a phenyl group, a fluorophenyl group, a biphenyl group, or a naphthyl group and/or
wherein L³ is a single bond, a phenylene group, or a biphenylene group, and R²² is a phenyl group, a naphthyl group, a terphenyl group, a 9,9-dialkylfluorenyl group, a carbazolyl group, a benzocarbazolyl group, a dibenzofuranyl group, or a dibenzothiophenyl group.

3. The compound according to claim 1 or 2, wherein R²³ to R²⁶ are each a hydrogen atom except at the position that is linked to L¹

4. The compound according to any one of claims 1 to 3, wherein R¹¹ is a phenyl group, a biphenyl group, a naphthyl group, a terphenyl group, or a 9,9-dialkylfluorenyl group.

5. An electronic device comprising the compound according to any one of claims 1 to 4.

6. A luminescent element comprising a positive electrode and a negative electrode opposed to each other, and an organic layer present between the positive electrode and the negative electrode, that emits light by electric energy, wherein the organic layer contains the compound according to any one of claims 1 to 4.

7. The luminescent element according to claim 6, wherein the organic layer has at least a luminescent layer and an electron transport layer, and the electron transport layer contains the compound according to any one of claims 1 to 4.

8. The luminescent element according to claim 7, wherein the luminescent layer has at least one luminescent layer containing at least one phosphorescent material
wherein, in particular, the luminescent layer contains at least one compound having an anthracene skeleton.

9. The luminescent element according to claim 7, wherein the electron transport layer is composed of two or more layers, and one of the electron transport layers that is in contact with the luminescent layer contains the compound according to any one of claims 1 to 4,
wherein, in particular, one of the electron transport layers that is in contact with the negative electrode contains a compound having a phenanthroline skeleton.

10. A photoelectric conversion element comprising the compound according to any one of claims 1 to 4.

11. An image sensor comprising the photoelectric conversion element according to claim 10.

## Patentansprüche

1. Verbindung, dargestellt durch die folgende allgemeine Formel (4) oder (5): 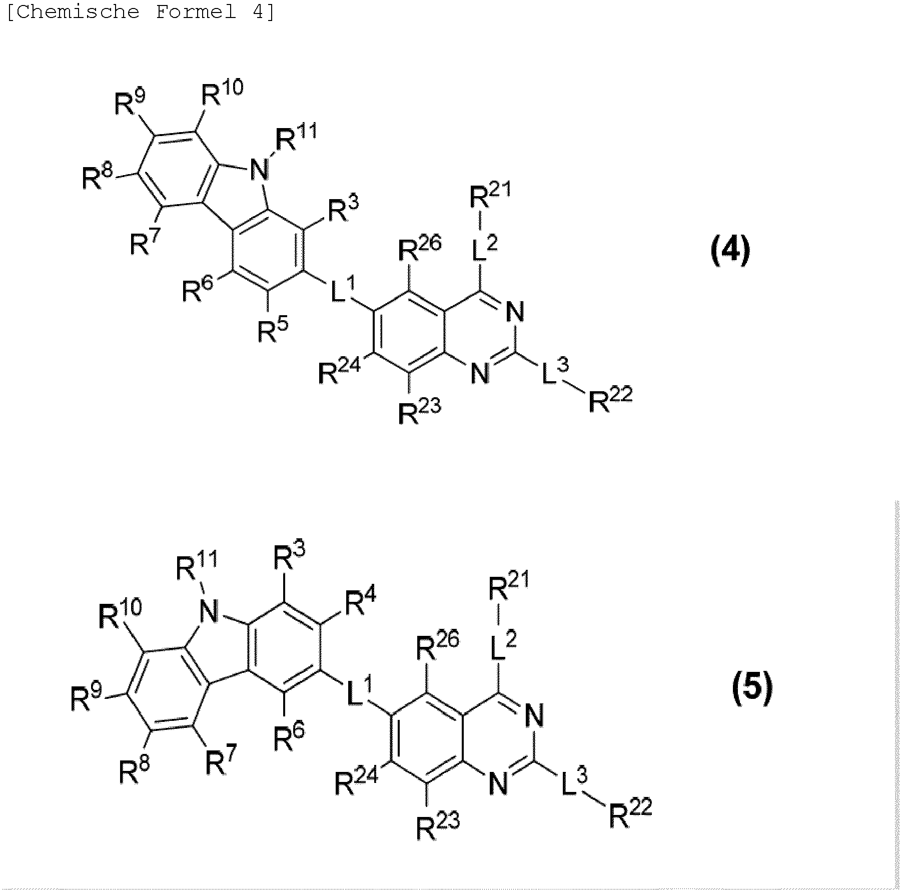
wobei L¹ eine Einfachbindung ist,
wobei R³ bis R¹⁰ jeweils ein Wasserstoffatom sind, außer an der Position, die mit L¹ verbunden ist,
R¹¹ ausgewählt ist aus der Gruppe bestehend aus einem Wasserstoffatom, einer Alkylgruppe, einer Cycloalkylgruppe, einer heterocyclischen Gruppe, einer Alkenylgruppe, einer Cycloalkenylgruppe, einer Arylgruppe, und einer Heteroarylgruppe,
wobei L² eine Einfachbindung, eine Phenylengruppe, eine Naphthalinylengruppe, oder eine Biphenylengruppe ist,
R²¹ ein Wasserstoffatom, ein Halogenatom, eine Carbazolylgruppe, eine Benzocarbazolylgruppe, eine Dibenzofuranylgruppe, oder eine Dibenzothiophenylgruppe ist,
L³ eine Einfachbindung, eine substituierte oder unsubstituierte Arylengruppe, oder eine substituierte oder unsubstituierte Heteroarylengruppe ist, und wenn L³ eine substituierte Arylengruppe oder eine substituierte Heteroarylengruppe ist, der Substituent ausgewählt ist aus der Gruppe bestehend aus einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Heteroarylgruppe, einem Halogenatom, einer Cyanogruppe, einer Aminogruppe, einer Carbonylgruppe, einer Carboxygruppe, einer Oxycarbonylgruppe, einer Carbamoylgruppe, und -P(= O)R¹R², und
R¹ und R² jeweils eine Arylgruppe oder eine Heteroarylgruppe sind, und R¹ und R² gegebenenfalls kondensiert sind, um einen Ring zu bilden;
R²² bis R²⁶ jeweils unabhängig voneinander ausgewählt sind aus der Gruppe bestehend aus einem Wasserstoffatom, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Heteroarylgruppe, einem Halogenatom, einer Cyanogruppe, einer Aminogruppe, einer Carbonylgruppe, einer Carboxygruppe, einer Oxycarbonylgruppe, einer Carbamoylgruppe, und -P(=O)R²⁷R²⁸, wobei R²⁷ und R²⁸ jeweils eine Arylgruppe oder eine Heteroarylgruppe sind, und R²⁷ und R²⁸ gegebenenfalls kondensiert sind, um einen Ring zu bilden.

2. Verbindung nach Anspruch 1, wobei L²-R²¹ eine Phenylgruppe, eine Fluorphenylgruppe, eine Biphenylgruppe, oder eine Naphthylgruppe ist und/oder
wobei L³ eine Einfachbindung, eine Phenylengruppe, oder eine Biphenylengruppe ist, und R²² eine Phenylgruppe, eine Naphthylgruppe, eine Terphenylgruppe, eine 9,9-Dialkylfluorenylgruppe, eine Carbazolylgruppe, eine Benzocarbazolylgruppe, eine Dibenzofuranylgruppe, oder eine Dibenzothiophenylgruppe ist.

3. Verbindung nach Anspruch 1 oder 2, wobei R²³ bis R²⁶ jeweils ein Wasserstoffatom sind, außer an der Position, die mit L¹ verbunden ist.

4. Verbindung nach einem der Ansprüche 1 bis 3, wobei R¹¹ eine Phenylgruppe, eine Biphenylgruppe, eine Naphthylgruppe, eine Terphenylgruppe, oder eine 9,9-Dialkylfluorenylgruppe ist.

5. Elektronische Vorrichtung, die die Verbindung nach einem der Ansprüche 1 bis 4 aufweist.

6. Lumineszenzelement, das eine positive Elektrode und eine negative Elektrode, die einander gegenüberliegen, und eine organische Schicht aufweist, die zwischen der positiven Elektrode und der negativen Elektrode vorhanden ist und Licht durch elektrische Energie emittiert, wobei die organische Schicht die Verbindung gemäß einem der Ansprüche 1 bis 4 enthält.

7. Lumineszenzelement nach Anspruch 6, wobei die organische Schicht mindestens eine Lumineszenzschicht und eine Elektronentransportschicht hat, und die Elektronentransportschicht die Verbindung nach einem der Ansprüche 1 bis 4 enthält.

8. Lumineszenzelement nach Anspruch 7, wobei die Lumineszenzschicht mindestens eine Lumineszenzschicht hat, die mindestens ein phosphoreszierendes Material enthält, wobei insbesondere die Lumineszenzschicht mindestens eine Verbindung mit einem Anthracengerüst enthält.

9. Lumineszenzelement nach Anspruch 7, wobei die Elektronentransportschicht aus zwei oder mehr Schichten besteht, und eine der Elektronentransportschichten, die mit der Lumineszenzschicht in Kontakt steht, die Verbindung nach einem der Ansprüche 1 bis 4 enthält,
wobei insbesondere eine der Elektronentransportschichten, die mit der negativen Elektrode in Kontakt steht, eine Verbindung mit einem Phenanthrolingerüst enthält.

10. Photoelektrisches Umwandlungselement, das die Verbindung gemäß einem der Ansprüche 1 bis 4 aufweist.

11. Bildsensor, der das photoelektrische Umwandlungselement nach Anspruch 10 aufweist.

## Revendications

1. Composé représenté par la formule générale (4) ou (5) suivante : 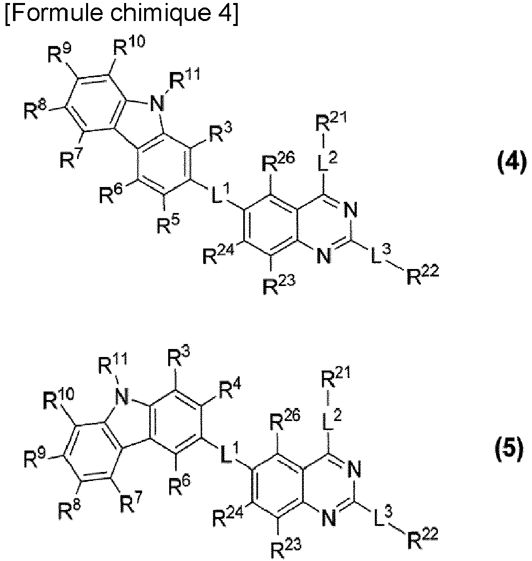
dans laquelle L¹ représente une liaison simple
dans laquelle R³ à R¹⁰ représentent chacun un atome d'hydrogène sauf à la position qui est liée à L¹,
R¹¹ est choisi dans le groupe consistant en un atome d'hydrogène, un groupe alkyle, un groupe cycloalkyle, un groupe hétérocyclique, un groupe alcényle, un groupe cycloalcényle, un groupe aryle et un groupe hétéroaryle,
dans laquelle L² représente une liaison simple, un groupe phénylène, un groupe naphtalénylène ou un groupe biphénylène,
R²¹ représente un atome d'hydrogène, un atome d'halogène, un groupe carbazolyle, un groupe benzocarbazolyle, un groupe dibenzofuranyle ou un groupe dibenzothiophényle,
L³ représente une liaison simple, un groupe arylène substitué ou non substitué, ou un groupe hétéroarylène substitué ou non substitué, et lorsque L³ est un groupe arylène substitué ou un groupe hétéroarylène substitué, le substituant est choisi dans le groupe consistant en un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un groupe aryle, un groupe hétéroaryle, un atome d'halogène, un groupe cyano, un groupe amino, un groupe carbonyle, un groupe carboxy, un groupe oxycarbonyle, un groupe carbamoyle et -P(=O)R¹R², et R¹ et R² représentent chacun un groupe aryle ou un groupe hétéroaryle, et R¹ et R² sont éventuellement condensés pour former un cycle ;
R²² à R²⁶ sont chacun indépendamment choisis dans le groupe consistant en un atome d'hydrogène, un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un groupe aryle, un groupe hétéroaryle, un atome d'halogène, un groupe cyano, un groupe amino, un groupe carbonyle, un groupe carboxy, un groupe oxycarbonyle, un groupe carbamoyle et - P(=O)R²⁷R^{28,} où R²⁷ et R²⁸ représentent chacun un groupe aryle ou un groupe hétéroaryle, et R²⁷ et R²⁸ sont éventuellement condensés pour former un cycle.

2. Composé selon la revendication 1, dans lequel L²-R²¹ représente un groupe phényle, un groupe fluorophényle, un groupe biphényle ou un groupe naphtyle, et/ou
dans lequel L³ représente une liaison simple, un groupe phénylène ou un groupe biphénylène et R²² représente un groupe phényle, un groupe naphtyle, un groupe terphényle, un groupe 9,9-dialkylfluorényle, un groupe carbazolyle, un groupe benzocarbazolyle, un groupe dibenzofuranyle ou un groupe dibenzothiophényle.

3. Composé selon la revendication 1 ou 2, dans lequel R²³ à R²⁶ représentent chacun un atome d'hydrogène sauf à la position qui est liée à L¹.

4. Composé selon l'une quelconque des revendications 1 à 3, dans lequel R¹¹ représente un groupe phényle, un groupe biphényle, un groupe naphtyle, un groupe terphényle ou un groupe 9,9-dialkylfluorényle.

5. Dispositif électronique comprenant le composé selon l'une quelconque des revendications 1 à 4.

6. Élément luminescent comprenant une électrode positive et une électrode négative opposées l'une à l'autre, et une couche organique présente entre l'électrode positive et l'électrode négative, qui émet de la lumière par énergie électrique, où la couche organique contient le composé selon l'une quelconque des revendications 1 à 4.

7. Élément luminescent selon la revendication 6, dans lequel la couche organique a au moins une couche luminescente et une couche de transport d'électrons, et la couche de transport d'électrons contient le composé selon l'une quelconque des revendications 1 à 4.

8. Élément luminescent selon la revendication 7, dans lequel la couche luminescente a au moins une couche luminescente contenant au moins un matériau phosphorescent,
dans lequel, en particulier, la couche luminescente contient au moins un composé ayant un squelette d'anthracène.

9. Élément luminescent selon la revendication 7, dans lequel la couche de transport d'électrons est composée de deux couches ou plus, et l'une des couches de transport d'électrons qui est en contact avec la couche luminescente contient le composé selon l'une quelconque des revendications 1 à 4,
dans lequel, en particulier, l'une des couches de transport d'électrons qui est en contact avec l'électrode négative contient un composé ayant un squelette de phénanthroline.

10. Élément de conversion photoélectrique comprenant le composé selon l'une quelconque des revendications 1 à 4.

11. Capteur d'image comprenant l'élément de conversion photoélectrique selon la revendication 10.
